# EUROPEAN PATENT APPLICATION

(11) **EP 4 696 688 A1**
(43) Date of publication of application: **18.02.2026**
(21) Application number: 24788144.4
(22) Date of filing: 11.04.2024
(51) Int. Cl.: C07D 405/10, C07D 413/10, C07D 417/10, C07D 401/14, C07D 471/04, C07D 487/04, C07D 519/00, A61K 31/444, A61K 31/4709, A61K 31/4725, A61P 3/12, A61P 9/00

(54) **BENZO-FUSED RING COMPOUND INHIBITOR, AND PREPARATION METHOD THEREFOR AND USE THEREOF**

(30) Priority: 11.04.2023 CN 202310384096; 26.05.2023 CN 202310611392; 21.07.2023 CN 202310907026; 25.08.2023 CN 202311086944; 01.12.2023 CN 202311645650
(71) Applicant: Shanghai Hansoh Biomedical Co., Ltd., Shanghai 201203 (CN); Jiangsu Hansoh Pharmaceutical Group Co., Ltd., Lianyungang, Jiangsu 222069 (CN)
(72) Inventor: MAO, Xiaofeng, Shanghai 201203 (CN); SU, Yidong, Shanghai 201203 (CN); YU, Wensheng, Shanghai 201203 (CN); WANG, Jun, Shanghai 201203 (CN); ZHOU, Xiaohan, Shanghai 201203 (CN)
(74) Representative: De Clercq & Partners
(86) International application number: PCT/CN2024/087227
(87) International publication number: WO 2024/213042

(57) **Abstract**

The present invention relates to a benzo-fused ring compound inhibitor, and a preparation method therefor and the use thereof. Specifically, the present invention relates to a compound as represented by general formula (I), a preparation method therefor, a pharmaceutical composition containing the compound, and the use thereof in a drug for the treatment of chronic kidney disease, kidney or cardiac fibrosis, diabetic nephropathy, congestive heart failure, hypertension, primary aldosteronism and Cushing's syndrome, wherein the definition of each substituent in the general formula (I) is the same as defined in the description.

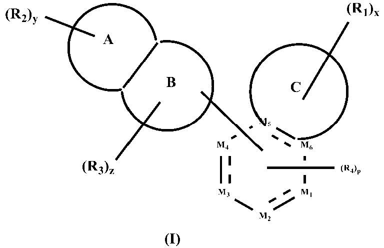

## Description

**The present application claims the right of priority for**
202310384096.5, filed on April 11, 2023;
202310611392.4, filed on May 26, 2023;
202310907026.3, filed on July 21, 2023;
202311086944.0, filed on August 25, 2023;
202311645650.7, filed on December 1, 2023.

### Technical Field

The present invention belongs to the field of biomedicine, and specifically relates to a benzo-fused ring compound inhibitor, a preparation method therefor, and the use thereof.

### Background Art

Aldosterone, a steroid hormone secreted from adrenal gland, binds and activates mineralocorticoid receptor (MR). In primary cells of distal renal tubules and renal collecting ducts, MR activation results in sodium and water retention accompanied with potassium excretion, leading to plasma volume expansion and elevated blood pressure (BP). Renin-angiotensin-aldosterone system (RAAS), as an endocrine system, regulates human blood pressure and body fluid balance. As current antihypertensive drugs, angiotensin-converting enzyme inhibitors (ACEi), angiotensin II receptor antagonists (ARBs), and mineralocorticoid receptor antagonists (MRAs) regulate blood pressure by inhibiting this pathway. Patients on long-term use of ACEi or ARB drugs develop "aldosterone breakthrough". The aldosterone level increases after a brief decrease, leading to damage to the target organ. At present, among aldosterone inhibitors currently available on the market, only spironolactone results in hyperkalemia. Excessive aldosterone measured in the circulation is known as primary aldosteronism (PA), which occurs when the renin-angiotensin-aldosterone system (RAAS) dysregulates aldosterone production. PA was first found in patients with adrenal adenoma. Recent evidences have shown that the prevalence of obesity-associated PA is increasing. PA is a common cause of secondary hypertension. The prevalence thereof is 14%-21 % of that of patients with refractory hypertension (RHTN). The refractory hypertension means that the blood pressure is still higher than the target blood pressure 140/90 mm Hg despite the use of three antihypertensive drugs (calcium channel blockers, angiotensin-converting enzyme inhibitors, angiotensin receptor blockers, and diuretics). Refractory hypertension is a high-risk disease, which has a high comorbidity rate with diabetes, chronic nephropathy, ischemic heart disease, and cerebrovascular diseases.

CYP11B2 is a gene encoding aldosterone synthase and is highly homologous to the sequence of CYP11B1 gene encoding cortisol synthase. Developing inhibitors with a high selectivity for CYP11B2 to inhibit aldosterone synthesis is the main direction for treating refractory hypertension and primary aldosteronism.

### Summary of the Invention

An object of the present invention is to provide a compound as represented by general formula (I), or a stereoisomer or a pharmaceutically acceptable salt thereof: 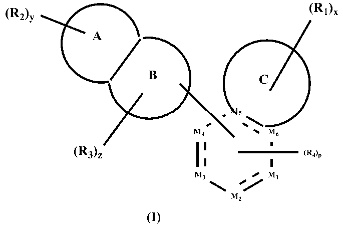 wherein 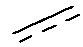
is a single bond or a double bond;
ring A is phenyl, 4- to 7-membered heterocyclyl, 5- to 6-membered heteroaryl, or absent;
ring B is 4- to 7-membered heterocyclyl, 5- to 6-membered heteroaryl, or phenyl;
ring C is cycloalkyl, heteroaryl, heterocyclyl, or absent;
M₁, M₂, M₄, M₅, and M₆ are each independently selected from N, NH, or CH;
M₃ is a bond, N, or CH;
each R₁ is independently selected from cycloalkyl, heterocyclyl, cycloalkyloxy, heterocyclyloxy, cycloalkylamino, heterocyclylamino, cycloalkylthio, heterocyclylthio, -C(O)(CH₂)ₙR_{b}, -NRₐC(O)(CH₂)ₙR_{b}, -O(CH₂)ₙR_{b}, -NH(CH₂)ₙR_{b}, - S(CH₂)ₙR_{b}, -S(O)₂(CH₂)ₙR_{b}, -S(O)(NH)(CH₂)ₙR_{b}, -NRₐS(O)₂(CH₂)ₙR_{b}, - NRₐS(O)(NH)(CH₂)ₙR_{b}, or -NS(O)(CH₂)ₙRₐR_{b}, wherein optionally the cycloalkyl, heterocyclyl, cycloalkyloxy, heterocyclyloxy, cycloalkylamino, heterocyclylamino, cycloalkylthio, or heterocyclylthio is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, - C(O)(CH₂)ₙR_{b}, -S(O)₂(CH₂)ₙR_{b}, -S(O)(NH)(CH₂)ₙR_{b}, -S(O)₂(CH₂)ₙR_{b}, or - NRₐS(O)(NH)(CH₂)ₙR_{b}; wherein exocyclic oxygen, nitrogen and sulfur atoms in the cycloalkyloxy, heterocyclyloxy, cycloalkylamino, heterocyclylamino, cycloalkylthio and heterocyclylthio are connected to ring C;
or any two R₁, together with adjacent carbon atoms, form 3- to 8-membered cycloalkyl or 4- to 7-membered heterocyclyl, wherein optionally the 3- to 8-membered cycloalkyl or 4- to 7-membered heterocyclyl is further substituted with oxo, -C(O)R_{b}, -NRₐC(O)R_{b}, -S(O)₂R_{b}, -S(O)(NH)R_{b}, -NRₐS(O)₂R_{b}, or - NRₐS(O)(NH)R_{b};
Rₐ or R_{b} is each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, aryl, heteroaryl, or heterocyclyl, wherein optionally the cycloalkyl, aryl, heteroaryl, or heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, or -S(O)₂alkyl;
R₂, R₃, or R₄ is each independently selected from hydrogen, deuterium, oxo, halogen, amino, hydroxyl, cyano, nitro, alkyl, alkenyl, alkynyl, deuteroalkyl, haloalkyl, alkoxy, haloalkoxy, hydroxyalkyl, cycloalkyl, heterocyclyl, aryl, or heteroaryl, wherein the alkyl, alkenyl, alkynyl, deuteroalkyl, haloalkyl, alkoxy, haloalkoxy, hydroxyalkyl, cycloalkyl, heterocyclyl, aryl, heteroaryl, - NRₐC(O)(CH₂)ₙR_{b}, or -C(O)NRₐ(CH₂)ₙR_{b} can be optionally further substituted;
or R₂ and R₃, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or any two R₂, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or R₂ and R₄, together with adjacent atoms, form 5- to 14-membered cycloalkyl, 5- to 14-membered heteroaryl, or 5-14-membered heterocyclyl, wherein optionally the 5- to 14-membered cycloalkyl, 5- to 14-membered heteroaryl, or 5-14-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
p, x, y, and z are each independently selected from 1, 2, 3, or 4; and
n is selected from 0, 1, 2, or 3;
when 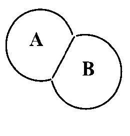 is 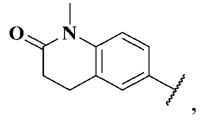 M₁ is N, and ring C is 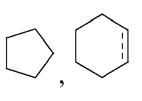 or 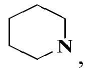 , R₁ is not -NRₐC(O)R_{b} and -NRₐS(O)₂R_{b};
when M₁ is N, and ring C is absent, or is 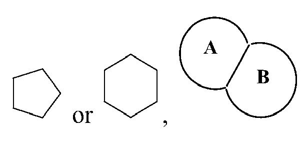 is not

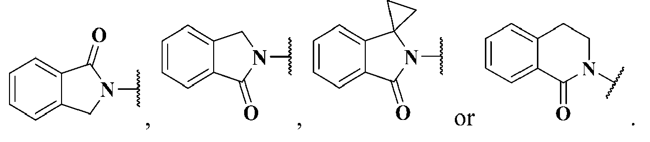

In certain embodiments of the present invention, a compound as represented by general formula (I), or a stereoisomer or a pharmaceutically acceptable salt thereof is provided: 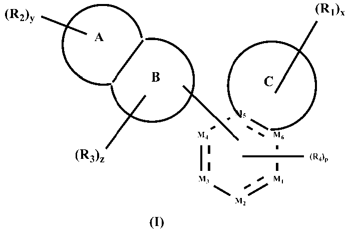 wherein 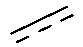
is a single bond or a double bond;
ring A is phenyl, 4- to 7-membered heterocyclyl, 5- to 6-membered heteroaryl, or absent; when ring A is absent, R₂ is connected to ring B;
ring B is 4- to 7-membered heterocyclyl, 5- to 6-membered heteroaryl, or phenyl;
ring C is cycloalkyl, heteroaryl, heterocyclyl, or absent; when ring C is absent, R₁ is connected to 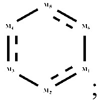
M₁, M₂, M₄, M₅, and M₆ are each independently selected from N, NH, or CH;
M₃ is a bond, N, or CH;
each R₁ is independently selected from cycloalkyl, heterocyclyl, cycloalkyloxy, heterocyclyloxy, cycloalkylamino, heterocyclylamino, cycloalkylthio, heterocyclylthio, -C(O)(CH₂)ₙR_{b}, -NRₐC(O)(CH₂)ₙR_{b}, -S(O)₂(CH₂)ₙR_{b}, - S(O)(NH)(CH₂)ₙR_{b}, -NRₐS(O)₂(CH₂)ₙR_{b}, or -NRₐS(O)(NH)(CH₂)ₙR_{b}, wherein optionally the cycloalkyl, heterocyclyl, cycloalkyloxy, heterocyclyloxy, cycloalkylamino, heterocyclylamino, cycloalkylthio, or heterocyclylthio is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, -C(O)(CH₂)ₙR_{b}, -S(O)₂(CH₂)ₙR_{b}, - S(O)(NH)(CH₂)ₙR_{b}, -S(O)₂(CH₂)ₙR_{b}, or -NRₐS(O)(NH)(CH₂)ₙR_{b};
or any two R₁, together with adjacent carbon atoms, form 3- to 8-membered cycloalkyl or 4- to 7-membered heterocyclyl, wherein optionally the 3- to 8-membered cycloalkyl or the 4- to 7-membered heterocyclyl is further substituted with -C(O)R_{b}, -NRₐC(O)R_{b}, -S(O)₂R_{b}, -S(O)(NH)R_{b}, -NRₐS(O)₂R_{b}, or - NRₐS(O)(NH)R_{b};
Rₐ or R_{b} is each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, aryl, heteroaryl, or heterocyclyl;
R₂, R₃, or R₄ is each independently selected from hydrogen, deuterium, oxo, halogen, amino, hydroxyl, cyano, nitro, alkyl, alkenyl, alkynyl, deuteroalkyl, haloalkyl, alkoxy, haloalkoxy, hydroxyalkyl, cycloalkyl, heterocyclyl, aryl, or heteroaryl, wherein the alkyl, alkenyl, alkynyl, deuteroalkyl, haloalkyl, alkoxy, haloalkoxy, hydroxyalkyl, cycloalkyl, heterocyclyl, aryl, heteroaryl, - NRₐC(O)(CH₂)ₙR_{b}, or -C(O)NRₐ(CH₂)ₙR_{b} can be optionally further substituted;
or R₂ and R₃, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or any two R₂, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
p, x, y, and z are each independently selected from 1, 2, 3, or 4; and
n is selected from 0, 1, 2, or 3;
when ring C is present or substituted with R₁, M₅ or M₆ is CH, and the H atom is further substituted with ring C or R₁.

In a preferred embodiment of the present invention, when 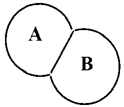 is 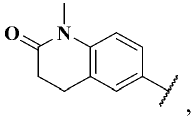 M₁ is N, and ring C is 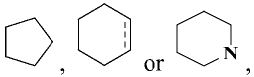 R₁ is not - NRₐC(O)R_{b} and -NRₐS(O)₂R_{b};
when M₁ is N, and ring C is absent, or is 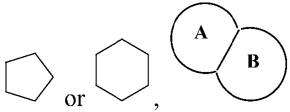 is not

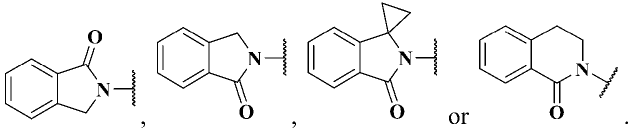

In a preferred embodiment of the present invention, ring A is not selected from 5- to 10-membered heterocyclyl or 5- to 10-membered heteroaryl containing 1-3 moieties selected from C(O), N, O, S, SO₂, or SONH.

In a preferred embodiment of the present invention, ring A is selected from 5-to 7-membered heterocyclyl, or 5- to 6-membered heteroaryl containing 1-3 moieties selected from C(O), N, O, S, SO₂ or SONH.

In a preferred embodiment of the present invention, ring B is selected from phenyl, or 5- to 7-membered heterocyclyl or 5- to 6-membered heteroaryl containing 1-3 moieties selected from C(O), N, O, or S.

In a preferred embodiment of the present invention, 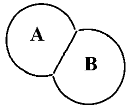 is selected from 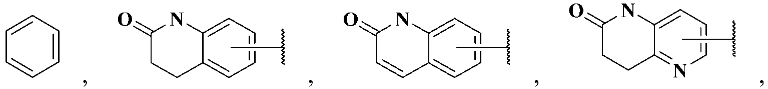 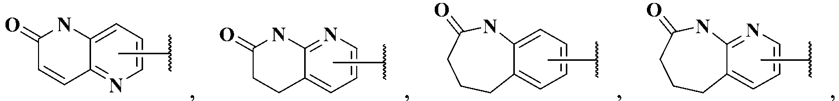 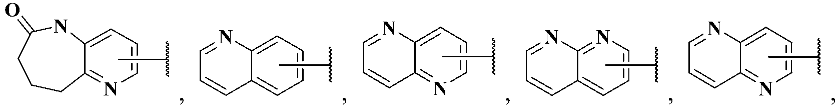 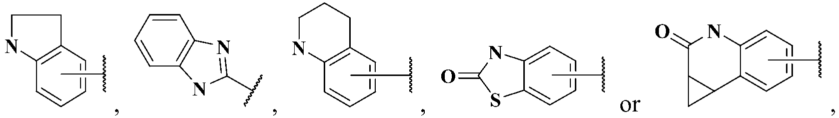 wherein 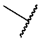 represents a connection site between

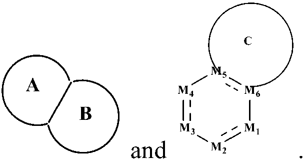

In a preferred embodiment of the present invention, 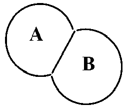 is selected from

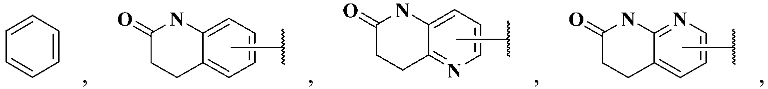

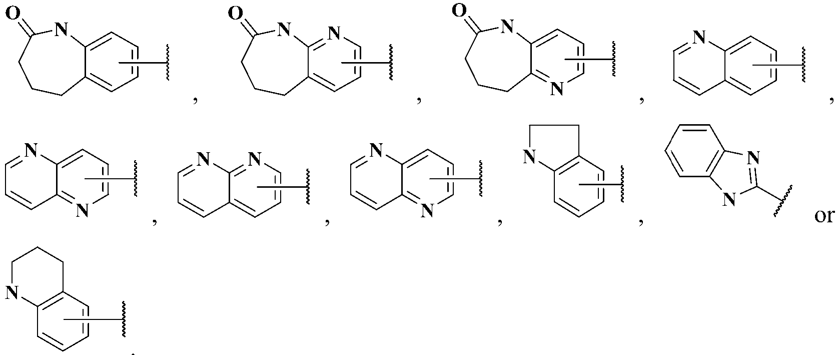

In a preferred embodiment of the present invention, 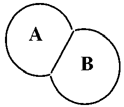 is selected from

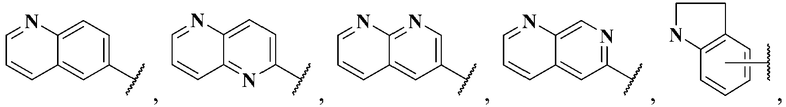

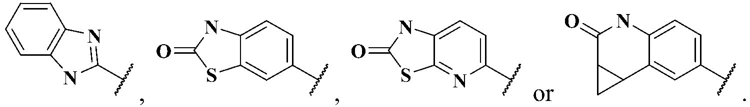

In a preferred embodiment of the present invention, 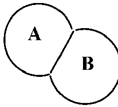 is selected from

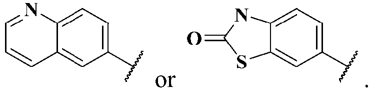

In a preferred embodiment of the present invention, ring C is selected from 3-to 10-membered cycloalkyl, or 4- to 10-membered heterocyclyl containing 1-3 moieties selected from C(O), N, O, S, SO₂, or SONH.

In a preferred embodiment of the present invention, ring C is selected from 5-to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, or 7- to 10-membered bicyclic heterocyclyl containing 1-3 moieties selected from C(O), N, O, S, SO₂, or SONH.

In a preferred embodiment of the present invention, ring C is selected from the following groups:

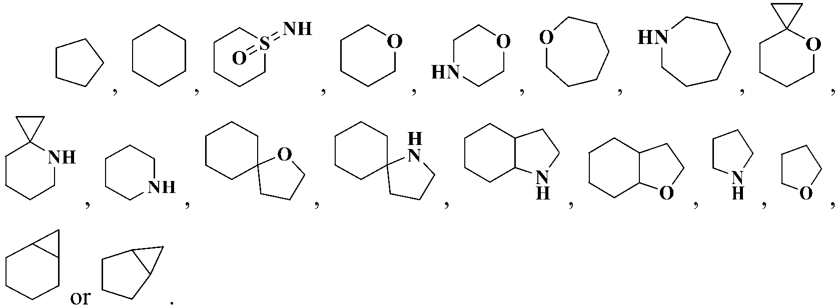

In a preferred embodiment of the present invention, ring C is absent.

In a preferred embodiment of the present invention, each R₁ is independently selected from 3- to 10-membered cycloalkyl, 4- to 10-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, 4- to 8-membered heterocyclylthio, -C(O)(CH₂)ₙR_{b}, - NRₐC(O)(CH₂)ₙR_{b}, -O(CH₂)ₙR_{b}, -NH(CH₂)ₙR_{b}, -S(CH₂)ₙR_{b}, -S(O)₂(CH₂)ₙR_{b}, - S(O)(NH)(CH₂)ₙR_{b}, -NRₐS(O)₂(CH₂)ₐR_{b}, or -NRₐS(O)(NH)(CH₂)ₙR_{b}, wherein optionally the 3- to 10-membered cycloalkyl, 4- to 10-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, or 4- to 8-membered heterocyclylthio is further substituted with one or more substituents selected from oxo, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, -C(O)(CH₂)ₙR_{b}, -S(O)₂(CH₂)ₙR_{b}, - S(O)(NH)(CH₂)ₙR_{b}, -S(O)₂(CH₂)ₙR_{b}, or -NRₐS(O)(NH)(CH₂)ₙR_{b};

Rₐ or R_{b} is each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 moieties selected from C(O), N, O, or S, or 4-to 8-membered heterocyclyl containing 1-3 moieties selected from C(O), N, O, or S is further substituted with one or more substituents selected from oxo, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, or - SO₂-C₁₋₆ alkyl.

In a preferred embodiment of the present invention, Rₐ or R_{b} is each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, 3- to 8-membered cycloalkyl, 5- to 8-membered heteroaryl containing 1-3 moieties selected from C(O), N, O, or S, or 4-to 8-membered heterocyclyl containing 1-3 moieties selected from C(O), N, O, or S, wherein optionally the 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S is further substituted with one or more substituents selected from oxo, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, 3- to 6-membered cycloalkyl, or -SO₂-C₁₋₆ alkyl.

In a preferred embodiment of the present invention, each R₁ is independently selected from 3- to 10-membered cycloalkyl, 4- to 10-membered heterocyclyl containing 1-3 moieties selected from N, O, S, SO₂, or SONH, 3- to 8-membered cycloalkyloxy, 4- to 8-membered heterocyclyloxy containing 1-3 moieties selected from N, O, S, SO₂, or SONH, -C(O)(CH₂)ₙR_{b}, -NRₐC(O)(CH₂)ₙR_{b}, -O(CH₂)ₙR_{b}, - S(O)₂(CH₂)ₙR_{b}, -S(O)(NH)(CH₂)ₙR_{b}, -NRₐS(O)₂(CH₂)ₙR_{b}, or - NRₐS(O)(NH)(CH₂)ₙR_{b}, wherein optionally the 3- to 10-membered cycloalkyl, 4- to 10-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH is further substituted with one or more substituents selected from oxo, -C(O)(CH₂)ₙR_{b}, -S(O)₂(CH₂)ₙR_{b}, - S(O)(NH)(CH₂)ₙR_{b}, -S(O)₂(CH₂)ₙR_{b}, or -NRₐS(O)(NH)(CH₂)ₙR_{b}; and

Rₐ or R_{b} is each independently selected from hydrogen, deuterium, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 5- to 8-membered heteroaryl containing 1-3 heteroatoms selected from N, O, or S or the 4-to 8-membered heterocyclyl containing 1-3 moieties selected from C(O), N, O, or S is further substituted with one or more substituents selected from oxo, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, or - SO₂-C₁₋₃ alkyl.

In a preferred embodiment of the present invention, each R₁ is independently selected from 3- to 8-membered cycloalkyl, 4- to 8-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, 4- to 8-membered heterocyclylthio, -C(O)(CH₂)ₙR_{b}, - NRₐC(O)(CH₂)ₙR_{b}, -S(O)₂(CH₂)ₙR_{b}, -S(O)(NH)(CH₂)ₙR_{b}, -NRₐS(O)₂(CH₂)ₙR_{b}, or - NRₐS(O)(NH)(CH₂)ₙR_{b}, wherein optionally the 3- to 8-membered cycloalkyl, 4- to 8-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, or 4- to 8-membered heterocyclylthio is further substituted with one or more substituents selected from oxo, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, -C(O)(CH₂)ₙR_{b}, -S(O)₂(CH₂)ₙR_{b}, -S(O)(NH)(CH₂)ₙR_{b}, - S(O)₂(CH₂)ₙR_{b}, or -NRₐS(O)(NH)(CH₂)ₙR_{b}; and

Rₐ or R_{b} is each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S.

In a preferred embodiment of the present invention, each R₁ is independently selected from 3- to 8-membered cycloalkyl, 4- to 8-membered heterocyclyl containing 1-3 moieties selected from N, O, S, SO₂, or SONH, 3- to 8-membered cycloalkyloxy, 3- to 8-membered cycloalkylamino, 4- to 8-membered heterocyclyloxy containing 1-3 moieties selected from N, O, S, SO₂, or SONH, 4-to 8-membered heterocyclylamino containing 1-3 moieties selected from N, O, S, SO₂, or SONH, -C(O)(CH₂)ₙR_{b}, -NRₐC(O)(CH₂)ₙR_{b}, -O(CH₂)ₙR_{b}, -S(O)₂(CH₂)ₙR_{b}, -S(O)(NH)(CH₂)ₙR_{b}, -NRₐS(O)₂(CH₂)ₙR_{b}, or -NRₐS(O)(NH)(CH₂)ₙR_{b}, wherein optionally the 3- to 8-membered cycloalkyl, 4- to 8-membered heterocyclyl containing 1-3 moieties selected from N, O, S, SO₂, or SONH, 3- to 8-membered cycloalkyloxy, 3- to 8-membered cycloalkylamino, 4- to 8-membered heterocyclyloxy containing 1-3 moieties selected from N, O, S, SO₂, or SONH, or 4- to 8-membered heterocyclylamino containing 1-3 moieties selected from N, O, S, SO₂, or SONH is further substituted with one or more substituents selected from oxo, -C(O)(CH₂)ₙR_{b}, -S(O)₂(CH₂)ₙR_{b}, -S(O)(NH)(CH₂)ₙR_{b}, -S(O)₂(CH₂)ₙR_{b}, or - NRₐS(O)(NH)(CH₂)ₙR_{b}; and
Rₐ or R_{b} is each independently selected from hydrogen, deuterium, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 5- to 8-membered heteroaryl containing 1-3 heteroatoms selected from N, O, or S, or 4- to 8-membered heterocyclyl containing 1-3 moieties selected from C(O), N, O, or S is further substituted with one or more substituents selected from oxo, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, 3- to 6-membered cycloalkyl, or -SO₂-C₁₋₃ alkyl.

In a preferred embodiment of the present invention, each R₁ is independently selected from 3- to 8-membered cycloalkyl, 4- to 8-membered heterocyclyl containing 1-3 moieties selected from N, O, S, SO₂, or SONH, 3- to 8-membered cycloalkyloxy, 4- to 8-membered heterocyclyloxy containing 1-3 moieties selected from N, O, S, SO₂, or SONH, -C(O)(CH₂)ₙR_{b}, -NRₐC(O)(CH₂)ₙR_{b}, -S(O)₂(CH₂)ₙR_{b}, -S(O)(NH)(CH₂)ₙR_{b}, -NRₐS(O)₂(CH₂)ₙR_{b}, or -NRₐS(O)(NH)(CH₂)ₙR_{b}, wherein optionally the 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH is further substituted with one or more substituents selected from oxo, -C(O)(CH₂)ₙR_{b}, -S(O)₂(CH₂)ₙR_{b}, - S(O)(NH)(CH₂)ₙR_{b}, -S(O)₂(CH₂)ₙR_{b}, or -NRₐS(O)(NH)(CH₂)ₙR_{b}; and
In a preferred embodiment of the present invention, R₂, R₃, or R₄ is each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ deuteroalkoxy, C₁₋₆ hydroxyalkyl, 3- to 8-membered cycloalkyl, 3- to 8-membered cycloalkyloxy, 3- to 8-membered cycloalkylamino, C₆₋₁₀ aryl, 5- to 6-membered heteroaryl containing 1-3 heteroatoms selected from N, O, or S or 4- to 8-membered heterocyclyl containing 1-3 moieties selected from C(O), N, O, or S, -NRₐR_{b}, -NRₐC(O)R_{b}, or - C(O)NRₐR_{b}.

In a preferred embodiment of the present invention, R₂, R₃, or R₄ is each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, 3- to 8-membered cycloalkyl, or 4- to 8-membered heterocyclyl containing 1-3 moieties selected from C(O), N, O, or S, - NRₐC(O)R_{b}, or -C(O)NRₐR_{b}; and
Rₐ or R_{b} is each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S.

In a preferred embodiment of the present invention, R₂, R₃, or R₄ is each independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy.

In a preferred embodiment of the present invention, R₂, R₃, or R₄ is each independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, or C₁₋₃ hydroxyalkyl.

In a more preferred embodiment of the present invention, the general formula (I) is further a compound as represented by general formulas (III-a)-(III-i), or a stereoisomer or a pharmaceutically acceptable salt thereof: 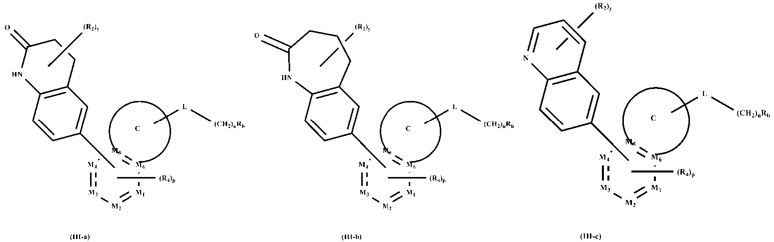 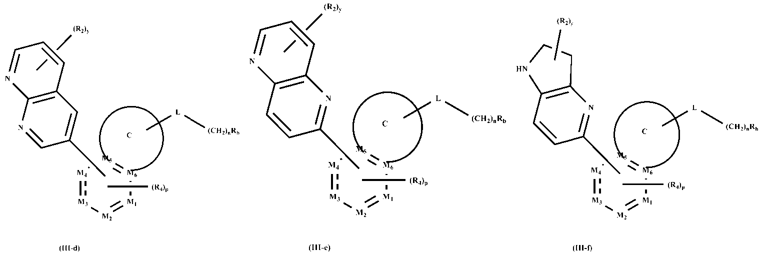 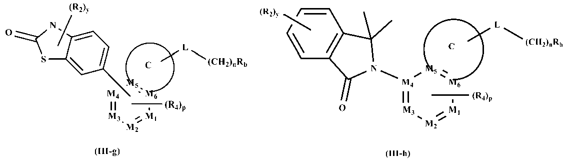 or 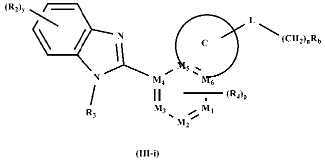
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, or 7- to 10-membered bicyclic heterocyclyl containing 1-3 moieties selected from C(O), N, O, S, SO₂, or SONH;
or ring C is absent;
L is selected from a bond, -O-, -R_{c}C(O)-, -R_{c}S(O)NH-, or -R_{c}S(O)₂, preferably -NHC(O)-;
R_{c} is selected from a bond, NH, 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH;
R₂, R₃, or R₄ is each independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyl;
R_{b} is selected from hydrogen, deuterium, halogen, hydroxyl, cyano, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂, or SONH; and
y is 1, 2, or 3.

In a more preferred embodiment of the present invention, R_{b} is selected from hydrogen, deuterium, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂ or SONH, wherein optionally the C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂ or SONH is further substituted with hydroxyl, amino, CN, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or 3- to 6-membered cycloalkyl.

In a more preferred embodiment of the present invention, when R_{c} is selected from 3- to 8-membered cycloalkyloxy, or 4- to 8-membered heterocyclyloxy containing 1-3 moieties selected from N, O, S, SO₂ or SONH, the exocyclic oxygen atom is connected to ring C, and if ring C is absent, the oxygen atom is connected to

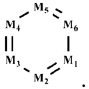

In a more preferred embodiment of the present invention, R_{b} is selected from hydrogen, deuterium, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂ or SONH, wherein optionally the C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂ or SONH is further substituted with hydroxyl, amino, CN, C₁₋₃ alkyl, C₁₋₃ deuteroalkyl, C₁₋₃ haloalkyl, C₁₋₃ alkoxy, C₁₋₃haloalkoxy, C₁₋₃ hydroxyalkyl, or 3- to 6-membered cycloalkyl.

In a more preferred embodiment of the present invention, when R_{c} is selected from 3- to 8-membered cycloalkyloxy, or 4- to 8-membered heterocyclyloxy containing 1-3 moieties selected from N, O, S, SO₂ or SONH, the oxygen atom is connected to ring C, and if ring C is absent, the oxygen atom is connected to

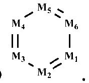

In a more preferred embodiment of the present invention, the general formula (I) is further a compound as represented by general formula (III') or general formula (IV'), or a stereoisomer or a pharmaceutically acceptable salt thereof: 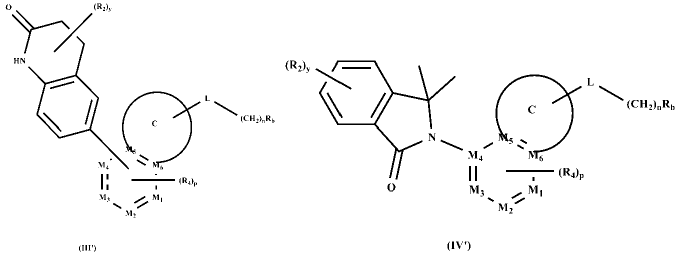
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, or 7- to 10-membered bicyclic heterocyclyl containing 1-3 moieties selected from C(O), N, O, S, SO₂, or SONH;
or ring C is absent;
L is selected from a bond, -O-, -R_{c}C(O)-, -R_{c}S(O)NH-, or -R_{c}S(O)₂;
R_{c} is selected from a bond, NH, 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH;
R₂ or R₄ is each independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, or C₁₋₃ hydroxyalkyl;
R_{b} is selected from hydrogen, deuterium, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂, or SONH; and
y is 1, 2, or 3.

In a more preferred embodiment of the present invention, the general formula (I) is further a compound as represented by general formula (IV-a) or (IV-b), or a stereoisomer or a pharmaceutically acceptable salt thereof: 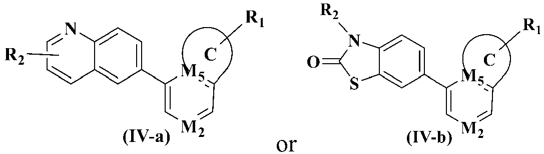 wherein
M₂ or M₃ is each independently selected from N or CH; when ring C is present or substituted with R₁, M₂ or M₃ is CH, and the H atom is further substituted;
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, 7- to 10-membered bicyclic heterocyclyl containing 1-3 moieties selected from C(O), N, O, S, SO₂, or SONH, or absent;
R₁ is selected from -C(O)R_{b}, -NRₐC(O)R_{b}, -OR_{b}, -S(O)₂R_{b}, -S(O)(NH)R_{b}, - NRₐS(O)₂R_{b}, -NRₐS(O)(NH)R_{b}, or 4- to 8-membered heterocyclyl, wherein the 4-to 8-membered nitrogen-containing heterocyclyl is optionally further substituted with -C(O)R_{b} or -S(O)₂R_{b};
Rₐ or R_{b} is each independently selected from hydrogen, deuterium, halogen, hydroxy, cyano, C₁₋₃ alkyl, C₁₋₃ deuteroalkyl, C₁₋₃ haloalkyl, C₁₋₃ alkoxy, C₁₋₃ haloalkoxy, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂ or SONH is further substituted with hydroxyl, amino, CN, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or 3- to 6-membered cycloalkyl;
R₂ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy.

In a more preferred embodiment of the present invention, the general formula (IV-a) is further a compound as represented by general formula (IV-a-1), or a stereoisomer or a pharmaceutically acceptable salt thereof:

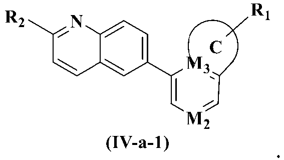

In a more preferred embodiment of the present invention, R_{b} is selected from hydrogen, deuterium, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂ or SONH, wherein optionally the C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂ or SONH is further substituted with hydroxyl, amino, CN, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or 3- to 6-membered cycloalkyl.

In a more preferred embodiment of the present invention, the general formula (I) is further a compound as represented by general formula (V-a) or (V-b), or a stereoisomer or a pharmaceutically acceptable salt thereof: 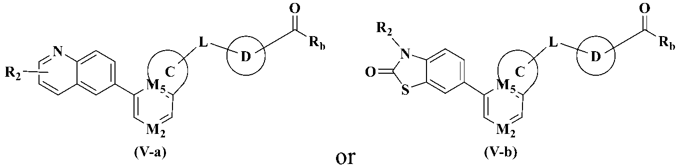 wherein
L is selected from a bond, NH or O; and
ring D is selected from 4- to 8-membered heterocyclyl containing 1 to 3 nitrogen atoms.

In a more preferred embodiment of the present invention, the general formula (I) is further a compound as represented by general formulas (VI-a)-(VI-j), or a stereoisomer or a pharmaceutically acceptable salt thereof: 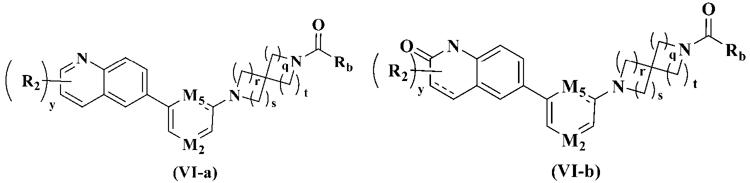 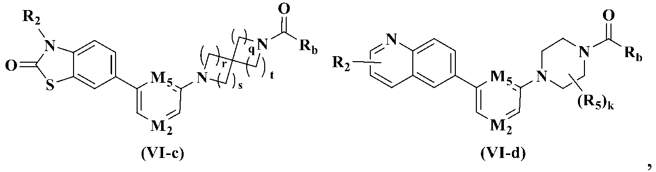 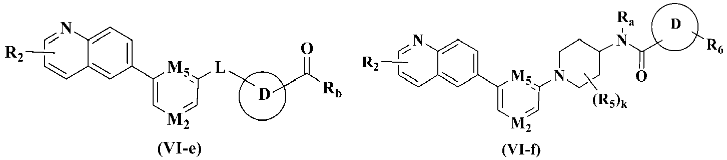 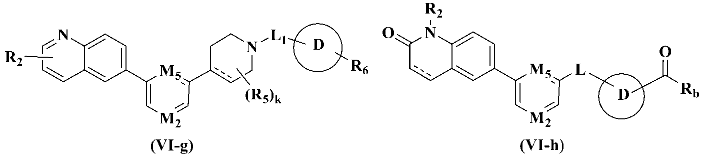 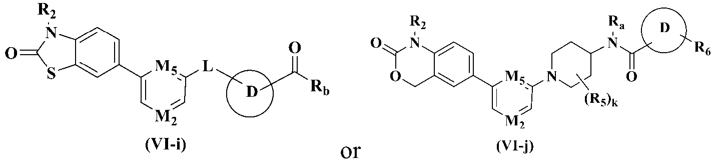 wherein
L is selected from NRₐ, O, or S;
L₁ is selected from C(O) or S(O)₂;
Rₐ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
M₂ is selected from N or CH;
M₅ is selected from N or CH;
R₂ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
or any two R₂, together with adjacent atoms, form 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S;
each R_{b} is independently selected from hydrogen, deuterium, C₁₋₃ alkyl, C₁₋₃ deuteroalkyl, C₁₋₃ haloalkyl, C₁₋₃ alkoxy, C₁₋₃haloalkoxy, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S is further substituted with halogen, hydroxyl, amino, CN, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or 3- to 6-membered cycloalkyl;
each R₅ is independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
R₆ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyl;
or any two R₅, together with adjacent carbon atoms, form 3- to 8-membered cycloalkyl or 3- to 8-membered heterocyclyl;
ring D is selected from 3- to 8-membered cycloalkyl or 4- to 8-membered heterocyclyl, preferably 4- to 6-membered nitrogen-containing heterocyclyl;
q, r, s, and t are each independently selected from 1 or 2; and
k is independently selected from 1, 2, or 3.

In a more preferred embodiment of the present invention, q, r, s, and t are 1.

In a more preferred embodiment of the present invention, ring D is 4- to 6-membered heterocyclyl containing 1-2 nitrogen atoms.

In a more preferred embodiment of the present invention, the general formula (I) is further a compound as represented by general formulas (VI-a)-(VI-i), or a stereoisomer or a pharmaceutically acceptable salt thereof: 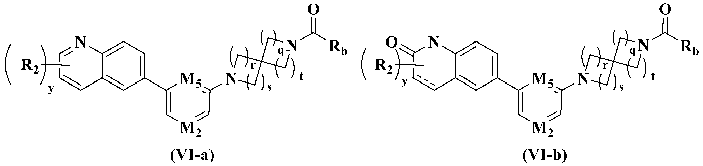 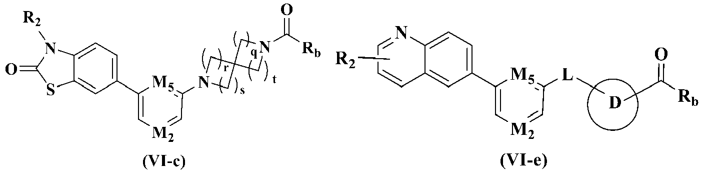 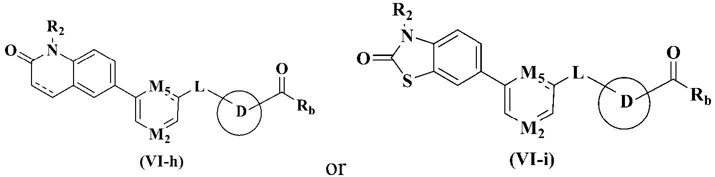 wherein
M₂ is selected from N or CH;
M₅ is selected from N or CH;
L is selected from NH, O or S;
R₂ is selected from hydrogen, deuterium, halogen, C₁₋₃ alkyl, or C₁₋₃ deuteroalkyl;
R_{b} is selected from hydrogen, deuterium, C₁₋₃ alkyl, C₁₋₃ deuteroalkyl, C₁₋₃ haloalkyl, C₁₋₃ alkoxy, C₁₋₃ haloalkoxy, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S is further substituted with halogen, hydroxyl, amino, CN, C₁₋₃ alkyl, C₁₋₃ deuteroalkyl, C₁₋₃haloalkyl, C₁₋₃ alkoxy, C₁₋₃ haloalkoxy, C₁₋₃ hydroxyalkyl, or 3- to 6-membered cycloalkyl;
ring D is selected from 4- to 6-membered heterocyclyl containing 1-2 nitrogen atoms;
q, r, s, and t are each independently selected from 1 or 2; and
y is independently selected from 0, 1, or 2.

In a more preferred embodiment of the present invention, R₂ is selected from methyl or methoxy, and y is 1.

In a more preferred embodiment of the present invention, M₂ is N, and M₃ is CH.

In a more preferred embodiment of the present invention, R_{b} is selected from C₁₋₃ alkyl, 3- to 6-membered cycloalkyl, or 5- to 6-membered heteroaryl containing 1-3 heteroatoms selected from N, O, or S, wherein optionally the 3- to 6-membered cycloalkyl or the 5- to 6-membered heteroaryl containing 1-3 heteroatoms selected from N, O, or S is further substituted with halogen, hydroxyl, CN, or C₁₋₃ alkyl.

In a more preferred embodiment of the present invention, q, r, s, and t are all 1.

In a more preferred embodiment of the present invention, the general formula (I) is further a compound as represented by general formulas (VI-k)-(VI-m), or a stereoisomer or a pharmaceutically acceptable salt thereof: 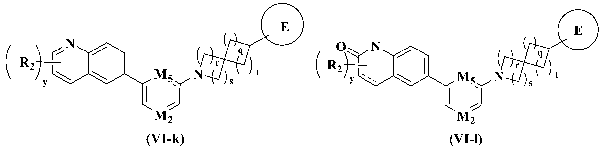 or 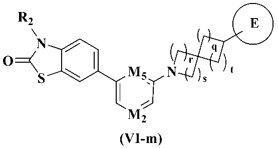 wherein
M₂ is selected from N or CH;
M₅ is selected from N or CH;
R₂ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
ring E is selected from 3- to 10-membered cycloalkyl, or 4- to 10-membered heterocyclyl, preferably 5- to 10-membered nitrogen-containing heterocyclyl, which is optionally further substituted with halogen, hydroxyl, amino, CN, C₁₋₃ alkyl, C₁₋₃ deuteroalkyl, C₁₋₃ haloalkyl, C₁₋₃ alkoxy, C₁₋₃ haloalkoxy, C₁₋₃hydroxyalkyl or 3- to 6-membered cycloalkyl;
q, r, s, and t are each independently selected from 1 or 2; and
y is independently selected from 0, 1, or 2.

In a more preferred embodiment of the present invention, the general formula (I) is further a compound as represented by general formula (VII-a), (VII-b), (VII-c) or (VII-d), or a stereoisomer or a pharmaceutically acceptable salt thereof: 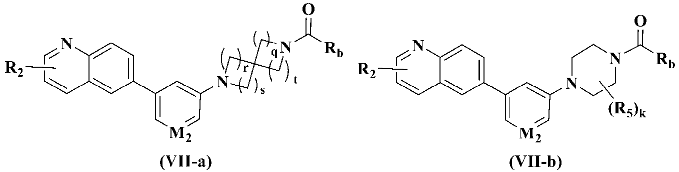 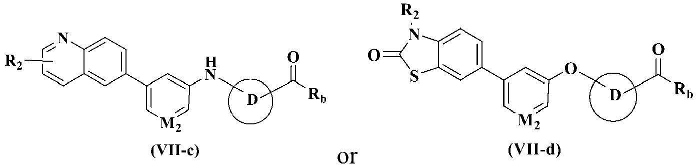 wherein
M₂ is selected from N or CH;
R₂ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
each R_{b} is independently selected from hydrogen, deuterium, C₁₋₃ alkyl, C₁₋₃ deuteroalkyl, C₁₋₃ haloalkyl, C₁₋₃ alkoxy, C₁₋₃ haloalkoxy, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S is further substituted with C₁₋₃ alkyl, C₁₋₃ deuteroalkyl, C₁₋₃haloalkyl, C₁₋₃ alkoxy, C₁₋₃ haloalkoxy, C₁₋₃ hydroxyalkyl, or 3- to 6-membered cycloalkyl;
each R₅ is independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
or any two R₅, together with adjacent carbon atoms, form 3- to 8-membered cycloalkyl or 3- to 8-membered heterocyclyl;
ring D is selected from 4- to 8-membered heterocyclyl, preferably 4- to 6-membered nitrogen-containing heterocyclyl;
q, r, s, and t are each independently selected from 1 or 2; and
k is independently selected from 1, 2, or 3.

In a more preferred embodiment of the present invention, q, r, s, and t are 1.

In a more preferred embodiment of the present invention, ring D is 4- to 6-membered heterocyclyl containing 1-2 nitrogen atoms.

In another aspect, the present invention provides a compound as represented by general formula (X-a) or (X-b), or a stereoisomer or a pharmaceutically acceptable salt thereof: 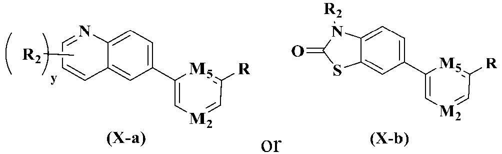
M₂ or M₅ is each independently selected from N or CH;
each R is independently selected from hydrogen, 3- to 10-membered cycloalkyl, 4- to 10-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, or 4- to 8-membered heterocyclylthio;
each R₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ deuteroalkoxy, C₁₋₆ hydroxyalkyl, 3- to 8-membered cycloalkyl, 3- to 8-membered cycloalkyloxy, 3- to 8-membered cycloalkylamino, C₆₋₁₀ aryl, 5- to 6-membered heteroaryl containing 1-3 heteroatoms selected from N, O, or S or 4- to 8-membered heterocyclyl containing 1-3 moieties selected from C(O), N, O, or S, -NRₐR_{b}, -NRₐC(O)R_{b}, or - C(O)NRₐR_{b};
y is selected from 1, 2 or 3.

In a more preferred embodiment of the present invention, each R is independently selected from hydrogen, 3- to 10-membered cycloalkyl, 4- to 8-membered heterocyclyl containing 1-3 moieties selected from N, O, S, SO₂ or SONH, 3- to 8-membered cycloalkyloxy, 3- to 8-membered cycloalkyl mercapto, 3-to 8-membered cycloalkylamino, 4- to 8-membered heterocyclyloxy containing 1-3 moieties selected from N, O, S, SO₂ or SONH, 4- to 8-membered heterocyclylamino containing 1-3 moieties selected from N, O, S, SO₂ or SONH, or 4- to 8-membered heterocyclyl mercapto containing 1-3 moieties selected from N, O, S, SO₂ or SONH.

In a more preferred embodiment of the present invention, each R is independently selected from hydrogen, 3- to 8-membered cycloalkyl, 7- to 8-membered heterocyclyl containing 1-3 moieties selected from N, O, S, SO₂ or SONH, 3- to 6-membered cycloalkyloxy, 3- to 6-membered cycloalkyl mercapto, 3-to 6-membered cycloalkylamino, 4- to 6-membered heterocyclyloxy containing 1-3 moieties selected from N, O, S, SO₂ or SONH, 4- to 6-membered heterocyclylamino containing 1-3 moieties selected from N, O, S, SO₂ or SONH, or 4- to 6-membered heterocyclyl mercapto containing 1-3 moieties selected from N, O, S, SO₂ or SONH.

In a more preferred embodiment of the present invention, each R₂ is independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy.

In another aspect, the present invention provides a compound as represented by general formula (V-a-4) or (V-b-3), or a stereoisomer or a pharmaceutically acceptable salt thereof: 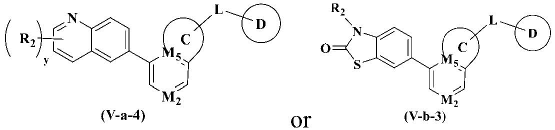 wherein M₂, M₅, L, R₂, ring C, ring D or y is as defined for general formula (V-a) or (V-b).

In another aspect, the present invention further relates to a pharmaceutical composition comprising a therapeutically effective dose of any compound as represented by the general formula, or a stereoisomer or a pharmaceutically acceptable salt thereof, and one or more pharmaceutically acceptable carriers, diluents, or excipients.

In certain embodiments of the present invention, the weight percentage of the compound, or the stereoisomer or the pharmaceutically acceptable salt thereof in the pharmaceutical composition is 0.1%-95%, preferably 5%-70%, e.g., 70%, 65%, 60%, 55%, 50%, 45%, 40%, 35%, 30%, 25%, 20%, 15%, 10%, or 5% on a free base basis.

In certain embodiments of the present invention, the pharmaceutical composition is selected from tablets, capsules, a liquid preparation, or an injection, preferably further comprises a filler, optionally further comprises a disintegrant, or further comprises one or more of a glidant or a lubricant.

In certain embodiments of the present invention, the pharmaceutical composition is an immediate-release preparation or a sustained-release preparation.

In certain embodiments of the present invention, the unit dose of the compound, or the stereoisomer or the pharmaceutically acceptable salt thereof in the pharmaceutical composition is 1-1000 mg, preferably 1-500 mg, or preferably 1 mg, 2 mg, 3 mg, 5 mg, 10 mg, 20 mg, 40 mg, 50 mg, 60 mg, 80 mg, 100 mg, 200 mg, 300 mg, 400 mg, or 500 mg on a free base basis.

In certain embodiments of the present invention, the compound, or the stereoisomer or the pharmaceutically acceptable salt thereof can be administered by any convenient method, for example, by oral, parenteral, buccal, sublingual, nasal, rectal, intrathecal or transdermal administration, and the pharmaceutical composition can be adjusted accordingly.

In certain embodiments of the present invention, the compound, or the stereoisomer or the pharmaceutically acceptable salt thereof can be formulated into a liquid or solid preparation, such as a syrup, a suspension, an emulsion, tablets, capsules, powder, granules, or a lozenge.

In another aspect, the present invention further relates to the use of any compound as represented by the general formula, or the stereoisomer or the pharmaceutically acceptable salt thereof, or the pharmaceutical composition in the preparation of a drug for treating a disease related to CYP11B2.

The present invention further relates to the use of the compound as represented by any general formula shown, or the stereoisomer or the pharmaceutically acceptable salt thereof, or the pharmaceutical composition thereof in the preparation of a drug for treating or preventing chronic kidney disease, kidney or cardiac fibrosis, diabetic nephropathy, congestive heart failure, hypertension, primary aldosteronism, and Cushing's syndrome.

In a preferred embodiment of the present invention, the hypertension is refractory hypertension.

In a preferred embodiment of the present invention, the chronic kidney disease is chronic kidney disease with type II diabetes.

In some embodiments, the EC₅₀ value of the compound of the present invention for CYP11B2 is between 0.0001 µM and 50 µM; preferably, the EC₅₀ value of the compound for CYP11B2 is between 0.0001 µM and 10 µM; more preferably, the EC₅₀ value of the compound for CYP11B2 is between 0.0001 µM and 1 µM; and further preferably, the EC₅₀ value of the compound for CYP11B2 is between 0.0001 µM and 0.1 µM.

In addition, the compound of the present invention has a relatively good selectivity for CYP11B1, and the selectivity is greater than 50, preferably greater than 100, more preferably greater than 200, further preferably greater than 500.

In another aspect, the present invention provides a method for preparing a compound as represented by formula (V-a-3) or (V-b-2), or a stereoisomer or a pharmaceutically acceptable salt thereof, comprising the following steps: 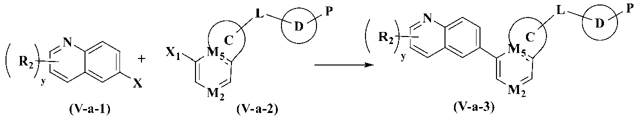
performing a coupling reaction between formula (V-a-1) and formula (V-a-2) to prepare a compound as represented by formula (V-a-3), or a stereoisomer or a pharmaceutically acceptable salt thereof; optionally, performing a deprotection on formula (V-a-3) to obtain a compound as represented by formula (V-a-4), or a stereoisomer or a pharmaceutically acceptable salt thereof; 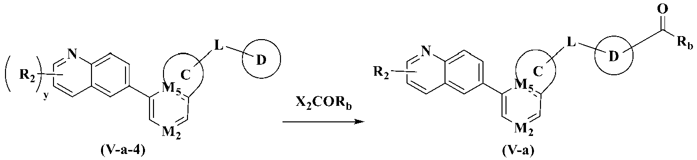
optionally, using formula (V-a-4) and X₂COR_{b} to prepare a compound as represented by general formula (V-a), or a stereoisomer or a pharmaceutically acceptable salt thereof; or 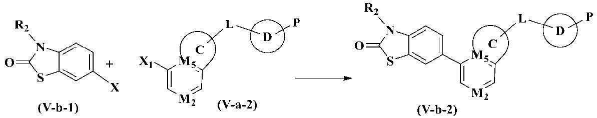
performing a coupling reaction between formula (V-b-1) and formula (V-a-2) to prepare a compound as represented by formula (V-b-2), or a stereoisomer or a pharmaceutically acceptable salt thereof; optionally, performing a deprotection to obtain a compound as represented by formula (V-b-3), or a stereoisomer or a pharmaceutically acceptable salt thereof; 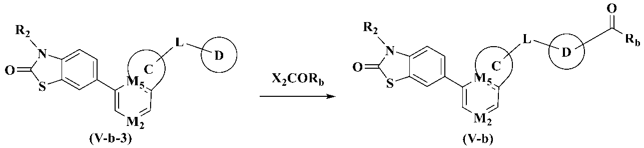
optionally, using formula (V-b-3) and X₂COR_{b} to prepare a compound as represented by formula (V-b), or a stereoisomer or a pharmaceutically acceptable salt thereof;
wherein
X is boranyl; X₁ is halogen; X₂ is halogen or hydroxy; P is an amino protecting group; and
M₂, M₃, L, R₂, R_{b}, r, q, s or t is as defined for general formula (V-a) or (V-b).

In another aspect, the present invention provides a method for preparing a compound as represented by general formula (V-a) or (V-b), or a stereoisomer or a pharmaceutically acceptable salt thereof, comprising the following steps: 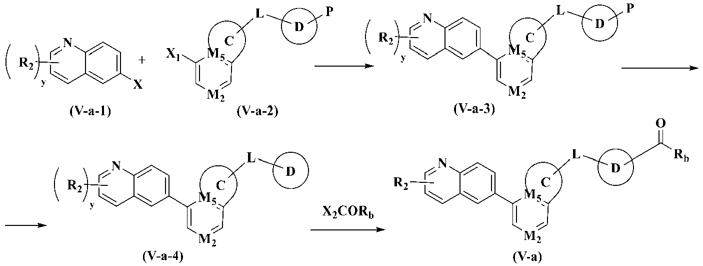
performing a coupling reaction between formula (V-a-1) and formula (V-a-2) to prepare formula (V-a-3), which is deprotected to obtain formula (V-a-4), followed by reaction of the formula (V-a-4) with X₂COR_{b} to prepare general formula (V-a), optionally followed by further preparation of a stereoisomer or a pharmaceutically acceptable salt thereof; or 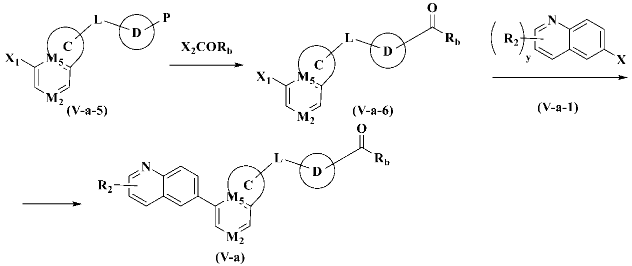
using formula (V-a-5) and X₂COR_{b} to prepare general formula (V-a-6), which is further subjected to coupling reaction with formula (V-a-1) to prepare formula (V-a), optionally followed by further preparation of a stereoisomer or a pharmaceutically acceptable salt thereof; or 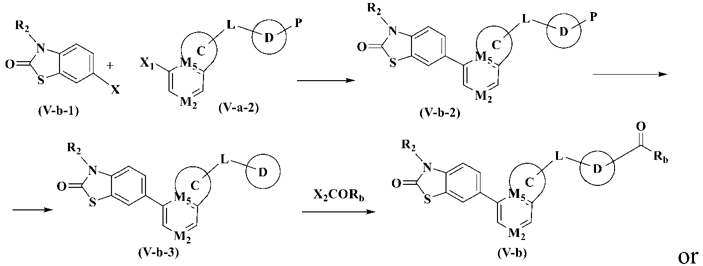
performing a coupling reaction between formula (V-b-1) and formula (V-a-2) to prepare formula (V-b-2), which is deprotected to obtain formula (V-b-2), followed by reaction of the formula (V-b-2) with X₂COR_{b} to prepare general formula (V-b), optionally followed by further preparation of a stereoisomer or a pharmaceutically acceptable salt thereof; or 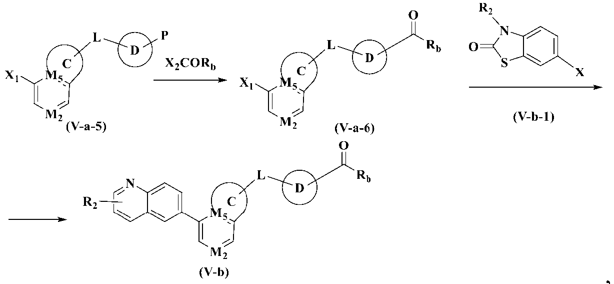
using formula (V-a-5) and X₂COR_{b} to prepare general formula (V-a-6), which is further subjected to coupling reaction with formula (V-b-1) to prepare formula (V-b), optionally followed by further preparation of a stereoisomer or a pharmaceutically acceptable salt thereof;
wherein
X is boranyl; X₁ is halogen; X₂ is halogen or hydroxy; P is an amino protecting group; and
M₂, M₅, L, R₂, R_{b}, ring C, ring D and y are as defined for general formula (V-a) or (V-b).

In a preferred embodiment of the present invention, the boranyl is dioxaborolanyl; X₁ is chloro or bromo; X₂ is chloro, bromo or hydroxy; and P is a Boc protecting group.

In a preferred embodiment of the present invention, there is provided a method for preparing a compound as represented by general formula (VI-a), (VI-c) or (VI-d), or a stereoisomer or pharmaceutically acceptable salt thereof, comprising the following steps: 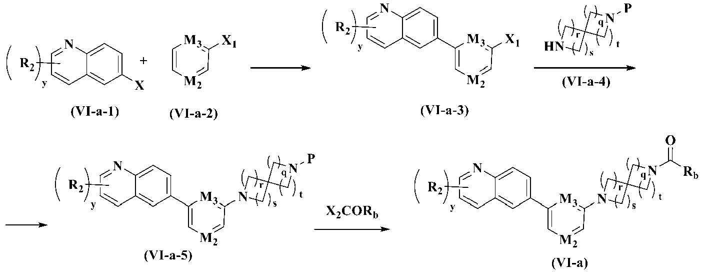
performing a coupling reaction between formula (VI-a-1) and formula (VI-a-2) to prepare formula (VI-a-3), which is further reacted with formula (VI-a-4) to obtain formula (VI-a-5), followed by deprotection of the formula (VI-a-5) and reaction of the resultant with X₂COR_{b} to prepare general formula (VI-a), optionally followed by further preparation of a stereoisomer or a pharmaceutically acceptable salt thereof; or 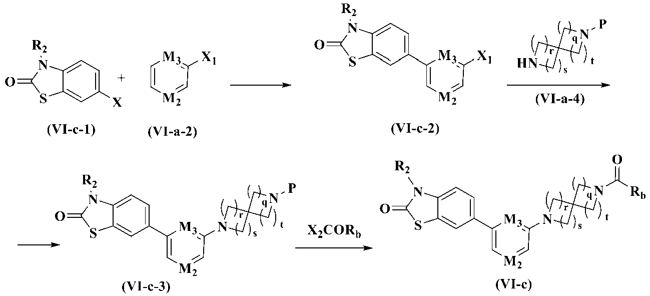
performing a coupling reaction between formula (VI-c-1) and formula (VI-a-2) to prepare formula (VI-c-2), which is further reacted with formula (VI-a-4) to obtain formula (VI-c-3), followed by deprotection of the formula (VI-c-3) and reaction of the resultant with X₂COR_{b} to prepare general formula (VI-c), optionally followed by further preparation of a stereoisomer or a pharmaceutically acceptable salt thereof; or 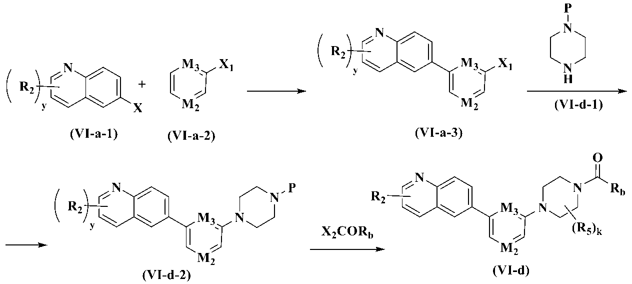
performing a coupling reaction between formula (VI-a-1) and formula (VI-a-2) to prepare formula (VI-a-3), which is further reacted with formula (VI-d-1) to obtain formula (VI-d-2), followed by deprotection of the formula (VI-d-2) and reaction of the resultant with X₂COR_{b} to prepare general formula (VI-a), optionally followed by further preparation of a stereoisomer or a pharmaceutically acceptable salt thereof;
wherein
X is boranyl; X₁ is halogen; X₂ is halogen or hydroxy; P is an amino protecting group; and
M₂, M₃, L, R₂, R_{b}, y, r, q, s or t is as defined for general formula (VI-a), (VI-c) or (VI-d).

### Detailed Description of the Invention

Unless stated to the contrary, the terms used in the description and claims have the following meanings.

The term "alkyl" refers to a saturated aliphatic hydrocarbon group, which is a linear or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted, and when substituted, the substituent can be substituted at any available connection point, and the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group. In the present invention, alkyl is preferably methyl, ethyl, isopropyl, tert-butyl, haloalkyl, deuteroalkyl, alkoxy-substituted alkyl, and hydroxyl-substituted alkyl.

The term "alkylene" refers to one hydrogen atom of alkyl being further substituted, for example: "methylene" refers to -CH₂-, "ethylene" refers to -(CH₂)₂-, "propylene" refers to -(CH₂)₃-, and "butylene" refers to -(CH₂)₄-, etc. The term "alkenyl" refers to an alkyl group as defined above consisting of at least two carbon atoms and at least one carbon-carbon double bond, such as ethenyl, 1-propenyl, 2-propenyl, 1-, 2- or 3 -butenyl, etc. Alkenyl can be substituted or unsubstituted. When the alkenyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "cycloalkyl" refers to a saturated or partially unsaturated monocyclic or polycyclic cyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spiro, fused and bridged cycloalkyl, preferably cyclopropyl, cyclobutyl, cyclohexyl, cyclopentyl, and cycloheptyl.

The term "spirocycloalkyl" refers to a polycyclic group with 5 to 20 membered monocyclic rings sharing one carbon atom (called a spiro atom). It may contain one or more double bonds, but no ring has a completely conjugated π electron system. Preferably, the group is 6- to 14-membered, and more preferably 7- to 10-membered. According to the number of shared spiro atoms between the rings, the spirocycloalkyl is divided into monospirocycloalkyl, bispirocycloalkyl or polyspirocycloalkyl, preferably monospirocycloalkyl and bispirocycloalkyl. More preferably, the group is a 3-membered/6-membered, 3-membered/5-membered, 4-membered/4-membered, 4-membered/5-membered, 4-membered/6-membered, 5-membered/5-membered or 5-membered/6-membered monospirocycloalkyl. Non-limiting examples of spirocycloalkyl include: 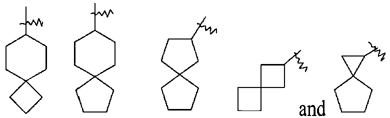 etc.;
and also include spirocycloalkyl in which monospirocycloalkyl and heterocycloalkyl share a spiro atom. Non-limiting examples include: 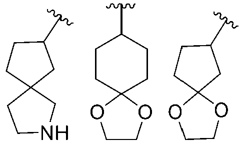 etc.

The term "fused cycloalkyl" refers to a 5- to 20-membered all-carbon polycyclic group in which each ring in the system shares an adjacent pair of carbon atoms with other rings in the system, one or more ring of which may contain one or more double bonds, but no ring has a fully conjugated π electron system. Preferably, the group is 6- to 14-membered, and more preferably 7- to 10-membered. According to the number of constituent rings, it can be divided into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include: 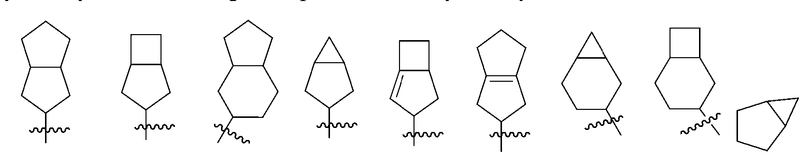 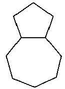 etc.

The term "bridged cycloalkyl" refers to a 5- to 20-membered all-carbon polycyclic group with any two rings sharing two carbon atoms that are not directly connected. It may contain one or more double bonds, but no ring has a fully conjugated π electron system. Preferably, the group is 6- to 14-membered, and more preferably 7- to 10-membered. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

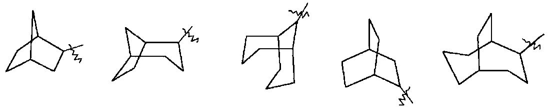

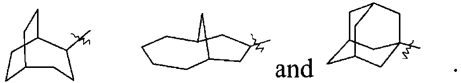

The cycloalkyl ring can be fused to an aryl, heteroaryl or heterocycloalkyl ring, wherein the ring connected to the parent structure is cycloalkyl, and non-limiting examples include indanyl, tetrahydronaphthyl, benzocycloheptanyl, etc. Cycloalkyl can be optionally substituted or unsubstituted. When the cycloalkyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen or S(O)ₘ (wherein m is an integer of 0 to 2), but excluding ring moieties of -O-O-, -O-S- or -S-S-, and the remaining ring atoms are carbon. Preferably, the heterocyclyl comprises 3 to 12 ring atoms, among which 1-4 are heteroatoms; more preferably, the heterocyclyl comprises 3 to 8 ring atoms; most preferably, the heterocyclyl comprises 3 to 8 ring atoms; further preferably, the heterocyclyl is a 4-to -8-membered heterocyclyl group containing 1-3 nitrogen atoms, which is optionally substituted with 1-2 oxygen atoms, sulfur atoms, or oxo groups, including nitrogen-containing monocyclic heterocyclyl, nitrogen-containing spiroheterocyclyl, or nitrogen-containing fused heterocyclyl.

Non-limiting examples of monocyclic heterocyclyl include azetidine, pyrrolidinyl, imidazolidinyl, tetrahydrofuryl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl, azepinyl, 1,4-diazacycloheptyl, pyranyl, etc., preferably pyrrolidinyl, morpholinyl, piperidinyl, azepinyl, 1,4-diazacycloheptyl, and piperazinyl. Polycyclic heterocyclyl includes spiro, fused and bridged heterocyclyl, wherein the spiro, fused and bridged heterocyclyl are optionally connected to other groups via a single bond, or further fused to other cycloalkyl, heterocyclyl, aryl and heteroaryl via any two or more atoms on the ring.

The term "spiroheterocyclyl" refers to 5- to 20-membered polycyclic heterocyclyl with monocyclic rings sharing one atom (called a spiro atom), wherein one or more ring atoms are heteroatoms selected from nitrogen, oxygen, or S(O)ₘ (wherein m is an integer of 0 to 2), and the remaining ring atoms are carbon. The term may contain one or more double bonds, and none of the rings has a fully conjugated π electron system. Preferably, the group is 6- to 14-membered, and more preferably 7- to 10-membered. According to the number of shared spiro atoms between the rings, the spiroheterocyclyl is divided into monospiroheterocyclyl, bispiroheterocyclyl, or polyspiroheterocyclyl, preferably monospiroheterocyclyl and bispiroheterocyclyl. More preferably, it is a 3-membered/5-membered, 3-membered/6-membered, 4-membered/4-membered, 4-membered/5-membered, 4-membered/6-membered, 5-membered/5-membered, or 5-membered/6-membered monospiroheterocyclyl. Non-limiting examples of spiroheterocyclyl include: 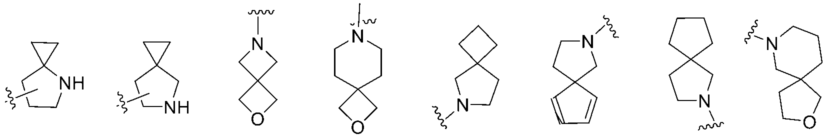 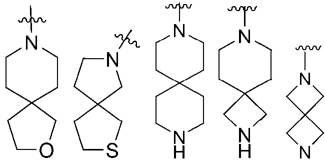 etc.

The term "fused heterocyclyl" refers to 5- to 20-membered polycyclic heterocyclyl with each ring in the system sharing a pair of atoms neighboring other rings in the system, and one or more rings may contain one or more double bonds, and none of the rings has a fully conjugated π electron system, wherein one or more ring atoms are heteroatoms selected from nitrogen, oxygen, or S(O)ₘ (wherein m is an integer of 0 to 2), and the remaining ring atoms are carbon. Preferably, the group is 6- to 14-membered, and more preferably 7- to 10-membered. According to the number of constituent rings, it can be divided into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include: 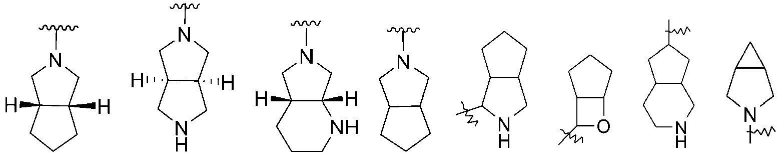 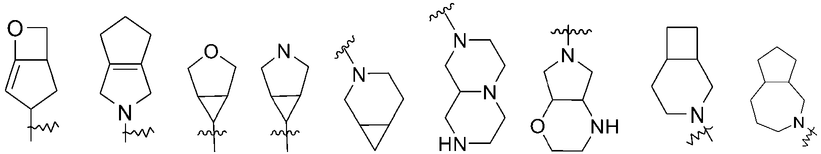 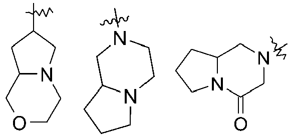 etc.

The term "bridged heterocyclyl" refers to a 5- to 14-membered polycyclic heterocyclyl with any two rings sharing two atoms that are not directly connected. It may contain one or more double bonds, and none of the rings has a fully conjugated π electron system, wherein one or more ring atoms are heteroatoms selected from nitrogen, oxygen, or S(O)ₘ (wherein m is an integer of 0 to 2), and the remaining ring atoms are carbon. Preferably, the group is 6- to 14-membered, and more preferably 7- to 10-membered. According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include: 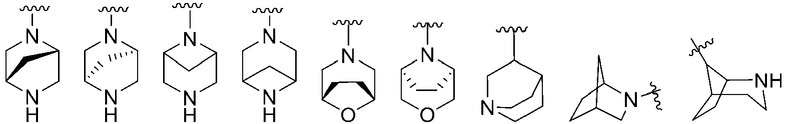 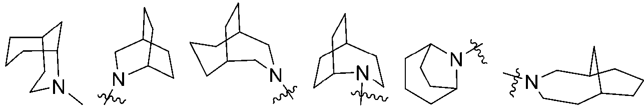 etc.

The heterocyclyl ring can be fused to an aryl, heteroaryl or cycloalkyl ring, wherein the ring connected to the parent structure is heterocyclyl, and the non-limiting examples thereof include: 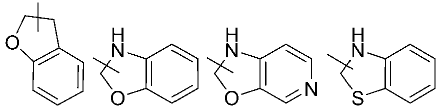 etc.

Heterocyclyl can be optionally substituted or unsubstituted. When the heterocyclyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "aryl" refers to a 6- to 14-membered all-carbon monocyclic or fused polycyclic (i.e., rings sharing an adjacent pair of carbon atoms) group having a conjugated π electron system, preferably 6- to 12-membered aryl, such as phenyl and naphthyl, and more preferably phenyl. The aryl ring can be fused to heteroaryl, heterocyclyl or cycloalkyl ring, including benzo 5- to -10-membered heteroaryl, benzo 3- to -8-membered cycloalkyl and benzo 3- to -8-membered heteroalkyl, preferably benzo 5- to -6-membered heteroaryl, benzo 3- to -6-membered cycloalkyl, and benzo 3- to -6-membered heteroalkyl, wherein the heterocyclyl is heterocyclyl containing 1-3 nitrogen atoms, oxygen atoms, or sulfur atoms; or further including a three-membered nitrogen-containing fused ring containing a benzene ring,
wherein the ring connected to the parent structure is an aryl ring, and the non-limiting examples thereof include: 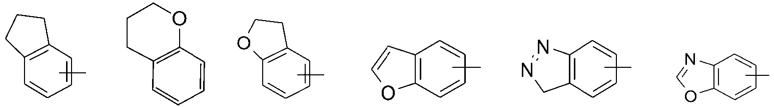 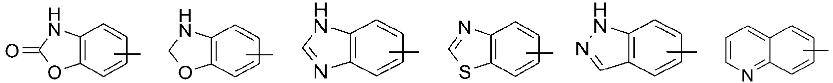 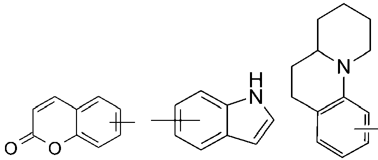 etc.

Aryl can be substituted or unsubstituted. When the aryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "heteroaryl" refers to a heteroaromatic system containing 1 to 4 heteroatoms and 5 to 14 ring atoms, wherein the heteroatoms are selected from oxygen, sulfur, and nitrogen. The heteroaryl is preferably 5- to 12-membered, more preferably 5-membered or 6-membered, such as imidazolyl, furyl, thienyl, thiazolyl, pyrazolyl, oxazolyl, pyrrolyl, triazolyl, tetrazolyl, pyridyl, pyrimidinyl, thiadiazole, pyrazinyl, etc., preferably triazolyl, thienyl, imidazolyl, pyrazolyl, oxazolyl, pyrimidinyl, or thiazolyl; more preferably, pyrazolyl, pyrrolyl and oxazolyl. The heteroaryl ring can be fused to an aryl, heterocyclyl, or cycloalkyl ring, wherein the ring connected to the parent structure is heteroaryl ring, and the non-limiting examples thereof include: 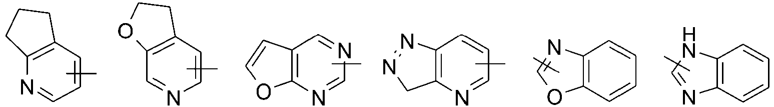 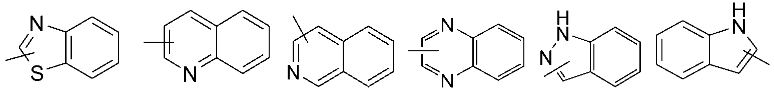 etc.

Heteroaryl can be optionally substituted or unsubstituted. When the heteroaryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropoxy, cyclobutoxy, cyclopentoxy, and cyclohexoxy. Alkoxy can be optionally substituted or unsubstituted. When the alkoxy is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Haloalkyl" refers to alkyl substituted with one or more halogen, wherein the alkyl is defined as above.

"Haloalkoxy" refers to alkoxy substituted with one or more halogen, wherein the alkoxy is defined as above.

"Hydroxyalkyl" refers to alkyl substituted with hydroxyl, wherein the alkyl is defined as above.

"Alkenyl" refers to an alkene group, also known as an olefinic group, which is a linear or branched unsaturated aliphatic hydrocarbon group containing at least one carbon-carbon double bond that can be located at any position within the alkenyl group. Alkenyl is a linear or branched unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), 2 to 4 (C₂₋₄), or 2 to 3 (C₂₋₃) carbon atoms. Non-limiting examples of alkenyl include: 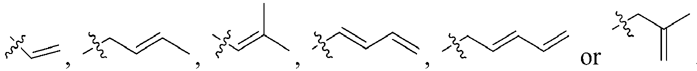 . The alkenyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Alkynyl" refers to (CH≡C-), which contains at least one carbon-carbon triple bond that can be located any position in the alkynyl and contains at least one carbon-carbon double bond that can be located at any position within the alkenyl group. The alkynyl is a linear or branched unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), 2 to 4 (C₂₋₄), or 2 to 3 (C₂₋₃) carbon atoms. Non-limiting examples of alkynyl include: 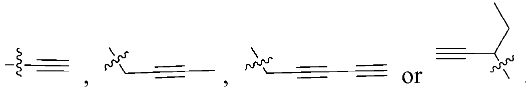 . The alkynyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "alkenylcarbonyl" refers to -C(O)-(alkenyl), wherein the alkenyl is defined as above. Non-limiting examples of alkenylcarbonyl include: ethenylcarbonyl, propenylcarbonyl, and butenylcarbonyl. Alkenylcarbonyl can be optionally substituted or unsubstituted. When the alkenylcarbonyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

" 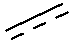 " refers to a single bond or a double bond.

"Hydroxyl" refers to an -OH group.

"Halogen" refers to fluorine, chlorine, bromine, or iodine.

"Amino" refers to -NH₂.

"Cyano" refers to -CN.

"Nitro" refers to -NO₂.

"Carbonyl" refers to -C(O)-.

"Carboxyl" refers to -C(O)OH.

"THF" refers to tetrahydrofuran.

"EtOAc" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"MeCN" refers to acetonitrile.

"DMA" refers to N,N-dimethylacetamide.

"Et₂O" refers to diethyl ether.

"DCE" refers to 1,2 dichloroethane.

"DIPEA" refers to N,N-diisopropylethylamine.

"NBS" refers to N-bromosuccinimide.

"NIS" refers to N-iodosuccinimide.

Different terms such as "X is selected from A, B, or C", "X is selected from A, B and C", "X is A, B or C", and "X is A, B and C" all express the same meaning, that is, X can be any one or more of A, B, and C.

Any formula or structure provided in the present invention is also intended to represent both the unlabeled form and the isotopically labeled form of the compound. The isotopically labeled compound has the structure described by the general formula or a specific compound provided in the present invention, except that one or more atoms are replaced with atoms with selected atomic masses or mass numbers. Examples of isotopes of the compounds of the present invention include isotopes of hydrogen, carbon, nitrogen, oxygen, phosphorus, fluorine, and chlorine.

"Optional" or "optionally" means that the event or circumstance subsequently described may but need not to occur, and the description includes the occasion where the event or circumstance occurs or does not occur. For example, "heterocyclyl optionally substituted with alkyl" means the alkyl may be present but be not necessarily present, and the description includes the case where the heterocyclyl is substituted with alkyl and the case where the heterocyclyl is not substituted with alkyl.

"Substituted" means that one or more hydrogen atoms, preferably at most 5, more preferably 1-3 hydrogen atoms in the group are each independently substituted with a corresponding number of substituents. It goes without saying, the substituents may be only in possible chemical positions thereof, and those skilled in the art can determine the possible or impossible substitutions (by experiment or theory) without paying too much effort. For example, an amino or hydroxyl group having a free hydrogen may be unstable when combined with the carbon atoms having an unsaturated (e.g., olefinic) bond.

"Pharmaceutical composition" denotes a mixture containing one or more compounds described herein or physiologically/pharmaceutically acceptable salts or prodrug thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

"Pharmaceutically acceptable salts" and "pharmaceutical salts" refer to salts of the compounds of the present invention, which are safe and effective when used in mammals, and have appropriate biological activity.

### Detailed Description of Embodiments

### Example

The structures of compounds are determined by nuclear magnetic resonance (NMR) or/and mass spectrometry (MS). The NMR shift (δ) is given in 10⁻⁶ (ppm). NMR was determined using a Bruker AVANCE-400 nuclear magnetic instrument. The solvents for determination were deuterated dimethyl sulfoxide (DMSO-*d₆*), deuterated chloroform (CDCl₃), and deuterated methanol (CD₃OD), and the internal standard was tetramethylsilane (TMS).

For MS determination, FINNIGAN LCQAd (ESI) mass spectrometer (manufacturer: Thermo, model: Finnigan LCQ advantage MAX) is used.

For HPLC determination, Agilent 1200DAD high-pressure liquid chromatograph instrument (Sunfire C18 150 × 4.6 mm chromatographic column) and Waters 2695-2996 high-pressure liquid chromatograph instrument (Gimini C18 150 × 4.6 mm chromatographic column) are used.

The average kinase inhibition rate and IC₅₀ value were measured using NovoStar microplate reader (BMG Company, Germany).

Yantai Huanghai HSGF254 or Qingdao GF254 silica gel plate is used as a thin layer chromatography silica plate, and the silica gel plate for thin layer chromatography (TLC) has a specification of 0.15 mm-0.2 mm, and the specification when separating and purifying a product by thin layer chromatography is 0.4 mm-0.5 mm.

For column chromatography, Yantai Huanghai silica gel of 200-300 mesh silica gel is generally used as a carrier.

The known starting materials of the present invention can be synthesized by methods known in the art, or can be purchased from ABCR GmbH & Co. KG, Acros Organics, Aldrich Chemical Company, Accela ChemBio Inc., Chembee Chemicals Inc. and other companies.

Unless otherwise specially specified in examples, reactions can all be carried out in an argon atmosphere or a nitrogen atmosphere.

Argon atmosphere or nitrogen atmosphere means that a reaction flask is connected to an argon or nitrogen balloon with a volume of about 1 L.

The hydrogen atmosphere means that a reaction flask is connected to a hydrogen balloon with a volume of about 1 L.

In a pressurized hydrogenation reaction, Parr 3916EKX hydrogenator and Qinglan QL-500 hydrogen generator or HC2-SS hydrogenator are used.

For a hydrogenation reaction, evacuation to a vacuum and filling with hydrogen are usually carried out, and the operation is repeated three times.

For a microwave reaction, CEM Discover-S 908860 microwave reactor is usually used.

Unless otherwise specially specified in examples, a solution refers to an aqueous solution.

Unless otherwise specially specified in examples, the reaction temperature is room temperature, which is 20-30°C.

The progress of a reaction in the examples is monitored by thin layer chromatography (TLC). Developing agent systems used in the reaction include: A: dichloromethane and methanol system, B: n-hexane and ethyl acetate system, and C: petroleum ether and ethyl acetate system, D: acetone, the volume ratio of the solvents is adjusted according to the polarity of the compounds.

Eluent systems for column chromatography and developing agent systems for thin layer chromatography, which are used to purify compounds, include: A: dichloromethane and methanol system, B: n-hexane and ethyl acetate system, and C: dichloromethane and acetone system, wherein the volume ratio between the solvents can be adjusted depending on the polarity of the compound, or can also be adjusted by adding a small amount of basic or acidic reagents such as triethylamine and acetic acid.

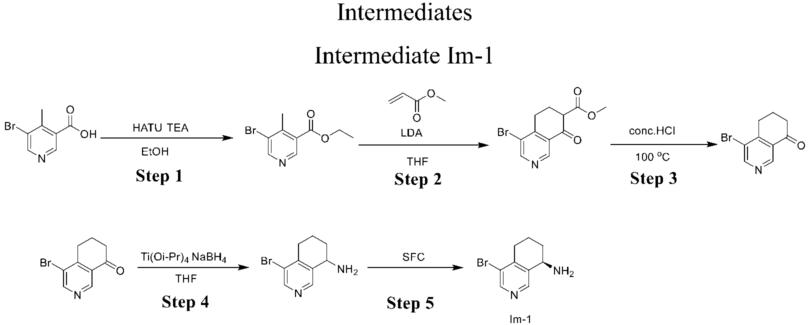

### Step 1. Ethyl 5-bromo-4-methyl-pyridine-3-carboxylate

5-Bromo-4-methyl-pyridine-3-carboxylic acid (20 g, 92.58 mmol) was dissolved in anhydrous N,N-dimethylformamide (200 mL), and anhydrous ethanol (42.65 g, 925.79 mmol, 54.06 mL), HATU (52.39 g, 138.87 mmol), and TEA (28.10 g, 277.74 mmol, 38.74 mL) were separately added thereto. The reaction system was stirred at room temperature 20°C for 16 hours. After the reaction was complete, the reaction was quenched by adding water (50 mL), and the mixture was extracted with ethyl acetate (100 mL x 3), washed with a saturated aqueous sodium chloride solution (100 mL x 2), dried over anhydrous sodium sulfate, filtered, and concentrated to obtain crude ethyl 5-bromo-4-methyl-pyridine-3-carboxylate (21 g, crude).

MS m/z (ESI): 244.0, 246.0 [M+1].

### Step 2. Methyl 4-bromo-8-carbonyl-6,7-dihydro-5H-isoquinoline-7-carboxylate

Ethyl 5-bromo-4-methyl-pyridine-3-carboxylate (7 g, 28.68 mmol) was dissolved in anhydrous tetrahydrofuran (200 mL), lithium diisopropylamide (2 M, 17.21 mL) was dropwise added thereto at -78°C, stirring was maintained at -78°C for 1 hour, and methyl acrylate (6.17 g, 71.70 mmol, 6.46 mL) was then slowly added thereto. The reaction system was naturally warmed to room temperature and stirring was continued for 5 hours. After the reaction was complete, the reaction was quenched by slowly dropwise adding water (20 mL) thereto, extracted with ethyl acetate (50 mL x 3), washed with a saturated aqueous sodium chloride solution (50 mL x 2), dried over anhydrous sodium sulfate, filtered, and concentrated to obtain a crude product. The crude product was purified by a column (PE/EtOAc = 3:1, uv = 254 nm) to finally obtain the product methyl 4-bromo-8-carbonyl-6,7-dihydro-5H-isoquinoline-7-carboxylate (4 g, 14.0 mmol, 48.8% yield).

MS m/z (ESI): 284.0, 286.0 [M+1].

### Step 3. 4-Bromo-6,7-dihydro-5H-isoquinolin-8-one

Methyl 4-bromo-8-carbonyl-6,7-dihydro-5H-isoquinoline-7-carboxylate (7 g, 24.64 mmol) was dissolved in hydrochloric acid (6 M) (30 mL). The reaction system was stirred in an oil bath at 100°C for 2 hours. After the reaction was complete, the mixture was cooled to room temperature, adjusted to pH 7-8 with a 6 M sodium hydroxide solution, washed with ethyl acetate (50 mL x 3) and a saturated aqueous sodium chloride solution (50 mL x 2), dried over anhydrous sodium sulfate, filtered, and concentrated to obtain crude 4-bromo-6,7-dihydro-5H-isoquinolin-8-one (4.8 g, 21.23 mmol, 86.17% yield). The crude product was directly used for the next reaction.

MS m/z (ESI): 226.0, 228.0 [M+1].

### Step 4. 4-Bromo-5,6,7,8-tetrahydroisoquinolin-8-amine

4-Bromo-6,7-dihydro-5H-isoquinolin-8-one (7 g, 30.96 mmol) was dissolved in a solution of ammonia in methanol (2 M, 100 mL), and tetraisopropyl titanate (17.60 g, 61.93 mmol, 18.33 mL) was added thereto. After the reaction system was stirred at 20°C for 16 hours, sodium borohydride (1.76 g, 46.45 mmol) was added thereto in portions in an ice-water bath. The reaction system was further stirred for 2 hours at room temperature. After the reaction was complete, the reaction was quenched by adding water (20 mL),
and the mixture was filtered through diatomite, extracted with ethyl acetate (50 mL x 3), washed with a saturated aqueous sodium chloride solution (50 mL x 2), dried over anhydrous sodium sulfate, filtered, and concentrated to obtain a crude product. The crude product was purified by a column (dichloromethane : methanol = 10:1, uv = 254 nm) to obtain 4-bromo-5,6,7,8-tetrahydroisoquinolin-8-amine (4.5 g, 19.82 mmol, 63.99% yield).

MS m/z (ESI): 227.0, 229.0 [M+1].

### Step 5. (R)-4-bromo-5,6,7,8-tetrahydroisoquinolin-8-amine

4-Bromo-5,6,7,8-tetrahydroisoquinolin-8-amine (4.5 g, 19.82 mmol) was subjected to the following chiral separation to obtain the products P1 and Im-1, respectively.

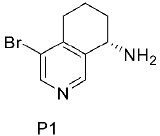

| Column type | Mobile phase | Flow rate | Detection wavelength | Tempe rature | Retention time of P1 | Retention time of Im-1 | Linear gradient |
|---|---|---|---|---|---|---|---|
| CHIRALPAK IB-H, 4.6 × 250 mm, 5 µm | Hexane: EtOH : MeOH = 70 : 15 : 15 | 1.0 ml/min | 214 nm | 35°C | 3.440 min | 3.740 min | Isometric |

MS m/z (ESI): 227.0, 229.0 [M+1].

### Example 1

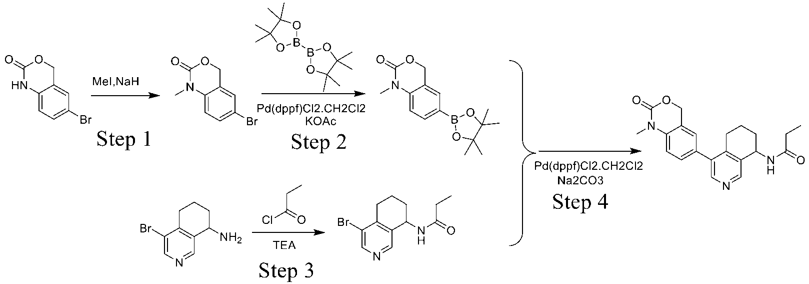

### Step 1

### 6-Bromo-1-methyl-1,4-dihydro-2H-benzo[d][1,3]oxazin-2-one

In a 25 mL reaction flask, 6-bromo-1,4-dihydro-2H-benzo[d][1,3]oxazin-2-one (500 mg, 2.19 mmol) and iodomethane (622.45 mg, 4.39 mmol) were dissolved in tetrahydrofuran (5 mL), sodium hydride (78.92 mg, 3.29 mmol, 60% purity) was then added at 0°C, and the reaction solution was stirred at 25°C for 10 hours. After the reaction was stopped, the reaction was quenched by adding water (5 mL), and the mixture was extracted with ethyl acetate (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product 6-bromo-1-methyl-1,4-dihydro-2H-benzo[d][1,3]oxazin-2-one (410 mg, a yellow solid) with a yield of 77.2%.

MS m/z (ESI): 242.0, 244.0 [M+1].

### Step 2

### 1-Methyl-6-(4,4,5,5-tetramethyl-1,3,2-dioxaborolan-2-yl)-1,4-dihydro-2H-benzo[d][1,3]oxazin-2-one

6-Bromo-1-methyl-1,4-dihydro-2H-benzo[d][1,3]oxazin-2-one (410 mg, 1.69 mmol), bis(pinacolato)diboron (860.2 mg, 3.39 mmol), palladium acetate (498.67 mg, 5.08 mmol), and [1,1'-bis(diphenylphosphino)ferrocene]palladium dichloride dichloromethane complex (68.65 mg, 84.69 µmol) were dissolved in dioxane (10 mL), and after displacement three times with nitrogen, the reaction solution was stirred at 90°C for 10 hours. After the reaction was stopped, the reaction solution was cooled to room temperature, filtered, and concentrated under reduced pressure, and the resulting residue was purified by silica gel column chromatography with petroleum ether and ethyl acetate as an eluent system to obtain the title product 1-methyl-6-(4,4,5,5-tetramethyl-1,3,2-dioxaborolan-2-yl)-1,4-dihydro-2H-benzo[d][1,3]oxazin-2-one (300 mg, yellow solid) with a yield of 61.2%.

MS m/z (ESI): 290.1 [M+1].

### Step 3

### N-(4-bromo-5,6,7,8-tetrahydroisoquinolin-8-yl)propanamide

In a 25 mL reaction flask, 4-bromo-5,6,7,8-tetrahydroisoquinolin-8-amine (500 mg, 2.20 mmol) and triethylamine (445.58 mg, 4.40 mmol) were dissolved in dichloromethane (5 mL), and propionyl chloride (224.07 mg, 2.42 mmol) was dropwise then added. The reaction solution was stirred at 25°C for 3 hours. After the reaction was stopped, the reaction was quenched by adding water (5 mL), and the mixture was extracted with dichloromethane (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product N-(4-bromo-5,6,7,8-tetrahydroisoquinolin-8-yl)propanamide (500 mg) with a yield of 80.2%.

MS m/z (ESI): 283.0, 285.0 [M+1].

### Step 4

### N-(4-(1-methyl-2-carbonyl-1,4-dihydro-2H-benzo[d][1,3]oxazin-6-yl)-5,6,7,8-tetrahydroisoquinolin-8-yl)propanamide

N-(4-bromo-5,6,7,8-tetrahydroisoquinolin-8-yl)propanamide (100 mg, 353.15 µmol), 1-methyl-6-(4,4,5,5-tetramethyl-1,3,2-dioxaborolan-2-yl)-1,4-dihydro-2H-benzo[d][1,3]oxazin-2-one (102.11 mg, 353.15 µmol), Na2CO3 (112.29 mg, 1.06 mmol), and [1,1'-bis(diphenylphosphino)ferrocene]palladium dichloride dichloromethane complex (14.31 mg, 17.66 µmol) were dissolved in dioxane (5 mL) and water (1 mL), and after displacement three times with nitrogen, the reaction solution was stirred at 90°C for 10 hours. After the reaction was stopped, the reaction was quenched by adding water (5 mL) and extracted with ethyl acetate (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product N-(4-(1-methyl-2-carbonyl-1,4-dihydro-2H-benzo[d][1,3]oxazin-6-yl)-5,6,7,8-tetrahydroisoquinolin-8-yl)propanamide (75 mg) with a yield of 58.2%.

MS m/z (ESI): 366.2 [M+1].

¹HNMR (400 MHz, DMSO) δ 8.26 (s, 1H), 8.22 (d, 1H), 8.14 (s, 1H), 7.30 (dd, 1H), 7.23 (d, 1H), 7.12 (d, 1H), 5.23 (s, 2H), 5.02 (q, 1H), 3.25 (s, 3H), 2.53 (q, 2H), 2.11-2.05 (m, 2H), 1.84 - 1.61 (m, 4H), 0.98 (t, 3H).

Example 1 was resolved to obtain 1-A and 1-B.

| | | |
|---|---|---|
| 1-A | 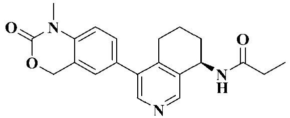 | MS m/z (ESI): 366.2 [M+1]. |
| 1-B | 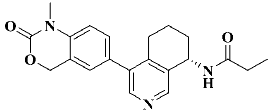 | MS m/z (ESI): 366.2 [M+1]. |

### Example 2

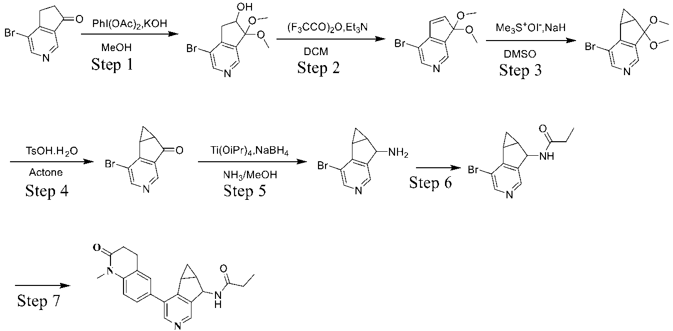

### Step 1

### 4-Bromo-7,7-dimethoxy-6,7-dihydro-5H-cyclopenta[c]pyridin-6-ol

Potassium hydroxide (5.29 g, 94.32 mmol) was dissolved in methanol (50 mL), and under ice-water bath cooling and nitrogen protection, 4-bromo-5,6-dihydro-7H-cyclopenta[c]pyridin-7-one was added. The mixture was reacted with stirring for 5 minutes at 0°C. Iodobenzene acetate (6.08 g, 18.86 mmol) was then added. The mixture was reacted with stirring for 4 hours at 18°C. The reaction solution was evaporated to dryness at a low temperature, and the reaction was quenched by adding a saturated aqueous sodium chloride solution (100 mL) to the crude product and extracted with ethyl acetate (50 mL × 3). The mixture was separated. The organic phases were combined and washed with a saturated aqueous sodium chloride solution (100 mL × 2), and the organic phase was dried over anhydrous sodium sulfate and concentrated under reduced pressure. The residue was purified by flash silica gel chromatography (eluted with dichloromethane:methanol = 100: 0 to 95: 5) to obtain the target product 4-bromo-7,7-dimethoxy-6,7-dihydro-5H-cyclopenta[c]pyridin-6-ol (1.5 g, a brown oil) with a yield of 58.02%.

MS m/z (ESI): 274.0, 276.0 [M+1].

### Step 2

### 4-Bromo-7,7-dimethoxy-7H-cyclopenta[c]pyridine

4-Bromo-7,7-dimethoxy-6,7-dihydro-5H-cyclopenta[c]pyridin-6-ol (1.5 g, 5.47 mmol) and triethylamine (2.77 g, 27.36 mmol, 3.82 mL) were dissolved in dichloromethane (60 mL), and under ice-water bath cooling and nitrogen protection, trifluoroacetic anhydride (2.30 g, 10.94 mmol, 1.52 mL) was added. The mixture was reacted with stirring for 12 hours at 20°C. The reaction was quenched by adding a saturated aqueous sodium bicarbonate solution (150 mL) to the mixture. The organic phase was separated, sequentially washed with a saturated aqueous sodium chloride solution (50 mL × 2), dried over anhydrous sodium sulfate, and concentrated under reduced pressure. The residue was purified by flash silica gel chromatography (eluted with petroleum ether:ethyl acetate = 100: 0 to 80: 20) to obtain the target product 4-bromo-7,7-dimethoxy-7H-cyclopenta[c]pyridine (1.3 g) with a yield of 92.76%.

MS m/z (ESI): 256.0, 258.0 [M+1].

### Step 3

### 4-Bromo-6,6-dimethoxy-4b,5,5a,6-tetrahydrocyclopropa[3,4]cyclopenta[1,2-c]pyridine

Trimethylsulfoxonium iodide (3.35 g, 15.23 mmol) was dissolved in dimethyl sulfoxide (20 mL), and under nitrogen protection, sodium hydride (609.09 mg, 15.23 mmol, 60% w/w) was added. The mixture was reacted with stirring for 3 hours at 18°C. Under nitrogen protection, 4-bromo-7,7-dimethoxy-7H-cyclopenta[c]pyridine (1.30 g, 5.08 mmol) was added. The mixture was reacted with stirring for 12 hours at 18°C. The reaction solution was quenched with a saturated aqueous sodium chloride solution (130 mL) and extracted with ethyl acetate (50 mL × 2). The organic phase was separated, washed with a saturated aqueous sodium chloride solution (50mL × 5), dried over anhydrous sodium sulfate, and concentrated under reduced pressure to obtain crude 4-bromo-6,6-dimethoxy-4b,5,5a,6-tetrahydrocyclopropa[3,4]cyclopenta[1,2-c]pyridine (1.1 g). The crude product was directly used for the next step.

MS m/z (ESI): 270.0, 272.0 [M+1].

### Step 4

### 4-Bromo-5,5a-dihydrocyclopropa[3,4]cyclopenta[1,2-c]pyridin-6(4bH)-one

4-Bromo-6,6-dimethoxy-4b,5,5a,6-tetrahydrocyclopropa[3,4]cyclopenta[1,2-c]pyridine (1.1 g, 4.07 mmol) was dissolved in acetone (30 mL), and under nitrogen protection, p-toluenesulfonic acid monohydrate (774.61 mg, 4.07 mmol) was added. The mixture was reacted with stirring for 2 hours at 18°C. The mixture was evaporated to dryness at a low temperature, the reaction was quenched by adding a saturated aqueous sodium chloride solution (50 mL), and the mixture was extracted with dichloromethane (50 mL × 2). The organic phases were combined, sequentially washed with a saturated aqueous sodium chloride solution (50 mL × 2), dried over anhydrous sodium sulfate, and concentrated under reduced pressure. The residue was purified by flash silica gel chromatography (eluted with petroleum ether:ethyl acetate = 100: 0 to 75: 25) to obtain the target product 4-bromo-5,5a-dihydrocyclopropa[3,4]cyclopenta[1,2-c]pyridin-6(4bH)-one (0.55 g, a pale brown solid) with a yield of 60.28%.

MS m/z (ESI): 224.0, 226.0 [M+1].

### Step 5

### 4-Bromo-4b,5,5a,6-tetrahydrocyclopropa[3,4]cyclopenta[1,2-c]pyridin-6-amine

4-Bromo-5,5a-dihydrocyclopropa[3,4]cyclopenta[1,2-c]pyridin-6(4bH)-one (0.55 g, 2.45 mmol) was dissolved in a solution of ammonia in methanol (2 M, 20 mL), and under nitrogen protection, tetraisopropyl titanate (2.16 g, 4.91 mmol) was added. The mixture was reacted with stirring for 3 hours at 60°C. The reaction solution was cooled to room temperature, and sodium borohydride (185.74 mg, 4.91 mmol) was then added. The mixture was reacted with stirring for hours at 20°C. The reaction solution was evaporated to dryness, and the crude product was dissolved with dichloromethane (60 mL) and washed with a saturated aqueous sodium chloride solution (30 mL × 2). The organic phase was separated, dried over anhydrous sodium sulfate, and concentrated under reduced pressure to obtain crude 4-bromo-4b,5,5a,6-tetrahydrocyclopropa[3,4]cyclopenta[1,2-c]pyridin-6-amine (0.44 g). The crude product was directly used for the next step.

MS m/z (ESI): 225.0, 227.0 [M+1].

### Step 6

### N-(4-bromo-4b,5,5a,6-tetrahydrocyclopropa[3,4]cyclopenta[1,2-c]pyridin-6-yl)propanamide

With 4-bromo-4b,5,5a,6-tetrahydrocyclopropa[3,4]cyclopenta[1,2-c]pyridin-6-amine and propionyl chloride as starting materials, N-(4-bromo-4b,5,5a,6-tetrahydrocyclopropa[3,4]cyclopenta[1,2-c]pyridin-6-yl)propanamide was synthesized according to step 3 of Example 1.

MS m/z (ESI): 281.0, 283.0 [M+1].

### Step 7

### N-(4-(1-methyl-2-carbonyl-1,2,3,4-tetrahydroisoquinolin-6-yl)-4b,5,5a,6-tetrahydrocyclopropa[3,4]cyclopenta[1,2-c]pyridin-6-yl)propanamide

With N-(4-bromo-4b,5,5a,6-tetrahydrocyclopropa[3,4]cyclopenta[1,2-c]pyridin-6-yl)propanamide and 1-methyl-6-(4,4,5,5-tetramethyl-1,3,2-dioxaborolan-2-yl)-3,4-dihydroquinolin-2(1H)-one as starting materials, N-(4-(1-methyl-2-carbonyl-1,2,3,4-tetrahydroisoquinolin-6-yl)-4b,5,5a,6-tetrahydrocyclopropa[3,4]cyclopenta[ 1,2-c]pyridin-6-yl)propanamide was synthesized according to step 4 of Example 1.

MS m/z (ESI): 362.2 [M+1].

The sample was subjected to chiral separation as follows to obtain products P1 and P2.

| Column type | Mobile phase | Flow rate | Detection wavelength | Tempera ture | Retentio n time of P1 | Retention time of P2 | Linear gradient |
|---|---|---|---|---|---|---|---|
| CHIRALCEL AS-H, 4.6 × 150 mm, 5HI | Hexane : EtOH = 70: 30 | 1.0 ml/min | 214 nm | 35°C | 3.7 min | 5.5 min | Isometric |

The product P1 was subjected to chiral resolution under the following conditions to obtain P1A and P1B

| Column type | Mobile phase | Flow rate | Detection wavelength | Tempe rature | Retention time of P1A | Retention time of P1B | Linear gradient |
|---|---|---|---|---|---|---|---|
| CHIRALPAK AD-H, 4.6 × 250 mm, 5RA | EtOH : MeOH = 50 : 50 | 0.5 ml/min | 214 nm | 35°C | 7.7 min | 9.8 min | Isometric |

The product P2 was subjected to chiral resolution under the following conditions to obtain P2A and P2B

| Column type | Mobile phase | Flow rate | Detection wavelength | Tempe rature | Retention time of P2A | Retention time of P2B | Linear gradient |
|---|---|---|---|---|---|---|---|
| CHIRALPAK AD-H, 4.6 × 250 mm, 5 µm | EtOH : MeOH = 50: 50 | 0.5 ml/min | 214 nm | 35°C | 10.1 min | 13.8 min | Isometric |

LCMS and HNMR of a P2B sample were as follows.

¹H NMR (400 MHz, CDCl₃) δ 8.48 (s, 1H), 8.40 (s, 1H), 7.50 (dd, J = 8.4, 2.0 Hz, 1H), 7.44 - 7.34 (m, 1H), 7.11 (d, J = 8.4 Hz, 1H), 6.04 - 5.88 (m, 1H), 5.77 - 5.58 (m, 1H), 3.41 (s, 3H), 3.00 (t, J = 7.4 Hz, 2H), 2.79 - 2.64 (m, 2H), 2.60 - 2.48 (m, 1H), 2.40 - 2.21 (m, 3H), 1.37 - 1.15 (m, 5H).

MS m/z (ESI): 362.2 [M+1].

### Example 3

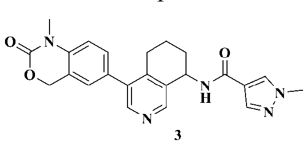

Referring to the synthesis route of Example 1, propionyl chloride was replaced with 1-methyl-1H-pyrazole-4-carbonyl chloride to obtain the title product 1-methyl-N-(4-(1-methyl-2-carbonyl-1,4-dihydro-2H-benzo[d][1,3]oxazin-6-yl)-5,6,7,8-tetrahydroisoquinolin-8-yl)-1H-pyrazole-4-carboxamide 3.

MS m/z (ESI): 418.2 [M+1].

### Example 4

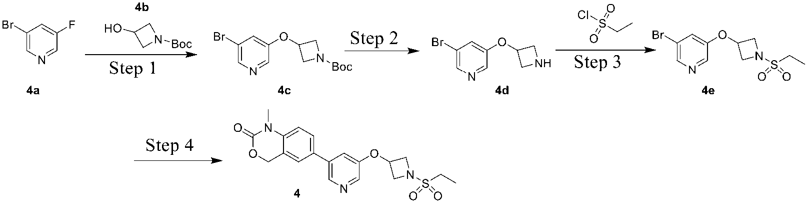

### Step 1

### Tert-butyl 3-((5-bromopyridin-3-yl)oxy)azetidine-1-carboxylate

Sodium hydride (693 mg, 17.34 mmol, 60%) was added to a solution of tert-butyl 3-hydroxyazetidine-1-carboxylate **4b** (2 g, 11.56 mmol) in tetrahydrofuran (25 mL) and stirred at room temperature for 30 minutes, and a solution of 3-bromo-5-fluoropyridine **4a** (1.6 g,9.25 mmol) in tetrahydrofuran (10 mL) was then dropwise added and stirred at room temperature overnight. Water was added. The mixture was extracted with dichloromethane (30 mL * 3). The organic phases were washed with a saturated aqueous sodium chloride solution, dried over anhydrous sodium sulfate, filtered, and rotary-evaporated to dryness. The residue was then separated by column chromatography to obtain the title product tert-butyl 3-((5-bromopyridin-3-yl)oxy)azetidine-1-carboxylate **4c** (1 g, a pale yellow solid) with a yield of 33%.

MS m/z (ESI): 329.0 [M+1].

### Step 2

### 3-(Azetidin-3-yloxy)-5-bromopyridine

Trifluoroacetic acid (5 mL) was dropwise added to a solution of tert-butyl 3-((5-bromopyridin-3-yl)oxy)azetidine-1-carboxylate **4c** (1 g, 3.04 mmol) in dichloromethane (15 mL), and the mixture was stirred at room temperature for 1 hour, concentrated under reduced pressure, and dried to obtain the title product 3-(azetidin-3-yloxy)-5-bromopyridine **4d** (1.1 g, crude).

MS m/z (ESI): 229.0 [M+1].

### Step 3

### 3-Bromo-5-((1-(ethylsulfonyl)azetidin-3-yl)oxy)pyridine

Referring to step 3 of the synthesis route of Example 1, propionyl chloride was replaced with ethanesulfonyl chloride to obtain the title product 3-bromo-5-((1-(ethylsulfonyl)azetidin-3-yl)oxy)pyridine **4e.**

MS m/z (ESI): 321.0 [M+1].

### Step 4

### 6-(5-((1-(Ethylsulfonyl)azetidin-3-yl)oxy)pyridin-3-yl)-1-methyl-1,4-dihydro-2H-benzo[d][1,3]oxazin-2-one

Referring to step 4 of the synthesis route of Example 1, N-(4-bromo-5,6,7,8-tetrahydroisoquinolin-8-yl)propanamide was replaced with 3-bromo-5-((1-(ethylsulfonyl)azetidin-3-yl)oxy)pyridine to obtain the title product.

MS m/z (ESI): 404.1 [M+1].

### Example 5

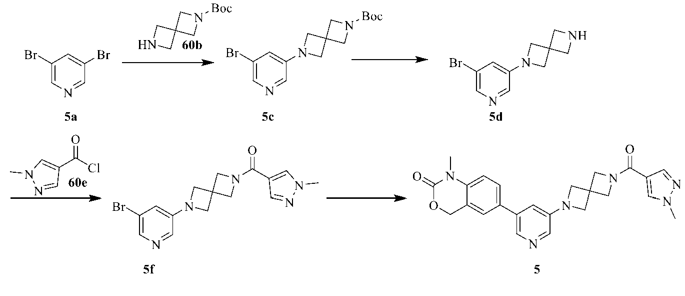

### Step 1

### Tert-butyl 6-(5-bromopyridin-3-yl)-2,6-diazaspiro[3.3]heptane-2-carboxylate

3,5-Dibromopyridine **5a** (3 g, 12.66 mmol) and tert-butyl 2,6-diazaspiro[3.3]heptane-2-carboxylate **5b** (2.51 g, 12.66 mmol) were dissolved in 1,4-dioxane (50 mL), and sodium tert-butoxide (2.43 g, 25.33 mmol), 4,5-bis(diphenylphosphino)-9,9-dimethylxanthene (733 mg, 1.27 mmol), and tris(dibenzylideneacetone)palladium (580 mg,633 µmol) were separately added thereto. After displacement three times with nitrogen, the reaction system was placed in an oil bath at 100°C and stirred for 16 hours. After the reaction was complete, the mixture was cooled to room temperature, diluted by adding water, extracted with ethyl acetate, washed with a saturated aqueous sodium chloride solution, dried over anhydrous sodium sulfate, filtered, and concentrated, and the crude product was separated and purified by column chromatography to obtain the title product tert-butyl 6-(5-bromopyridin-3-yl)-2,6-diazaspiro[3.3]heptane-2-carboxylate **5c** (2 g, a beige solid) with a yield of 44.6%.

MS m/z (ESI): 354.1 [M+1].

### Step 2

### 2-(5-Bromopyridin-3-yl)-2,6-diazaspiro[3.3]heptane

Referring to step 2 of the synthesis route of Example 58, the title product 2-(5-bromopyridin-3-yl)-2,6-diazaspiro[3.3]heptane **5d** was obtained.

MS m/z (ESI): 254.1 [M+1].

### Step 3

### (6-(5-Bromopyridin-3-yl)-2,6-diazaspiro[3.3]heptan-2-yl)(1-methyl-1H-pyrazol-4-yl)methanone

Referring to step 3 of the synthesis route of Example 1, the title product 2-(5-bromopyridin-3-yl)-2,6-diazaspiro[3.3]heptane **5f** was obtained.

MS m/z (ESI): 362.1 [M+1].

### Step 4

### 1-Methyl-6-(5-(6-(1-methyl-1H-pyrazole-4-carbonyl)-2,6-diazaspiro[3.3]heptan-2-yl)pyridin-3-yl)-1,4-dihydro-2H-benzo[d][1,3]oxazin-2-one

Referring to step 4 of the synthesis route of Example 1, the title product was obtained.

MS m/z (ESI): 445.2 [M+1].

### Example 6

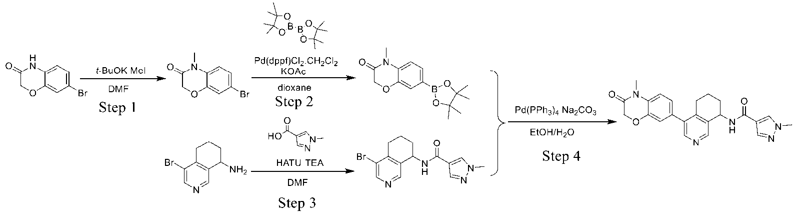

### Step 1

7-Bromo-2H-benzo[b][1,4]oxazin-3(4H)-one (5 g, 21.93 mmol) was dissolved in N,N-dimethylformamide (50 mL), and in an ice-water bath, potassium tert-butoxide (4.92 g, 43.85 mmol) was slowly added thereto. After stirring for 0.5 hours, iodomethane (4.67 g, 32.89 mmol, 2.05 mL) was dropwise added thereto. The reaction system was further stirred at room temperature 20°C for 5.5 hours. After the reaction was complete, the reaction was quenched by slowly dropwise adding H₂O (20 mL) under ice-water bath condition, and the mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 3:1, uv = 254 nm) to finally obtain 7-bromo-4-methyl-2H-benzo[b][1,4]oxazin-3(4H)-one as a pale yellow solid (4.5 g, yield 84.7%).

MS m/z (ESI): 241.9 [M+1].

### Step 2

7-Bromo-4-methyl-2H-benzo[b][1,4]oxazin-3(4H)-one (4.5 g, 18.59 mmol) was dissolved in dioxane (50 mL), and bis(pinacolato)diboron (5.66 g, 22.31 mmol), potassium acetate (1.95 g, 19.91 mmol), and [1,1'-bis(diphenylphosphino)ferrocene]palladium dichloride dichloromethane complex (1.35 g, 1.86 mmol) were separately added thereto. After displacement multiple times with nitrogen, the reaction system was placed in an oil bath at 100°C and stirred for 16 hours. After the reaction was complete, the mixture was cooled to room temperature and filtered through diatomite, H₂O (20 mL) was slowly added, and the mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain 4-methyl-7-(4,4,5,5-tetramethyl-1,3,2-dioxaborolan-2-yl)-2H-benzo[b][1,4]oxazin-3(4H)-one as a white solid (5 g, 17.29 mmol, 93.02% yield).

MS m/z (ESI): 290.1 [M+1].

### Step 3

4-Bromo-5,6,7,8-tetrahydroisoquinolin-8-amine (500 mg, 2.20 mmol) and 1-methyl-1H-pyrazole-4-carboxylic acid (333 mg, 2.64 mmol) were dissolved in N,N-dimethylformamide (20 mL), and triethylamine (668 mg, 6.60 mmol) and 2-(7-azabenzotriazole)-N,N,N',N'-tetramethyluronium hexafluorophosphate (1.26 g, 3.30 mmol) were separately added thereto. The reaction solution was stirred at 25°C for 4 hours. After the reaction was stopped, the reaction was quenched by slowly dropwise adding water (10 mL), and the mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 1 : 1, UV = 254 nm) to obtain the target product N-(4-bromo-5,6,7,8-tetrahydroisoquinolin-8-yl)-1-methyl-1H-pyrazole-4-carboxamide (400 mg, yield: 52.1%).

MS m/z (ESI): 335.0 [M+1].

### Step 4

N-(4-bromo-5,6,7,8-tetrahydroisoquinolin-8-yl)-1-methyl-1H-pyrazole-4-carboxamide (100 mg, 285.6 µmol) and 4-methyl-7-(4,4,5,5-tetramethyl-1,3,2-dioxaborolan-2-yl)-2H-benzo[b][1,4]oxazin-3(4H)-one (99.6 mg, 342.7 µmol) were dissolved in a mixed solvent of H₂O (2 mL) and EtOH (10 mL), and sodium carbonate (91 mg, 857.0 µmol) and tetrakis(triphenylphosphine)palladium (33.0 mg, 28.6 µmol) were sequentially added thereto. After displacement multiple times with nitrogen, the reaction system was placed in an oil bath at 100°C and stirred for 16 hours. After the reaction was complete, the mixture was cooled to room temperature, diluted by adding water (5 mL), extracted with ethyl acetate (10 mL × 2), washed with a saturated aqueous sodium chloride solution (10 mL × 2), dried over anhydrous sodium sulfate, filtered, and concentrated to obtain a crude product. The crude product was purified by a column (DCM/MeOH = 10:1, UV = 254 nm) to finally obtain 1-methyl-N-(4-(4-methyl-3-carbonyl-3,4-dihydro-2H-benzo[b][1,4]oxazin-7-yl)-5,6,7,8-tetrahydroisoquinolin-8-yl)-1H-parazole-4-carboxamide (50 mg, 41.7% yield).

MS m/z (ESI): 418.1 [M+1].

### Example 7

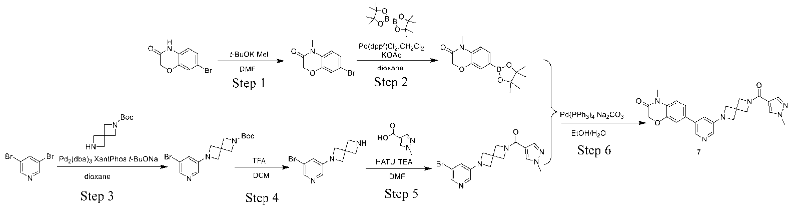

### Step 1

### 7-Bromo-4-methyl-2H-benzo[b][1,4]oxazin-3(4H)-one

7-Bromo-2H-benzo[b][1,4]oxazin-3(4H)-one (5 g, 21.93 mmol) was dissolved in N,N-dimethylformamide (50 mL), and in an ice-water bath, potassium tert-butoxide (4.92 g, 43.85 mmol) was slowly added thereto. After stirring for 0.5 hours, iodomethane (4.67 g, 32.89 mmol, 2.05 mL) was dropwise added thereto. The reaction system was further stirred at room temperature 20°C for 5.5 hours. After the reaction was complete, the reaction was quenched by slowly dropwise adding H₂O (20 mL) under ice-water bath condition, and the mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 3:1, uv = 254 nm) to finally obtain 7-bromo-4-methyl-2H-benzo[b][1,4]oxazin-3(4H)-one as a pale yellow solid (4.5 g, yield 84.7%).

MS m/z (ESI): 241.9 [M+1].

### Step 2

### 4-Methyl-7-(4,4,5,5-tetramethyl-1,3,2-dioxaborolan-2-yl)-2H-benzo[b][1,4]oxazin-3(4H)-one

7-Bromo-4-methyl-2H-benzo[b][1,4]oxazin-3(4H)-one (4.5 g, 18.59 mmol) was dissolved in dioxane (50 mL), and bis(pinacolato)diboron (5.66 g, 22.31 mmol), potassium acetate (1.95 g, 19.91 mmol), and [1,1'-bis(diphenylphosphino)ferrocene]palladium dichloride dichloromethane complex (1.35 g, 1.86 mmol) were separately added thereto. After displacement multiple times with nitrogen, the reaction system was placed in an oil bath at 100°C and stirred for 16 hours. After the reaction was complete, the mixture was cooled to room temperature and filtered through diatomite, H₂O (20 mL) was slowly added, and the mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain 4-methyl-7-(4,4,5,5-tetramethyl-1,3,2-dioxaborolan-2-yl)-2H-benzo[b][1,4]oxazin-3(4H)-one as a white solid (5 g, 17.29 mmol, 93.02% yield).

MS m/z (ESI): 290.1 [M+1].

### Step 3

### Tert-butyl 6-(5-bromopyridin-3-yl)-2, 6-diazaspiro[3.3]heptane-2-carboxylate

3,5-Dibromopyridine (3 g, 12.66 mmol) and tert-butyl 2,6-diazaspiro[3.3]heptane-2-carboxylate (2.51 g, 12.66 mmol) were dissolved in dioxane (40 mL), and sodium tert-butoxide (2.43 g, 25.33 mmol), 4,5-bis(diphenylphosphino)-9,9-dimethylxanthene (732.76 mg, 1.27 mmol), and tris(dibenzylideneacetone)dipalladium (579.83 mg, 633.20 µmol) were separately added thereto. After displacement multiple times with nitrogen, the reaction system was placed in an oil bath at 100°C and stirred for 16 hours. After the reaction was complete, the mixture was cooled to room temperature, and the reaction was quenched by slowly dropwise adding water (10 mL). The mixture was extracted with ethyl acetate (20 mL × 2), washed with a saturated aqueous sodium chloride solution (20 mL × 2), dried over anhydrous sodium sulfate, filtered, and concentrated to obtain a crude product. The crude product was purified by a column (PE/EtOAc = 5 : 1, uv = 254 nm) to finally obtain tert-butyl 6-(5-bromopyridin-3-yl)-2,6-diazaspiro[3.3]heptane-2-carboxylate as a beige solid (2 g, 5.65 mmol, 44.58% yield).

MS m/z (ESI): 354.0 [M+1].

### Step 4

### 2-(5-Bromopyridin-3-yl)-2,6-diazaspiro[3.3]heptane

Tert-butyl 6-(5-bromopyridin-3-yl)-2,6-diazaspiro[3.3]heptane-2-carboxylate (500 mg, 1.41 mmol) was dissolved in dichloromethane (10 mL), and trifluoroacetic acid (1 mL) was added thereto. The reaction system was stirred for 4 hours at room temperature. After the starting material was completely reacted, the excess solvent was directly removed by concentration to obtain crude 2-(5-bromopyridin-3-yl)-2,6-diazaspiro[3.3]heptane (360 mg, crude). The crude product was directly dissolved for the next reaction.

MS m/z (ESI): 254.0 [M+1].

### Step 5

### (6-(5-Bromopyridin-3-yl)-2,6-diazaspiro[3.3]heptan-2-yl)(1-methyl-1H-pyrazol-4-yl)methanone

2-(5-Bromopyridin-3-yl)-2,6-diazaspiro[3.3]heptane (360 mg, crude) was dissolved in tetrahydrofuran (10 mL), and triethylamine (430 mg, 4.23 mmol) and 1-methyl-1H-pyrazole-4-carboxylic acid (270 mg, 2.12 mmol) were separately added thereto. The reaction system was stirred for 4 hours at room temperature. After the reaction was complete, the reaction was quenched by adding water (5 mL), extracted with ethyl acetate (10 mL × 2), washed with a saturated aqueous sodium chloride solution (10 mL × 2), dried over anhydrous sodium sulfate, filtered, and concentrated to obtain a crude product. The crude product was purified by passing through a column (PE/EtOAc = 3 : 1, uv = 254 nm) to finally obtain the target product (6-(5-bromopyridin-3-yl)-2,6-diazaspiro[3.3]heptan-2-yl)(1-methyl-1H-pyrazol-4-yl)methanone (350 mg, yield: 68.7%).

MS m/z (ESI): 362.1 [M+1].

### Step 6

### 4-Methyl-7-(5-(6-(1-methyl-1H-pyrazole-4-carbonyl)-2,6-diazaspiro[3.3]heptan-2-yl)pyridin-3-yl)-2H-benzo[b][1,4]oxazin-3(4H)-one

(6-(5-Bromopyridin-3-yl)-2,6-diazaspiro[3.3]heptan-2-yl)(1-methyl-1H-pyrazol-4-yl)methanone (100 mg, 276.2 µmol) and 4-methyl-7-(4,4,5,5-tetramethyl-1,3,2-dioxaborolan-2-yl)-2H-benzo[b][1,4]oxazin-3(4H)-one (96.5 mg, 331.4 µmol) were dissolved in a mixed solvent of H₂O (2 mL) and EtOH (10 mL), and sodium carbonate (88.0 mg, 828.6 µmol) and tetrakis(triphenylphosphine)palladium (32.0 mg, 27.6 µmol) were sequentially added thereto. After displacement multiple times with nitrogen, the reaction system was placed in an oil bath at 100°C and stirred for 16 hours. After the reaction was complete, the mixture was cooled to room temperature, diluted by adding water (5 mL), extracted with ethyl acetate (10 mL × 2), washed with a saturated aqueous sodium chloride solution (10 mL × 2), dried over anhydrous sodium sulfate, filtered, and concentrated to obtain a crude product. The crude product was purified by a column (DCM/MeOH = 10 : 1, UV = 254 nm) to finally obtain the target product (45 mg, 36.7% yield).

MS m/z (ESI): 445.1 [M+1].

### Example 8

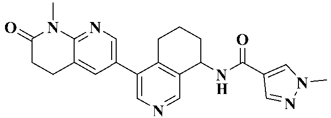

Referring to Example 6, with 6-bromo-3,4-dihydro-1,8-diazanaphthalen-2(1H)-one and 4-bromo-5,6,7,8-tetrahydroisoquinolin-8-amine as starting materials, the target product 1-methyl-N-(4-(8-methyl-7-carbonyl-5,6,7,8-tetrahydro-1,8-diazanaphthalen-3-yl)-5,6,7,8-tetrahydroisoquinolin-8-yl)-1H-pyrazole-4-carboxamide was finally obtained.

MS m/z (ESI): 417.2 [M+1].

### Example 9

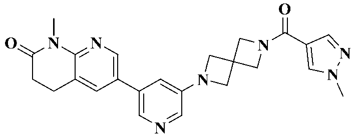

Referring to Example 7, with 6-bromo-3,4-dihydro-1,8-diazanaphthalen-2(1H)-one, 3,5-dibromopyridine, and tert-butyl 2,6-diazaspiro[3.3]heptane-2-carboxylate as starting materials, the target product 1-methyl-6-(5-(6-(1-methyl-1H-pyrazole-4-carbonyl)-2,6-diazaspiro[3.3]heptan-2-yl)pyridin-3-yl)-3,4-dihydro-1,8-diazanaphthalen-2(1H)-one was finally obtained.

MS m/z (ESI): 444.2 [M+1].

### Example 10

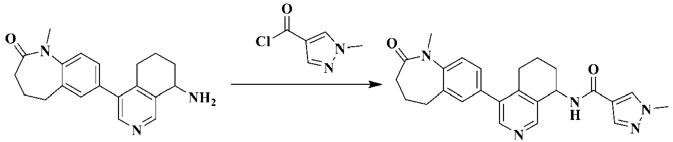

With 7-(8-Amino-5,6,7,8-tetrahydroisoquinolin-4-yl)-1-methyl-1,3,4,5-tetrahydro-2H-benzo[b]azepin-2-one as a starting material, 1-methyl-N-(4-(1-methyl-2-carbonyl-2,3,4,5-tetrahydro-1H-benzo[b]azepin-7-yl)-5,6,7,8-tetrahydroisoquinolin-8-yl)-1H-pyrazole-4-carboxamide was obtained following the procedure in step 3 of Example 1.

MS m/z (ESI): 430.2 [M+1].

### Example 11

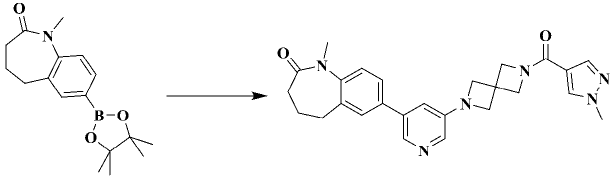

With 1-methyl-7-(4,4,5,5-tetramethyl-1,3,2-dioxaborolan-2-yl)-1,3,4,5-tetrahydro-2H-benzo[b]azepin-2-one as a starting material, 1-methyl-7-(5-(6-(1-methyl-1H-pyrazole-4-carbonyl)-2,6-diazaspiro[3.3]heptan-2-yl)pyridin-3-yl)-1,3,4,5-tetrahydro-2H-benzo[b]azepin-2-one was obtained following the procedure in Example 5.

MS m/z (ESI): 457.2 [M+1].

### Example 12

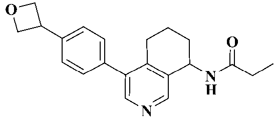

Referring to the synthesis route of Example 1, 6-bromo-1-methyl-1,4-dihydro-2H-benzo[d][1,3]oxazin-2-one was replaced with 3-(4-bromophenyl)oxetane to obtain the title product N-(4-(4-(oxetan-3-yl)phenyl)-5,6,7,8-tetrahydroisoquinolin-8-yl)propanamide.

MS m/z (ESI): 337.2[M+1].

### Example 13

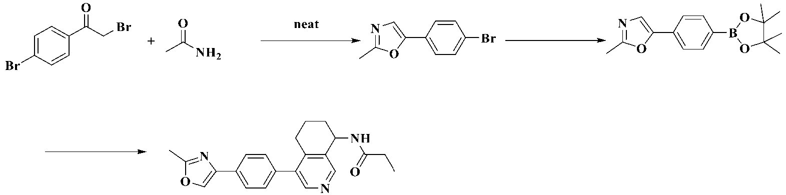

### Step 1

### 5-(4-Bromophenyl)-2-methyloxazole

At room temperature, acetamide (0.50 g, 8.47 mmol) was heated to 120°C, then 2-bromo-1-(4-bromophenyl)ethanone (1.57 g, 5.65 mmol) was added, and the mixture was reacted at 120°C for 3 hours. LCMS indicated the formation of the target product, and the product was cooled to room temperature. The reaction solution was dissolved in ethyl acetate (30 mL), then washed sequentially with a saturated sodium bicarbonate solution (10 mL × 2) and a saturated aqueous sodium chloride solution (10 mL × 2). The organic phase was dried over anhydrous sodium sulfate, filtered, and rotary-evaporated to dryness. The residue was separated by Flash column chromatography (eluted with PE : EA = 98 : 2-94 : 6) to obtain the target product 5-(4-bromophenyl)-2-methyloxazole (560 mg, a pale yellow solid) with a yield of 41.6%.

MS m/z (ESI): 238.0, 240.0 [M+1].

### Step 2

### 2-Methyl-5-(4-(4,4,5,5-tetramethyl-1,3,2-dioxaborolan-2-yl)phenyl)oxazole

At room temperature, bis(pinacolato)diboron (147.19 mg, 579.64 µmol), 5-(4-bromophenyl)-2-methyloxazole (92 mg, 386.42 µmol), [1,1'-bis(diphenylphosphino)ferrocene]dichloropalladium (31.56 mg, 38.64 µmol), and potassium acetate (75.74 mg, 772.85 µmol) were dissolved in 1'4-dioxane (5 mL). The atmosphere was displaced with nitrogen, and the mixture was heated to 100°C, reacted for 14 hours and cooled to room temperature. LCMS indicated the completion of the reaction. The mixture was filtered, and the filter residue was washed with ethyl acetate (15 mL × 2). The organic phases were combined, washed with a saturated aqueous sodium chloride solution (10 mL × 2), dried over anhydrous sodium sulfate, filtered, and rotary-evaporated to dryness. The residue was purified by thin layer preparative chromatography to obtain the target product, 2-methyl-5-(4-(4,4,5,5-tetramethyl-1,3,2-dioxaborolan-2-yl)phenyl)oxazole (80 mg, a pale yellow solid) with a yield of 72.6%.

MS m/z (ESI): 286.1 [M+1].

### Step 3

### N-(4-(4-(2-methyloxazol-4-yl)phenyl)-5,6,7,8-tetrahydroisoquinolin-8-yl)propanamide

At room temperature, N-(4-bromo-5,6,7,8-tetrahydroisoquinolin-8-yl)propanamide (30 mg, 105.95 µmol), 2-methyl-5-(4-(4,4,5,5-tetramethyl-1,3,2-dioxaborolan-2-yl)phenyl)oxazole (36.25 mg, 127.13 µmol), sodium carbonate (33.69 mg, 317.84 µmol), and tetrakis(triphenylphosphine)palladium (12.24 mg, 10.59 µmol) were dissolved in water (1 mL) and ethanol (5 mL). The atmosphere was displaced with nitrogen, and the mixture was heated to 100°C, reacted for 14 hours and cooled to room temperature. LCMS indicated the completion of the reaction. The mixture was filtered, and rotary-evaporated to dryness. The residue was dissolved in ethyl acetate (15 mL), then washed with a saturated aqueous sodium chloride solution (15 mL × 2). The organic phase was dried over anhydrous sodium sulfate, filtered, and rotary-evaporated to dryness. The residue was separated by reversed-phase preparative chromatography (neutral), and lyophilized to obtain the target product (11 mg, white solid) with a yield of 28.7%.

MS m/z (ESI): 362.2[M+1].

¹H NMR (400 MHz, DMSO-d₆) δ 8.55 (s, 1H), 8.34 (s, 1H), 8.30 (d, 1H), 8.25 (s, 1H), 7.85 (d, 2H), 7.42 (d, 2H), 5.14-5.08 (m, 1H), 2.63-2.60 (m, 2H), 2.49 (s, 3H), 2.21-2.20 (m, 2H), 1.92-1.63(m, 4H), 1.06 (t, 3H).

Example 13 was resolved to obtain 13-A and 13-B.

| | | |
|---|---|---|
| 13-A | 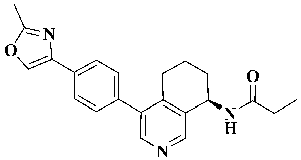 | MS m/z (ESI): 362.2[M+1]. |
| 13-B | 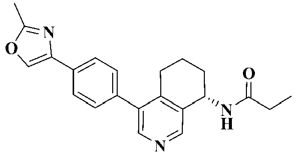 | MS m/z (ESI): 362.2[M+1]. |

### Example 14

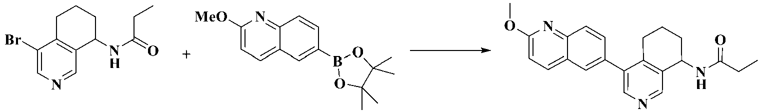

Referring to the synthesis conditions of Example 1, with N-(4-bromo-5,6,7,8-tetrahydroisoquinolin-8-yl)propanamide and 2-methoxy-6-(4,4,5,5-tetramethyl-1,3,2-dioxaborolan-2-yl)quinoline as starting materials, the title product N-(4-(2-methoxyquinolin-6-yl)-5,6,7,8-tetrahydroisoquinolin-8-yl)propanamide was synthesized.

MS m/z (ESI): 362.2[M+1].

Example 14 was resolved to obtain 14-A and 14-B.

| | | |
|---|---|---|
| 14-A | 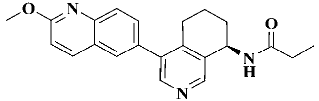 | MS m/z (ESI): 362.2[M+1]. |
| 14-B | 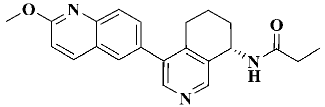 | MS m/z (ESI): 362.2[M+1]. |

14-A: ¹H NMR (400 MHz, CDCl₃) δ 8.53 (s, 1H), 8.37 (s, 1H), 7.98 (d, 1H), 7.90 (d, 1H), 7.60 (d, 1H), 7.52 (dd, 1H), 6.95 (d, 1H), 5.84 (d, 1H), 5.37 (q, 1H), 4.10 (s, 3H), 2.67 (tdd, 2H), 2.30 (q, 2H), 2.16 - 2.04 (m, 1H), 1.92 - 1.73 (m, 2H), 1.23 (t, 3H).

### Example 15

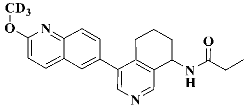

Referring to the synthesis conditions of Example 1, with N-(4-bromo-5,6,7,8-tetrahydroisoquinolin-8-yl)propanamide and 2-deuteromethoxy-6-(4,4,5,5-tetramethyl-1,3,2-dioxaborolan-2-yl)quinoline as starting materials, the title product N-(4-(2-deuteromethoxyquinolin-6-yl)-5,6,7,8-tetrahydroisoquinolin-8-yl)propanamide was synthesized.

MS m/z (ESI): 365.2[M+1].

### Example 16

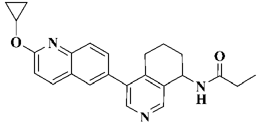

Referring to the synthesis conditions of Example 1, with N-(4-bromo-5,6,7,8-tetrahydroisoquinolin-8-yl)propanamide and 2-cyclopropoxy-6-(4,4,5,5-tetramethyl-1,3,2-dioxaborolan-2-yl)quinoline as starting materials, the title product N-(4-(2-cyclopropoxyquinolin-6-yl)-5,6,7,8-tetrahydroisoquinolin-8-yl)propanamide was synthesized.

MS m/z (ESI): 388.2[M+1].

### Example 17

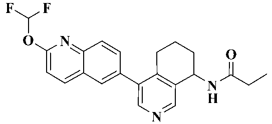

Referring to the synthesis conditions of Example 1, with N-(4-bromo-5,6,7,8-tetrahydroisoquinolin-8-yl)propanamide and 2-difluoromethoxy-6-(4,4,5,5-tetramethyl-1,3,2-dioxaborolan-2-yl)quinoline as starting materials, the title product N-(4-(2-difluoromethoxyquinolin-6-yl)-5,6,7,8-tetrahydroisoquinolin-8-yl)propanamide was synthesized.

MS m/z (ESI): 398.2[M+1].

### Example 18

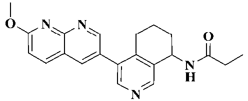

Referring to the synthesis conditions of Example 1, with N-(4-bromo-5,6,7,8-tetrahydroisoquinolin-8-yl)propanamide and 2-methoxy-6-(4,4,5,5-tetramethyl-1,3,2-dioxaborolan-2-yl)-1,8-naphthyridine as starting materials, the title product N-(4-(7-methoxy-1,8-naphthyridin-3-yl)-5,6,7,8-tetrahydroisoquinolin-8-yl)propanamide was synthesized.

MS m/z (ESI): 363.2[M+1].

### Example 19

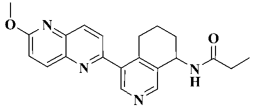

Referring to the synthesis conditions of Example 1, with N-(4-bromo-5,6,7,8-tetrahydroisoquinolin-8-yl)propanamide and 2-methoxy-6-(4,4,5,5-tetramethyl-1,3,2-dioxaborolan-2-yl)-1,5-naphthyridine as starting materials, the title product N-(4-(6-methoxy-1,5-naphthyridin-2-yl)-5,6,7,8-tetrahydroisoquinolin-8-yl)propanamide was synthesized.

MS m/z (ESI): 363.2[M+1].

### Example 20

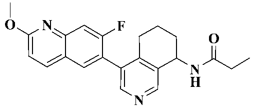

Referring to the synthesis conditions of Example 1, with N-(4-bromo-5,6,7,8-tetrahydroisoquinolin-8-yl)propanamide and 7-fluoro-2-methoxy-6-(4,4,5,5-tetramethyl-1,3,2-dioxaborolan-2-yl)quinoline as starting materials, the title product N-(4-(7-fluoro-2-methoxyquinolin-6-yl)-5,6,7,8-tetrahydroisoquinolin-8-yl)propanamide was synthesized.

MS m/z (ESI): 380.2[M+1].

### Example 21

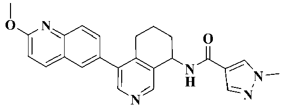

Referring to the synthesis conditions of Example 3, the title product N-(4-(2-methoxyquinolin-6-yl)-5,6,7,8-tetrahydroisoquinolin-8-yl)-1-methyl-1H-pyrazole-4-carboxamide was synthesized.

MS m/z (ESI): 414.2[M+1].

### Example 22

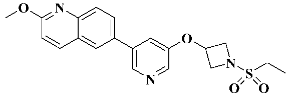

Referring to the synthesis conditions of Example 4, the title product 6-(5-((1-(ethylsulfonyl)azetidin-3-yl)oxy)pyridin-3-yl)-2-methoxyquinoline was synthesized.

MS m/z (ESI): 400.1[M+1].

¹H NMR (400 MHz, DMSO) δ 8.62 (d, 1H), 8.29 - 8.15 (m, 3H), 8.01 (dd, 1H), 7.81 (d, 1H), 7.60 (t, 1H), 7.02 (d, 1H), 5.23 (td, 1H), 4.33 (dd, 2H), 3.94 (s, 3H), 3.92 (dd, 2H), 3.14 (q, 2H), 1.18 (t, 3H).

### Example 23

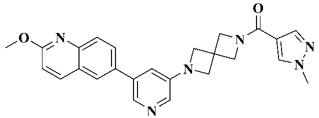

Referring to the synthesis conditions of Example 5, the title product (6-(5-(2-methoxyquinolin-6-yl)pyridin-3-yl)-2,6-diazaspiro[3.3]heptan-2-yl)(1-methyl-1H-pyrazol-4-yl)methanone was synthesized.

MS m/z (ESI): 441.2[M+1].

¹H NMR (400 MHz, MeOD) δ 8.28 (s, 1H), 8.20 (d, 1H), 8.06 (d, 2H), 7.95 - 7.86 (m, 2H), 7.82 (d, 2H), 7.23 (t, 1H), 6.99 (d, 1H), 4.68 (s, 2H), 4.35 (s, 2H), 4.22 (s, 4H), 4.07 (s, 3H), 3.93 (s, 3H).

### Example 24

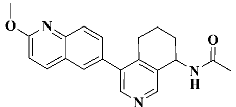

Referring to the synthesis conditions of Example 14, the title product N-(4-(2-methoxyquinolin-6-yl)-5,6,7,8-tetrahydroisoquinolin-8-yl)acetamide was synthesized.

MS m/z (ESI): 348.2[M+1].

Example 24 was resolved to obtain 24-A and 24-B.

| | | |
|---|---|---|
| 24-A | 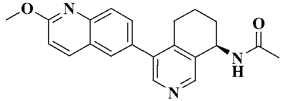 | MS m/z (ESI): 348.2 [M+1]. |
| 24-B | 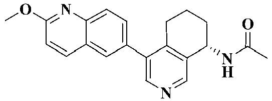 | MS m/z (ESI): 348.2 [M+1]. |

81-A: 1H NMR (400 MHz, CDCl₃) δ 8.54 (s, 1H), 8.36 (s, 1H), 7.97 (d, 1H), 7.90 (d, 1H), 7.59 (d, 1H), 7.51 (dd, 1H), 6.95 (d, 1H), 5.93 (d, 1H), 5.35 (q, 1H), 4.10 (s, 3H), 2.66 (qt, 2H), 2.09 (s, 1H), 2.08 (s, 3H), 1.94 - 1.73 (m, 2H).

### Example 25

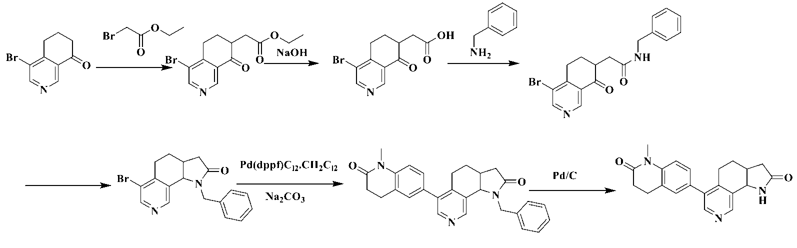

### Step 1

In a 50 mL reaction flask, 4-bromo-6,7-dihydroisoquinolin-8(5H)-one (2 g, 8.85 mmol) was dissolved in tetrahydrofuran (20 mL). Then, LiHMDS (8.85 mL, 1 M) was added at -78°C, and the reaction solution was stirred at -78°C for 1 hour. Then, a solution of ethyl 2-bromoacetate (1.48 g, 8.85 mmol) in tetrahydrofuran was added gradually, and the reaction solution was warmed to room temperature and reacted for 10 hours. After the reaction was stopped, the reaction was quenched by adding water (10 mL), the mixture was concentrated and extracted with ethyl acetate (10 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product ethyl 2-(4-bromo-8-carbonyl-5,6,7,8-tetrahydroisoquinolin-7-yl)acetate (1.1 g, a yellow solid) with a yield of 39.8%.

MS m/z (ESI): 312.0 [M+1].

### Step 2

In a 50 mL reaction flask, 5-chloro-N-cyclopropyl-4-fluoro-2-nitroaniline (1 g, 3.2 mmol) was dissolved in methanol (10 mL). Then, an aqueous solution of NaOH (256.26 mg, 6.41 mmol) was added at 25°C, and the reaction solution was stirred at 25°C for 2 hours. After the reaction was stopped, the mixture was adjusted to pH=5 with HCl (1 M), and the organic phase was extracted with ethyl acetate (10 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product 2-(4-bromo-8-carbonyl-5,6,7,8-tetrahydroisoquinolin-7-yl)acetic acid (800 mg, a yellow solid) with a yield of 87.8%.

MS m/z (ESI): 283.9 [M+1].

### Step 3

In a 50 mL reaction flask, 2-(4-bromo-8-carbonyl-5,6,7,8-tetrahydroisoquinolin-7-yl)acetic acid (500 mg, 1.76 mmol), benzylamine (188.58 mg, 1.76 mmol), HATU (669.22 mg, 1.76 mmol) and triethylamine (356.18 mg, 3.52 mmol) were dissolved in DMF (5 mL), then the reaction solution was stirred at 25°C for 10 hours. After the reaction was stopped, the reaction was quenched by adding water (10 mL), the mixture was extracted with ethyl acetate (10 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product N-benzyl-2-(4-bromo-8-carbonyl-5,6,7,8-tetrahydroisoquinolin-7-yl)acetamide (350 mg, a yellow solid) with a yield of 53.2%.

MS m/z (ESI): 373.0 [M+1].

### Step 4

In a 25 mL reaction flask, N-benzyl-2-(4-bromo-8-carbonyl-5,6,7,8-tetrahydroisoquinolin-7-yl)acetamide (350 mg, 937.71 µmol) was dissolved in acetonitrile (5 mL). Then, triethylsilane (545.18 mg, 4.69 mmol) and triethylamine (284.67 mg, 2.81 mmol) were added, and the reaction solution was stirred at 100°C for 2 hours. After the reaction was stopped, the reaction was quenched by adding water (5 mL), and the mixture was extracted with ethyl acetate (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product benzyl-6-bromo-1,3,3a,4,5,9b-hexahydro-2H-pyrrolo[3,2-h]isoquinolin-2-one (200 mg) with a yield of 59.7%.

MS m/z (ESI): 357.0 [M+1].

### Step 5

Referring to step 4 of Example 1, with benzyl-6-bromo-1,3,3a,4,5,9b-hexahydro-2H-pyrrolo[3,2-h]isoquinolin-2-one as a starting material, the product benzyl-6-(1-methyl-2-carbonyl-1,2,3,4-tetrahydroquinolin-6-yl)-1,3,3a,4,5,9b-hexahydro-2H-pyrrolo[3,2-h]isoquinolin-2-one was obtained.

MS m/z (ESI): 438.2 [M+1].

### Step 6

### 6-(1-Methyl-2-carbonyl-1,2,3,4-tetrahydroquinolin-6-yl)-1,3,3a,4,5,9b-hexahydro-2H-pyrrolo[3,2-h]isoquinolin-2-one

In a 25 mL reaction flask, benzyl-6-(1-methyl-2-carbonyl-1,2,3,4-tetrahydroquinolin-6-yl)-1,3,3a,4,5,9b-hexahydro-2H-pyrrolo[3,2-h]isoquinolin-2-one (100 mg, 228.55 µmol) was dissolved in methanol (5 mL). Then, Pd/C (100 mg, 5% palladium on carbon (55% water)) was added, and the reaction solution was stirred at 25°C under a hydrogen atmosphere for 2 hours. After the reaction was stopped, the mixture was filtered and concentrated. The resulting residue was purified by silica gel column chromatography with petroleum ether and ethyl acetate as an eluent system to obtain the title product with a yield of 31.5%.

MS m/z (ESI): 348.1 [M+1].

### Example 26

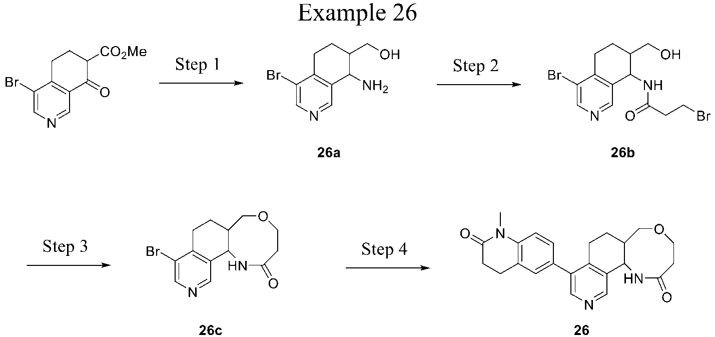

### Step 1

Methyl 4-bromo-8-oxo-5,6,7,8-tetrahydroisoquinoline-7-carboxylate (500 mg, 1.76 mmol) and tetraisopropyl titanate (1.0 g, 3.52 mmol) were dissolved in NH₃ (7 mol in MeOH, 10 mL), and the reaction mixture was stirred at room temperature for 24 hours. Sodium borohydride (200 mg, 5.28 mmol) was added to the reaction solution, and the reaction solution was heated to 65°C and stirred for 18 hour. After the heating was stopped, the reaction solution was cooled to room temperature, the reaction was quenched by adding a saturated ammonium chloride solution to the reaction solution, and the mixture was extracted with dichloromethane 3 times. The combined organic phases were dried over anhydrous sodium sulfate, and filtered. The filtrate was concentrated under reduced pressure. The resulting residue was purified by silica gel column chromatography with petroleum ether and ethyl acetate as an eluent system to obtain the title product (8-amino-4-bromo-5,6,7,8-tetrahydroisoquinolin-7-yl)methanol (374 mg, a yellow oil) with a yield of 82.7%.

MS m/z (ESI): 257.0 [M+1].

### Step 2

(8-Amino-4-bromo-5,6,7,8-tetrahydroisoquinolin-7-yl)methanol (350 mg, 1.36 mmol), HATU (776 mg, 2.04 mmol), and 3-bromopropanoic acid (248 mg, 1.63 mmol) were dissolved in DMF (10 mL), and the reaction solution was heated to 50°C and reacted with stirring for 18 hours. The reaction solution was cooled to room temperature, the reaction was quenched by adding a saturated ammonium chloride solution to the reaction solution, and the mixture was extracted with dichloromethane 3 times. The combined organic phases were dried over anhydrous sodium sulfate, and filtered. The filtrate was concentrated under reduced pressure. The resulting residue was purified by silica gel column chromatography with petroleum ether and ethyl acetate as an eluent system to obtain the title product 3-bromo-N-(4-bromo-7-(hydroxymethyl)-5,6,7,8-tetrahydroisoquinolin-8-yl)propanamide (411 mg, a yellow oil) with a yield of 77.0%.

MS m/z (ESI): 391.0 [M+1].

### Step 3

3-Bromo-N-(4-bromo-7-(hydroxymethyl)-5,6,7,8-tetrahydroisoquinolin-8-yl)propanamide (400 mg, 1.02 mmol) and potassium tert-butoxide (229 mg, 2.04 mmol) were dispersed in THF (10 mL), and the reaction solution was heated to 50°C, and reacted with stirring for 8 hours. The reaction solution was cooled to room temperature, the reaction was quenched by adding a saturated ammonium chloride solution to the reaction solution, and the mixture was extracted with dichloromethane 3 times. The combined organic phases were dried over anhydrous sodium sulfate, and filtered. The filtrate was concentrated under reduced pressure. The resulting residue was purified by silica gel column chromatography with petroleum ether and ethyl acetate as an eluent system to obtain the title product 9-bromo-1,3,4,6,6a,7,8,12b-octahydro-2H-[1,5]oxazino[3,4-h]isoquinolin-2-one (225 mg, a yellow oil) with a yield of 70.9%.

MS m/z (ESI): 311.0 [M+1].

### Step 4

Referring to the synthesis conditions of Example 14, with 9-bromo-1,3,4,6,6a,7,8,12b-octahydro-2H-[1,5]oxazino[3,4-h]isoquinolin-2-one (100 mg, 0.321 mmol) as a starting material, the title product 9-(1-methyl-2-oxo-1,2,3,4-tetrahydroquinolin-6-yl)-1,3,4,6,6a,7,8,12b-octahydro-2H-[1,5]oxazino[3,4-h]isoquinolin-2-one (102 mg, a colorless oil) was synthesized with a yield of 81.1%.

MS m/z (ESI): 392.2[M+1].

### Example 27

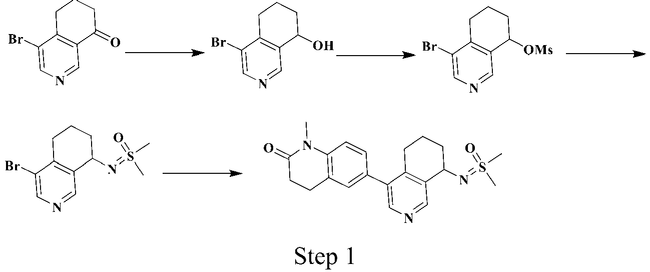

### Step 1

### 4-Bromo-5,6,7,8-tetrahydroisoquinolin-8-ol

4-Bromo-6,7-dihydroisoquinolin-8(5H)-one (0.1 g, 442.34 µmol) was dissolved in methanol (5 mL), then sodium borohydride (33.47 mg, 884.68 µmol) was added under nitrogen protection. The mixture was reacted with stirring for 2 hours at room temperature. The reaction solution was evaporated to dryness, and the crude product was extracted with ethyl acetate (30 mL) and washed with a saturated aqueous sodium chloride solution (30 mL). The organic phase was separated, dried over anhydrous sodium sulfate, and concentrated under reduced pressure to obtain crude 4-bromo-5,6,7,8-tetrahydroisoquinolin-8-ol (0.1 g). The crude product was directly used for the next step.

MS m/z (ESI): 228.0, 230.0 [M+1].

### Step 2

### (4-Bromo-5,6,7,8-tetrahydroisoquinolin-8-yl)methanesulfonate

4-Bromo-5,6,7,8-tetrahydroisoquinolin-8-ol (0.1 g, 438.43 µmol) and triethylamine (133.09 mg, 1.32 mmol) were dissolved in dichloromethane (4 mL), and under nitrogen protection, methanesulfonic anhydride (114.56 mg, 657.65 µmol) was added. The mixture was reacted with stirring for 12 hours at room temperature. The reaction solution was quenched with a saturated aqueous sodium chloride solution (30 mL), extracted with dichloromethane (30 mL), the organic phase was separated, washed with a saturated aqueous sodium chloride solution (30 mL × 2), dried over anhydrous sodium sulfate, and concentrated under reduced pressure to obtain the crude product (4-bromo-5,6,7,8-tetrahydroisoquinolin-8-yl) methanesulfonate (0.13 g). The crude product was directly used for the next step.

### Step 3

### (4-Bromo-5,6,7,8-tetrahydroisoquinolin-8-yl)imino-dimethyl-carbonyl-sulfane

(4-Bromo-5,6,7,8-tetrahydroisoquinolin-8-yl)methanesulfonate (0.13 g, 424.59 µmol) and dimethylsulfinimine (79.10 mg, 849.19 µmol) were dissolved in acetonitrile (6 mL), then cesium carbonate (415.02 mg, 1.27 mmol) was added under nitrogen protection. The mixture was reacted with stirring for 12 hours at 80°C. The reaction solution was evaporated to dryness and purified by fast silica gel chromatographic plate (petroleum ether:ethyl acetate = 1: 5), to obtain the target product (4-bromo-5,6,7,8-tetrahydroisoquinolin-8-yl)imino-dimethyl-carbonylsulfane (16 mg, a white solid) with a yield of 12.43%.

MS m/z (ESI): 303.0, 305, 0 [M+1].

### Step 4

### 6-(8-((dimethyl(carbonyl)-16-sulfanylidene)amino)-5,6,7,8-tetrahydroisoquinolin-4-yl)-1-methyl-3,4-dihydroquinolin-2(1H)-one

(4-Bromo-5,6,7,8-tetrahydroisoquinolin-8-yl)imino-dimethyl-carbonylsulfane (16 mg, 52.77 µmol), 1-methyl-6-(4,4,5,5-tetramethyl-1,3,2-dioxaborolan-2-yl)-3,4-dihydroquinolin-2-one (30.31 mg, 105.53 µmol) and sodium carbonate (11.19 mg, 105.53 µmol) were dissolved in ethanol (10 mL) and water (2 mL). Tetrakis(triphenylphosphine)palladium (6.10 mg, 5.28 µmol) was added under nitrogen protection. The mixture was reacted with stirring for 12 hours at 90°C. The reaction solution was evaporated to dryness, and the crude product was separated by prep-HPLC to obtain the target product.

MS m/z (ESI): 384.2 [M+1].

### Example 28

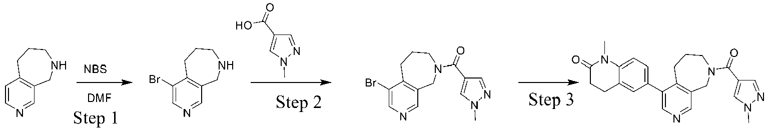

### Step 1

### 4-Bromo-6,7,8,9-tetrahydro-5H-pyrido[3,4-c]azepine

In a 50 mL reaction flask, 6,7,8,9-tetrahydro-5H-pyrido[3,4-c]azepine (1 g, 6.75 mmol) was dissolved in DMF (20 mL). Then, NBS (1.20 g, 6.75 mmol) was added at 25°C, and the reaction solution was stirred at 80°C for 3 hours. After the reaction was stopped, the reaction was quenched by adding water (10 mL), the mixture was concentrated and extracted with ethyl acetate (10 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product 4-bromo-6,7,8,9-tetrahydro-5H-pyrido[3,4-c]azepine (300 mg, a yellow solid) with a yield of 19.5%.

MS m/z (ESI): 227.0 [M+1].

### Step 2

### (4-Bromo-5,6,7,9-tetrahydro-8H-pyrido[3,4-c]azepin-8-yl)(1-methyl-1H-pyrazol-4-yl)methanone

Referring to step 3 of Example 25, with 4-bromo-6,7,8,9-tetrahydro-5H-pyrido[3,4-c]azepine as a starting material, the product (4-bromo-5,6,7,9-tetrahydro-8H-pyrido[3,4-c]azepin-8-yl)(1-methyl-1H-pyrazol-4-yl)methanone was obtained.

MS m/z (ESI): 335.0 [M+1].

### Step 3

### Methyl-6-(8-(1-methyl-1H-pyrazole-4-carbonyl)-6,7,8,9-tetrahydro-5H-pyrido[3,4-c]azepin-4-yl)-3,4-dihydroquinolin-2(1H)-one

Referring to step 4 of Example 1, with (4-bromo-5,6,7,9-tetrahydro-8H-pyrido[3,4-c]azepin-8-yl)(1-methyl-1H-pyrazol-4-yl)methanone as a starting material, the product was obtained.

MS m/z (ESI): 416.2 [M+1].

### Example 29

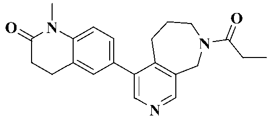

Referring to steps 2 and 3 of Example 28, with 4-bromo-6,7,8,9-tetrahydro-5H-pyrido[3,4-c]azepine as a starting material, the product 1-methyl-6-(8-propionyl-6,7,8,9-tetrahydro-5H-pyrido[3,4-c]azepin-4-yl)-3,4-dihydroquinolin-2(1H)-one was obtained.

MS m/z (ESI): 364.1 [M+1].

### Example 30

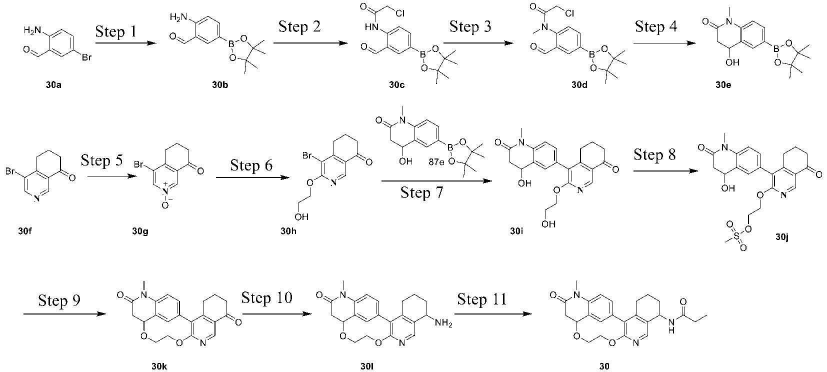

### Step 1

### 2-Amino-5-(4,4,5,5-tetramethyl-1,3,2-dioxaborolan-2-yl)benzaldehyde

Referring to step 2 of the synthesis route of Example 1, the title product 30b was obtained.

MS m/z (ESI): 248.1[M+1].

### Step 2

### 2-Chloro-N-(2-formyl-4-(4,4,5,5-tetramethyl-1,3,2-dioxaborolan-2-yl)phenyl)acetamide

Referring to step 3 of the synthesis route of Example 1, the title product 30c was obtained.

MS m/z (ESI): 324.1[M+1].

### Step 3

### 2-Chloro-N-(2-formyl-4-(4,4,5,5-tetramethyl-1,3,2-dioxaborolan-2-yl)phenyl)-N-methylacetamide

A mixture of 2-chloro-N-(2-formyl-4-(4,4,5,5-tetramethyl-1,3,2-dioxaborolan-2-yl)phenyl)acetamide 30c (2.1 g, 6.5 mmol), cesium carbonate (4.2 g, 13 mmol), iodomethane (1.8 g, 13 mmol) and acetonitrile (25 mL) was stirred at 80°C for 12 hours. Water was added, and the mixture was extracted with dichloromethane. The organic phase was washed with a saturated aqueous sodium chloride solution, dried over anhydrous sodium sulfate, filtered, and rotary-evaporated to dryness. The residue was then separated by column chromatography to obtain the title product 30d (1.5 g, a yellow solid) with a yield of 68%.

MS m/z (ESI): 338.1[M+1].

### Step 4

### 4-Hydroxy-1-methyl-6-(4,4,5,5-tetramethyl-1,3,2-dioxaborolan-2-yl)-3,4-dihydroquinolin-2(1H)-one

To a solution of 2-chloro-N-(2-formyl-4-(4,4,5,5-tetramethyl-1,3,2-dioxaborolan-2-yl)phenyl)-N-methylacetamide **30d** (1.5 g, 4.45 mmol) in tetrahydrofuran (20 mL) was added dropwise samarium diiodide (89 mL, 8.9 mmol, 0.1 M). The mixture was stirred at room temperature for 1 hour. Water was added, and the mixture was extracted with dichloromethane. The organic phase was washed with a saturated aqueous sodium chloride solution, dried over anhydrous sodium sulfate, filtered, and rotary-evaporated to dryness. The residue was then separated by column chromatography to obtain the title product 30e (700 mg, a yellow solid) with a yield of 52%.

MS m/z (ESI): 304.2[M+1].

### Step 5

### 4-Bromo-8-carbonyl-5,6,7,8-tetrahydroisoquinoline 2-oxide

4-Bromo-6,7-dihydro-5H-isoquinolin-8-one 30f (2.5 g, 11.06 mmol) was dissolved in dichloromethane (30 mL), then 3-chloroperoxybenzoic acid (4.49 g, 22.12 mmol, 85%) was added. The mixture was stirred at room temperature for 1.5 hours, then a saturated aqueous sodium bicarbonate solution was added, and the mixture was extracted with dichloromethane. The organic phase was washed with a saturated aqueous sodium chloride solution, dried over anhydrous sodium sulfate, filtered, and rotary-evaporated to dryness. The residue was then separated by column chromatography to obtain **30g** (1.1 g, a pale yellow oil) with a yield of 41%

MS m/z (ESI): 242.0[M+1].

### Step 6

### 4-Bromo-3-(2-hydroxyethoxy)-6,7-dihydroisoquinolin-8(5H)-one

4-Bromo-8-carbonyl-5,6,7,8-tetrahydroisoquinoline 2-oxide **30g** (1.1 g, 4.54 mmol) was dissolved in carbon tetrachloride (15 mL). Ethylene glycol (563 mg, 9.08 mmol) and N,N-diisopropylethylamine (1.2 g, 9.08 mmol) were added, followed by the dropwise addition of a solution of diethyl phosphite (1.25 g, 9.08 mmol) in acetonitrile (10 mL). The mixture was then stirred at 60°C for 12 hours. Water was added, and the mixture was extracted with dichloromethane. The organic phase was washed with a saturated aqueous sodium chloride solution, dried over anhydrous sodium sulfate, filtered, and rotary-evaporated to dryness. The residue was then separated by column chromatography to obtain **30h** (400 mg, a pale yellow oil) with a yield of 31%

MS m/z (ESI): 286.0[M+1].

### Step 7

### 4-Hydroxy-6-(3-(2-hydroxyethoxy)-8-carbonyl-5,6,7,8-tetrahydroisoquinolin-4-yl)-1-methyl-3,4-dihydroquinolin-2(1H)-one

Referring to step 4 of the synthesis route of Example 1, the title product 30i was obtained.

MS m/z (ESI): 383.2[M+1].

### Step 8

### 2-((4-(4-Hydroxy-1-methyl-2-carbonyl-1,2,3,4-tetrahydroquinolin-6-yl)-8-carbonyl-5,6,7,8-tetrahydroisoquinolin-3-yl)oxy)ethyl methanesulfonate

Referring to step 3 of the synthesis route of Example 1, and replacing propionyl chloride with methanesulfonyl chloride, the title product 30j was obtained.

MS m/z (ESI): 461.1[M+1].

### Step 9

### 2-Methyl-4,4a,6,7,13,14-hexahydro-1,15-(cycloethane[1,2]diylidene)pyrido[3',4':9,10][1,4]dioxadecino[5,6-c]isoquinolin-3,11(2H,12H)-dione

To a solution of 30j (300 mg, 0.65 mmol) in tetrahydrofuran was added sodium hydride (52 mg, 1.3 mmol, 60%). The mixture was then stirred at room temperature for 12 hours. Water was added, and the mixture was extracted with dichloromethane. The organic phase was washed with a saturated aqueous sodium chloride solution, dried over anhydrous sodium sulfate, filtered, and rotary-evaporated to dryness. The residue was then separated by column chromatography to obtain 30k (100 mg, a pale yellow oil) with a yield of 42%

MS m/z (ESI): 365.1[M+1].

### Step 10

### 11-Amino-2-methyl-4,4a,6,7,11,12,13,14-octahydro-1,15-(cycloethane[1,2]diylidene)pyrido[3',4':9,10][1,4]dioxadecino[5,6-c]isoquinolin-3(2H)-one

To a solution of 2-methyl-4,4a,6,7,13,14-hexahydro-1,15-(cycloethane[1,2]diylidene)pyrido[3',4':9,10][1,4]dioxadecino[5,6-c]isoquinolin-3,11(2H,12H)-dione 30k (100 mg, 0.27 mmol) and tetraisopropyl titanate (153 mg, 0.54 mmol) in methanol (5 mL) was added a solution of ammonia in methanol (2 mL, 7.0 M). The mixture was stirred at 50°C for 5 hours, and cooled. Water was added, and the mixture was extracted with dichloromethane. The organic phase was washed with a saturated aqueous sodium chloride solution, dried over anhydrous sodium sulfate, filtered, and rotary-evaporated to dryness to obtain an oil. This oil was then dissolved in methanol (3 mL), and sodium borohydride (21 mg, 0.54 mmol) was added. The mixture was stirred for 1 hour. Water was added, and the mixture was extracted with dichloromethane. The organic phase was washed with a saturated aqueous sodium chloride solution, dried over anhydrous sodium sulfate, filtered, and rotary-evaporated to dryness. The residue was then separated by column chromatography to obtain the title product 30l (20 mg, a pale yellow oil) with a yield of 20%

MS m/z (ESI): 366.2 [M+1].

### Step 11

### N-(2-methyl-3-carbonyl-2,3,4,4a,6,7,11,12,13,14-decahydro-1,15-(cycloethane[1,2]diylidene)pyrido[3',4':9,10][1,4]dioxadecino[5,6-c]isoquinolin-11-yl)propanamide

Referring to step 3 of the synthesis route of Example 1, the title product 30 was obtained.

MS m/z (ESI): 422.2[M+1].

### Example 31

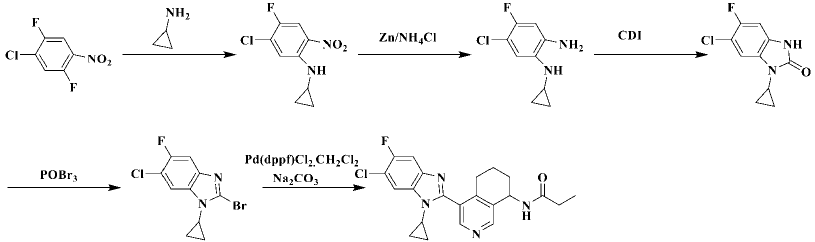

### Step 1

### 5-Chloro-N-cyclopropyl-4-fluoro-2-nitroaniline

In a 50 mL reaction flask, 1-chloro-2,5-difluoro-4-nitrobenzene (2 g, 10.33 mmol) was dissolved in ethanol (20 mL). Cyclopropylamine (590.04 mg, 10.33 mmol) was then added at 0°C, and the reaction solution was stirred at 0°C for 1 hour. After the reaction was stopped, the reaction was quenched by adding water (10 mL), the mixture was concentrated and extracted with ethyl acetate (10 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product (1.5 g, a yellow solid) with a yield of 62.9%.

MS m/z (ESI): 231.0 [M+1].

### Step 2

### 4-Chloro-N2-cyclopropyl-5-fluoro-benzene-1,2-diamine

In a 50 mL reaction flask, 5-chloro-N-cyclopropyl-4-fluoro-2-nitroaniline (1 g, 4.34 mmol) was dissolved in methanol (10 mL). Then, Zn (2.84 g, 43.36 mmol) and NH4Cl (1.16 g, 21.68 mmol) were added at 25°C, and the reaction solution was stirred at 25°C for 2 hours. After the reaction was stopped, the mixture was filtered, and the organic phase was concentrated and extracted with ethyl acetate (10 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product (500 mg, a yellow solid) with a yield of 57.5%.

MS m/z (ESI): 201.0 [M+1].

### Step 3

### 5-Chloro-3-cyclopropyl-6-fluoro-1H-benzimidazol-2-one

In a 50 mL reaction flask, 4-chloro-N2-cyclopropyl-5-fluoro-benzene-1,2-diamine (500 mg, 2.49 mmol) was dissolved in tetrahydrofuran (10 mL). Then 1,1'-carbonyldiimidazole (606.12 mg, 3.74 mmol) was added at 25°C, and the reaction solution was stirred at 25°C for 10 hours. After the reaction was stopped, the reaction was quenched by adding water (10 mL), and the mixture extracted with ethyl acetate (10 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product (300 mg, a yellow solid) with a yield of 53.1%.

MS m/z (ESI): 227.0 [M+1].

### Step 4

### 2-Bromo-6-chloro-1-cyclopropyl-5-fluoro-benzimidazole

In a 25 mL reaction flask, 5-chloro-3-cyclopropyl-6-fluoro-1H-benzimidazol-2-one (100 mg, 441.24 µmol) was dissolved in toluene (5 mL). Then, POBr3 (379.49 mg, 1.32 mmol) was added, and the reaction solution was stirred at 80°C for 3 hours. After the reaction was stopped, the reaction was quenched by adding water (5 mL), and the mixture extracted with ethyl acetate (5 mL × 2). The combined organic phases were washed with saturated sodium chloride (5 mL), dried over anhydrous sodium sulfate, and filtered, and the resulting residue was purified by silica gel column chromatography with petroleum ether and ethyl acetate as an eluent system to obtain the title product (80 mg, a yellow solid) with a yield of 62.6%.

MS m/z (ESI): 288.9 [M+1].

### Step 5

### N-(4-(6-chloro-1-cyclopropyl-5-fluoro-1H-benzo[d]imidazol-2-yl)-5,6,7,8-tetrahydroisoquinolin-8-yl)propanamide

Referring to step 4 of Example 1, with 2-bromo-6-chloro-1-cyclopropyl-5-fluoro-benzimidazole as a starting material, the product was obtained.

MS m/z (ESI): 413.1 [M+1].

### Example 32

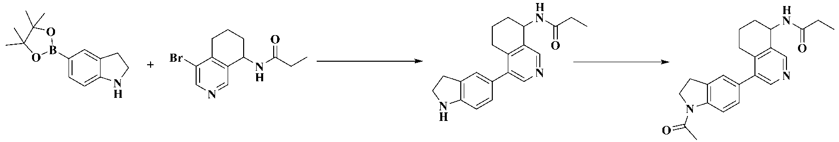

### Step 1

N-(4-(indolin-5-yl)-5,6,7,8-tetrahydroisoquinolin-8-yl)propanamide Referring to step 3 of Example 13, with N-(4-bromo-5,6,7,8-tetrahydroisoquinolin-8-yl)propanamide and 5-(4,4,5,5-tetramethyl-1,3,2-dioxaborolan-2-yl)indoline as starting materials, the target compound was obtained.

MS m/z (ESI): 322.1[M+1].

### Step 2

### N-(4-(1-acetyl indolin-5-yl)-5,6,7,8-tetrahydroisoquinolin-8-yl)propanamide

In an ice bath, N-(4-(indolin-5-yl)-5,6,7,8-tetrahydroisoquinolin-8-yl)propanamide (0.08 g, 0.25 mmol) was dissolved in tetrahydrofuran (5 mL). Triethylamine (0.05 g, 0.50 mmol) was then added, followed by the dropwise addition of acetyl chloride (23.5 mg, 0.3 mmol). The mixture was stirred at room temperature for 2 hours, and LCMS indicated the completion of the reaction. The resultant was rotary-evaporated to dryness, and the residue was dissolved in ethyl acetate (30 mL), then washed sequentially with a saturated sodium bicarbonate solution (10 mL × 2) and a saturated aqueous sodium chloride solution (10 mL × 2). The organic phase was dried over anhydrous sodium sulfate, filtered, and rotary-evaporated to dryness. The residue was separated by reverse-phase preparative chromatography (neutral) and lyophilized to obtain the target product (20 mg) with a yield of 22.1%.

MS m/z (ESI): 364.1[M+1].

### Example 33

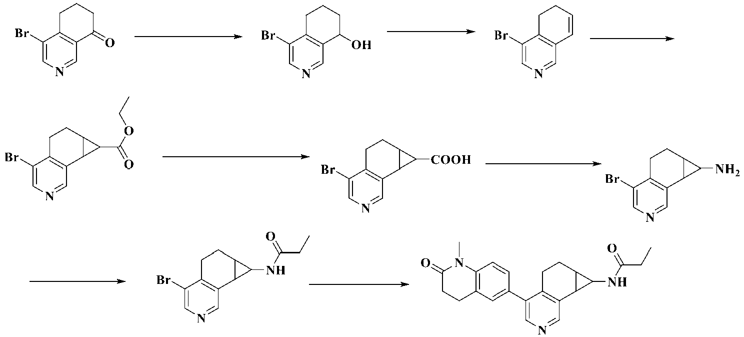

### Step 1

In an ice bath, 4-bromo-6,7-dihydroisoquinolin-8(5H)-one (2.26 g, 10 mmol) was dissolved in methanol (30 mL). Then, sodium borohydride (0.57 g, 15 mmol) was slowly added, and the mixture was stirred in an ice bath for 1 hour. LCMS indicated the completion of the reaction. The reaction solution was quenched with a saturated ammonium chloride solution (30 mL), and methanol was removed by rotary evaporation. The aqueous phase was extracted with ethyl acetate (20 mL × 3). The organic phases were combined, dried over anhydrous sodium sulfate, filtered, and rotary-evaporated to dryness to obtain the crude product 4-bromo-5,6,7,8-tetrahydroisoquinolin-8-ol (2.10 g) with a yield of 92%.

MS m/z (ESI): 228.1, 230.1[M+1].

### Step 2

In an ice bath, 4-bromo-5,6,7,8-tetrahydroisoquinolin-8-ol (2.10 g, 9.2 mmol) was dissolved in dichloromethane (30 mL). Then triethylamine (2.79 g, 27.6 mmol) and trifluoroacetic anhydride (3.86 g, 18.4 mmol) were added, and the mixture was stirred at room temperature overnight. LCMS indicated the completion of the reaction. The reaction solution was diluted with dichloromethane (30 mL), then washed with a saturated sodium bicarbonate solution (10 mL × 2) and a saturated aqueous sodium chloride solution (20 mL ×1). The organic phase was dried over anhydrous sodium sulfate, filtered, and rotary-evaporated to dryness. The residue was separated by Flash column chromatography to obtain the target product 4-bromo-5,6-dihydroisoquinoline (1.0 g) with a yield of 51.8%.

MS m/z (ESI): 210.1, 212.1[M+1].

### Step 3

At room temperature, 4-bromo-5,6-dihydroisoquinoline (1.0 g, 4.76 mmol) was dissolved in methyl tert-butyl ether (20 mL). Then ethyl diazoacetate (1.09 g, 9.52 mmol), palladium acetate (0.107 g, 0.476 mmol) and triethylamine (1.44 g, 14.28 mmol) were added. The atmosphere was displaced with nitrogen, and the mixture was heated to 80°C and reacted for 14 hours. The mixture was cooled to room temperature. LCMS indicated the completion of the reaction. The reaction solution was quenched with a saturated ammonium chloride solution (30 mL), then extracted with ethyl acetate (20 mL × 3). The organic phases were combined, dried over anhydrous sodium sulfate, filtered, and rotary-evaporated to dryness. The residue was separated by Flash column chromatography to obtain ethyl 4-bromo-6,6a,7,7a-tetrahydro-5H-cyclopropa[h]isoquinoline-7-carboxylate (0.50 g) with a yield of 35.7%.

MS m/z (ESI): 296.1, 298.1[M+1].

### Step 4

At room temperature, 4-bromo-6,6a,7,7a-tetrahydro-5H-cyclopropa[h]isoquinoline-7-carboxylate (0.50 g, 1.69 mmol) was dissolved in tetrahydrofuran (10 mL). Then an aqueous sodium hydroxide solution (2N, 5 mL) was added, and the mixture was stirred at room temperature for 2 hours. LCMS indicated the completion of the reaction. The pH of the reaction solution was adjusted to 6-7 with dilute hydrochloric acid. The reaction solution was extracted with ethyl acetate (10 mL × 3). The organic phases were combined, dried over anhydrous sodium sulfate, filtered, and rotary-evaporated to dryness to obtain the crude product 4-bromo-6,6a,7,7a-tetrahydro-5H-cyclopropa[h]isoquinoline-7-carboxylic acid (0.35 g) with a yield of 77.8%.

MS m/z (ESI): 268.1, 270.1[M+1].

### Step 5

At room temperature, 4-bromo-6,6a,7,7a-tetrahydro-5H-cyclopropa[h]isoquinoline-7-carboxylic acid (0.35 g, 1.31 mmol) was dissolved in tetrahydrofuran (6 mL). Then, triethylamine (0.27 g, 2.62 mmol) was added, and the atmosphere was displaced with nitrogen. Then diphenylphosphoryl azide (0.43 g, 1.57 mmol) was added, and the mixture was heated to 70°C and reacted for 3 hours. LCMS indicated the completion of the reaction. After cooling to room temperature, the mixture was quenched by adding water (5 mL), heated again to 70°C and reacted for 3 hours. LCMS indicated the completion of the reaction. After cooling to room temperature, the reaction solution was extracted with ethyl acetate (20 mL × 3). The organic phases were combined, dried over anhydrous sodium sulfate, filtered, and rotary-evaporated to dryness. The residue was separated by Flash column chromatography to obtain 4-bromo-6,6a,7,7a-tetrahydro-5H-cyclopropa[h]isoquinoline-7-amine (0.20 g) with a yield of 51.9%.

MS m/z (ESI): 239.1, 241.1[M+1].

### Step 6

Referring to step 3 of Example 1, with 4-bromo-6,6a,7,7a-tetrahydro-5H-cyclopropa[h]isoquinolin-7-amine as a starting material, the product N-(4-bromo-6,6a,7,7a-tetrahydro-5H-cyclopropa[h]isoquinolin-7-yl)propanamide was obtained.

MS m/z (ESI): 295.1, 297.1[M+1].

### Step 7

Referring to step 3 of Example 13, with N-(4-bromo-6,6a,7,7a-tetrahydro-5H-cyclopropa[h]isoquinolin-7-yl)propanamide as a starting material, the product N-(4-(1-methyl-2-carbonyl-1,2,3,4-tetrahydroquinolin-6-yl)-6,6a,7,7a-tetrahydro-5H-cyclopropa[h]isoquinolin-7-yl)propanamide was obtained.

MS m/z (ESI): 376.2[M+1].

### Example 34

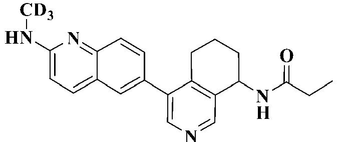

Referring to Example 17, with 2-amino-6-bromoquinoline as a starting material, the product N-(4-(2-((methyl-d3)amino)quinolin-6-yl)-5,6,7,8-tetrahydroisoquinolin-8-yl)propanamide was obtained.

MS m/z (ESI): 364.2 [M+1].

### Example 35

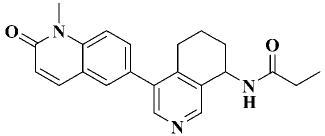

Referring to Example 1, with 6-bromo-1-methyl-1H-quinolin-2-one as a starting material, the product N-(4-(1-methyl-2-oxo-1,2-dihydroquinolin-6-yl)-5,6,7,8-tetrahydroisoquinolin-8-yl)propanamide was obtained.

MS m/z (ESI): 362.2 [M+1].

Example 35 was resolved to obtain 35-A and 35-B.

| | | |
|---|---|---|
| 35-A | 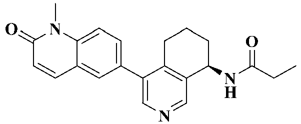 | MS m/z (ESI): 362.2 [M+1]. |
| 35-B | 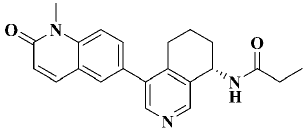 | MS m/z (ESI): 362.2 [M+1]. |

### Example 36

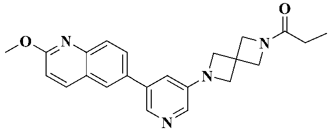

Referring to Example 23, the product 1-(6-(5-(2-methoxyquinolin-6-yl)pyridin-3-yl)-2,6-diazaspiro[3.3]heptan-2-yl)propan-1-one was obtained.

MS m/z (ESI): 389.2 [M+1].

¹H NMR (400 MHz, DMSO) δ 8.29 (d, 1H), 8.24 (d, 1H), 8.17 (d, 1H), 7.94 (dd, 1H), 7.84 - 7.75 (m, 2H), 7.11 (t, 1H), 7.00 (d, 1H), 4.24 (s, 2H), 4.04 (s, 4H), 3.98 (s, 2H), 3.94 (s, 3H), 1.99 (q, 2H), 0.90 (t, 3H).

### Example 37

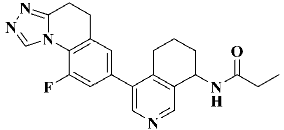

Referring to Example 1, the product N-(4-(9-fluoro-4,5-dihydro-[1,2,4]triazolo[4,3-a]quinolin-7-yl)-5,6,7,8-tetrahydroisoquinolin-8-yl)propanamide was obtained.

MS m/z (ESI): 392.2 [M+1].

### Example 38

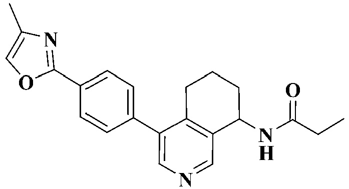

Referring to Example 13, the product N-(4-(4-(4-methyloxazol-2-yl)phenyl)-5,6,7,8-tetrahydroisoquinolin-8-yl)propanamide was obtained.

MS m/z (ESI): 362.2[M+1].

### Example 39

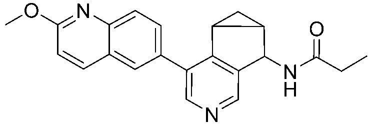

Referring to step 7 of Example 2, with 2-methoxy-6-(4,4,5,5-tetramethyl-1,3,2-dioxaborolan-2-yl)quinoline and N-(4-bromo-4b,5,5a,6-tetrahydrocyclopropa[3,4]cyclopenta[1,2-c]pyridin-6-yl)propanamide as raw materials, N-((4bR,5aS)-4-(2-methoxyquinolin-6-yl)-4b,5,5a,6-tetrahydrocyclopropa[3,4]cyclopenta[1,2-c]pyridin-6-yl)propanamide was synthesized.

MS m/z (ESI): 360.2 [M+1].

### Example 40

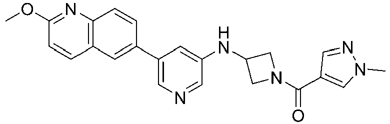

Referring to Example 5, with 3,5-dibromopyridine and tert-butyl 3-aminoazetidine-1-carboxylate as starting materials, the product (3-((5-(2-methoxyquinolin-6-yl)pyridin-3-yl)amino)azetidin-1-yl)(1-methyl-1H-pyrazol-4-yl)methanone was obtained.

MS m/z (ESI): 415.2 [M+1].

¹H NMR (400 MHz, DMSO-d₆) δ 8.31 (d, *J=* 8.8 Hz, 1H), 8.28 (d, *J* = 2.0 Hz, 1H), 8.21 (d, *J* = 2.0 Hz, 1H), 8.16 (s, 1H), 8.01-7.98 (m, 2H), 7.87 (d, *J* = 8.8 Hz, 1H), 7.75 (s, 1H),7.21 (d, *J =* 2.0 Hz, 1H), 7.07 (d, *J =* 8.8 Hz, 1H), 6.77 (d, *J* = 7.2 Hz, 1H), 4.79-4.72 (m, 1H), 4.53-4.37 (m, 2H), 4.13-4.10(m, 1H), 4.01 (s, 3H), 3.91-3.87 (m, 1H), 3.85 (s, 3H),

### Example 41

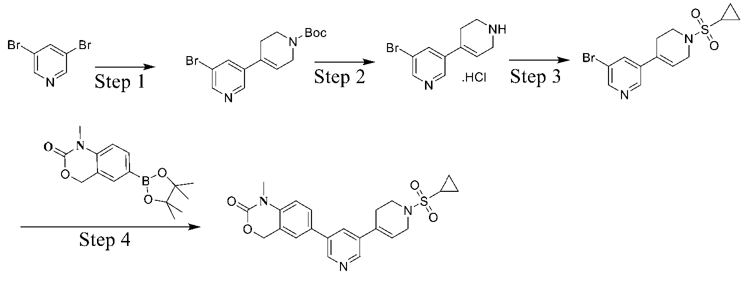

### Step 1

3,5-Dibromopyridine (0.38 g, 1.60 mmol), tert-butyl 4-(4,4,5,5-tetramethyl-1,3,2-dioxaborolan-2-yl)-3,6-dihydropyridin-1(2H)-carboxylate (496.00 mg, 1.60 mmol), and sodium carbonate (510.05 mg, 4.81 mmol) were dissolved in 1'4-dioxane (12 mL) and water (3 mL), and under nitrogen protection, bis(triphenyl)palladium dichloride (56.30 mg, 80.21 µmol) was added. The mixture was reacted with stirring for 5 hours at 90°C. The reaction was quenched by adding a saturated aqueous sodium chloride solution (50 mL) to the mixture, and the mixture was extracted with ethyl acetate (30 mL × 3). The organic phases were combined, sequentially washed with a saturated aqueous sodium chloride solution (50 mL × 2), dried over anhydrous sodium sulfate, and concentrated under reduced pressure. The residue was purified by flash silica gel chromatography (eluted with petroleum ether: ethyl acetate = 100 : 0 to 70 : 30) to obtain the target product tert-butyl 5-bromo-3',6'-dihydro-[3,4'-bipyridine]-1'(2'H)-carboxylate (0.32 g, a white solid) with a yield of 58.81%.

MS m/z (ESI): 339.1, 341.1 [M+1].

### Step 2

Tert-butyl 5-bromo-3',6'-dihydro-[3,4'-bipyridine]-1'(2'H)-carboxylate (0.2 g, 589.58 µmol) was dissolved in hydrogen chloride dioxane (4 M, 10 mL), and the mixture was reacted with stirring for 2 hours at 20°C. The reaction solution was concentrated under reduced pressure to obtain crude 5-bromo-1',2',3',6'-tetrahydro-3,4'-bipyridine hydrochloride (0.16 g). The crude product was directly used for the next step.

MS m/z (ESI): 239.0, 241.0 [M+1].

### Step 3

5-Bromo-1',2',3',6'-tetrahydro-3,4'-bipyridine hydrochloride (0.16 g, 580.61 µmol, CL) and triethylamine (293.76 mg, 2.90 mmol, 404.90 µL) were dissolved in dichloromethane (10 mL), and under nitrogen protection, cyclopropylsulfonyl chloride (106.12 mg, 754.79 µmol) was added. The mixture was reacted with stirring for 1 hours at 20°C. The reaction solution was washed with a saturated aqueous sodium chloride solution (30 mL × 2). The organic phase was separated, dried over anhydrous sodium sulfate, and concentrated under reduced pressure to obtain crude 5-bromo-1'-(cyclopropylsulfonyl)-1',2',3',6'-tetrahydro-3,4'-bipyridine (0.19 g). The crude product was directly used for the next step.

MS m/z (ESI): 343.0, 345.0 [M+1].

### Step 4

Referring to step 4 of Example 1, with 1-methyl-6-(4,4,5,5-tetramethyl-1,3,2-dioxaborolan-2-yl)-1,4-dihydro-2H-benzo[d][1,3]oxazin-2-one and 5-bromo-1'-(cyclopropylsulfonyl)-1',2',3',6'-tetrahydro-3,4'-bipyridine as starting materials, 6-(1'-(cyclopropylsulfonyl)-1',2',3',6'-tetrahydro-[3,4'-bipyridine]-5-yl)-1-methyl-1,4-dihydro-2H-benzo[d][1,3]oxazin-2-one was synthesized.

MS m/z (ESI): 426.1 [M+1].

### Example 42

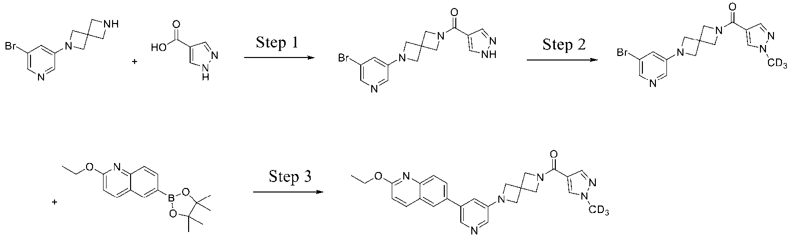

### Step 1

2-(5-Bromopyridin-3-yl)-2,6-diazaspiro[3.3]heptane (120 mg, 0.472 mmol), 1H-pyrazole-4-carboxylic acid (64 mg, 0.567 mmol), HATU (269 mg, 0.708 mmol), and triethylamine (143 mg, 1.42 mmol) were dissolved in DMF (5 mL), and the reaction mixture was stirred at room temperature for 18 hours. The reaction solution was concentrated under reduced pressure, and the resulting residue was purified by silica gel column chromatography with methanol and ethyl acetate as an eluent system to obtain the title product 42a (98 mg, a colorless oil) with a yield of 59.6%.

MS m/z (ESI): 348.0, 350.0 [M+1].

### Step 2

42a (98 mg, 0.281 mmol), potassium carbonate (117 mg, 0.843 mmol), and deuterated iodomethane (49 mg, 0.338 mmol) were dispersed in DMF (5 mL), and the reaction mixture was stirred at room temperature for 18 hours. The reaction solution was filtered through celite, the filtrate was concentrated under reduced pressure, and the resulting residue was purified by silica gel column chromatography with methanol and ethyl acetate as an eluent system to obtain the title product 42b (78 mg, a colorless oil) with a yield of 75.9%.

MS m/z (ESI): 365.1, 367.1 [M+1].

### Step 3

Referring to the synthesis conditions of Example 5, the title product 6-(5-(2-ethoxyquinolin-6-yl)pyridin-3-yl)-2,6-diazaspiro[3.3]heptan-2-yl)(1-(methyl-d3)-1H-pyrazol-4-yl)methanone was synthesized.

MS m/z (ESI): 458.2[M+1].

### Example 43

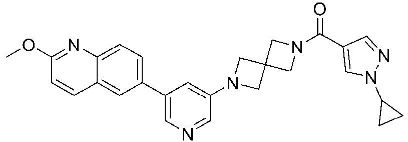

Referring to Example 5, with 3,5-dibromopyridine and tert-butyl 2,6-diazaspiro[3.3]heptane-2-carboxylate as starting materials, the product (1-cyclopropyl-1H-pyrazol-4-yl)(6-(5-(2-methoxyquinolin-6-yl)pyridin-3-yl)-2,6-diazaspiro[3.3]heptan-2-yl)methanone was obtained.

MS m/z (ESI): 467.2 [M+1].

¹H NMR (400 MHz, MeOD) δ 8.28 (s, 1H), 8.20 (d, 1H), 8.13 - 8.06 (m, 2H), 7.93 (d, 2H), 7.81 (s, 2H), 7.27 - 7.21 (m, 1H), 6.99 (d, 1H), 4.69 (s, 2H), 4.34 (s, 2H), 4.22 (s, 4H), 4.07 (s, 3H), 3.77 - 3.68 (m, 1H), 1.16 - 1.12 (m, 2H), 1.12 - 1.04 (m, 2H).

### Example 44

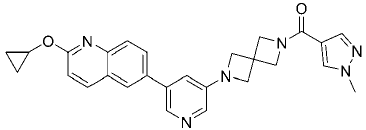

Referring to the synthesis conditions of Example 5, the title product (6-(5-(2-cyclopropoxyquinolin-6-yl)pyridin-3-yl)-2,6-diazaspiro[3.3]heptan-2-yl)(1-methyl-1H-pyrazol-4-yl)methanone was synthesized.

MS m/z (ESI): 467.2[M+1].

### Example 45

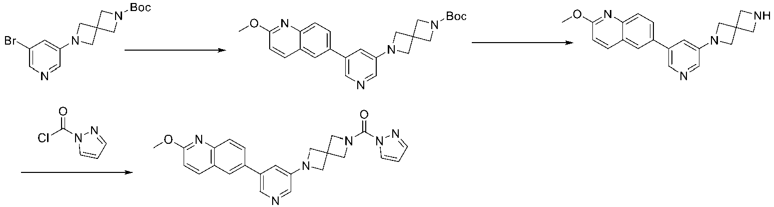

### Step 1

Referring to step 4 of Example 1, with tert-butyl 6-(5-bromopyridin-3-yl)-2,6-diazaspiro[3.3]heptane-2-carboxylate as a starting material, the product tert-butyl 6-(5-(2-methoxyquinolin-6-yl)pyridin-3-yl)-2,6-diazaspiro[3.3]heptane-2-carboxylate was obtained.

MS m/z (ESI): 433.2 [M+1].

### Step 2

Referring to step 2 of Example 4, with tert-butyl 6-(5-(2-methoxyquinolin-6-yl)pyridin-3-yl)-2,6-diazaspiro[3.3]heptane-2-carboxylate as a starting material, the product 6-(5-(2,6-diazaspiro[3.3]heptan-2-yl)pyridin-3-yl)-2-methoxyquinoline was obtained.

MS m/z (ESI): 333.2 [M+1].

### Step 3

Referring to step 3 of Example 1, with 6-(5-(2,6-diazaspiro[3.3]heptan-2-yl)pyridin-3-yl)-2-methoxyquinoline and 1H-pyrazol-1-carbonyl chloride as starting materials, the product (6-(5-(2-methoxyquinolin-6-yl)pyridin-3-yl)-2,6-diazaspiro[3.3]heptan-2-yl)(1H-pyrazol-1-yl)methanone was obtained.

MS m/z (ESI): 427.2 [M+1].

### Example 46

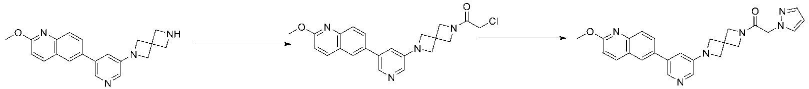

### Step 1

Referring to step 3 of Example 1, with 6-(5-(2,6-diazaspiro[3.3]heptan-2-yl)pyridin-3-yl)-2-methoxyquinoline and chloroacetyl chloride as starting materials, the product 2-chloro-1-(6-(5-(2-methoxyquinolin-6-yl)pyridin-3-yl)-2,6-diazaspiro[3.3]heptan-2-yl)ethan-1-one was obtained.

MS m/z (ESI): 409.1 [M+1].

### Step 2

At room temperature, 2-chloro-1-(6-(5-(2-methoxyquinolin-6-yl)pyridin-3-yl)-2,6-diazaspiro[3.3]heptan-2-yl)ethan-1-one (40 mg, 0.1 mmol) was dissolved in N,N-dimethylformamide (2 mL). Then potassium carbonate (41.5 mg, 0.3 mmol) was added followed by addition of pyrazole (13.6 mg, 0.2 mmol). The mixture was heated to 100°C, reacted for 14 hours and cooled to room temperature. LCMS indicated the completion of the reaction. The reaction solution was dissolved in ethyl acetate (30 mL), then washed with a saturated aqueous sodium chloride solution (10 mL × 2). The organic phase was dried over anhydrous sodium sulfate, filtered, and rotary-evaporated to dryness. The residue was purified by reverse-phase preparative chromatography to obtain the product: 1-(6-(5-(2-methoxyquinolin-6-yl)pyridin-3-yl)-2,6-diazaspiro[3.3]heptan-2-yl)-2-(1H-pyrazol-1-yl)ethan-1-one.

MS m/z (ESI): 441.2 [M+1].

### Example 47

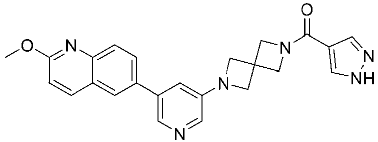

Referring to the synthesis conditions of Example 5, the title product (6-(5-(2-methoxyquinolin-6-yl)pyridin-3-yl)-2,6-diazaspiro[3.3]heptan-2-yl)(1H-pyrazol-4-yl)methanone was synthesized.

MS m/z (ESI): 427.2[M+1].

### Example 48

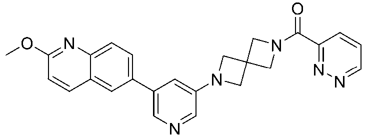

Referring to Example 7, with 6-bromo-2-methoxyquinoline, 3,5-dibromopyridine, pyridazine-3-carboxylic acid, tert-butyl 2,6-diazaspiro[3.3]heptane-2-carboxylate as starting materials, the target product (6-(5-(2-methoxyquinolin-6-yl)pyridin-3-yl)-2,6-diazaspiro[3.3]heptan-2-yl)(pyridazin-3-yl)methanone was finally obtained.

MS m/z (ESI): 439.2 [M+1].

### Example 49

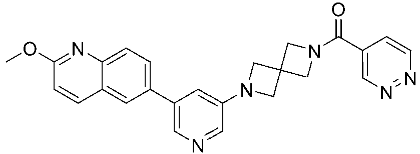

Referring to Example 7, with 6-bromo-2-methoxyquinoline, 3,5-dibromopyridine, pyridazine-4-carboxylic acid, tert-butyl 2,6-diazaspiro[3.3]heptane-2-carboxylate as starting materials, the target product 6-(5-(2-methoxyquinolin-6-yl)pyridin-3-yl)-2,6-diazaspiro[3.3]heptan-2-yl)(pyridazin-4-yl)methanone was finally obtained.

MS m/z (ESI): 439.2 [M+1].

### Example 50

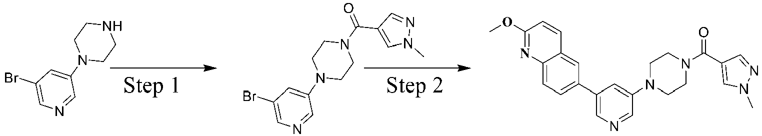

### Step 1

1-(5-bromopyridin-3-yl)piperazine (0.1 g, 413.03 µmol), 1-methylpyrazole-4-carboxylic acid (62.51 mg, 495.63 µmol) and N,N-diisopropylethylamine (160.14 mg, 1.24 mmol, 204.78 µL) were dissolved in N,N-dimethylformamide (4 mL). Under nitrogen protection, 2-(7-azabenzotriazol)-N,N,N',N'-tetramethyluronium hexafluorophosphate (235.57 mg, 619.54 µmol) was added. The mixture was reacted with stirring for 12 hours at 20°C. The reaction was quenched by adding a saturated aqueous sodium chloride solution (10 mL) to the reaction solution. The mixture was separated. The aqueous phase was extracted with dichloromethane (15 mL × 2). The organic phases were combined, sequentially washed with a saturated aqueous sodium chloride solution (20 mL × 5), dried over anhydrous sodium sulfate, and concentrated under reduced pressure. The residue was purified by flash silica gel chromatography (eluted with dichloromethane:methanol = 100: 0 to 95: 5) to obtain the target product (4-(5-bromopyridin-3-yl)piperazin-1-yl)(1-methyl-1H-pyrazol-4-yl)methanone (0.1 g, a white solid) with a yield of 69.13%.

MS m/z (ESI): 350.1, 352.1 [M+1].

### Step 2

(4-(5-Bromopyridin-3-yl)piperazin-1-yl)(1-methyl-1H-pyrazol-4-yl)methanone (30 mg, 85.66 µmol), 2-methoxy-6-(4,4,5,5-tetramethyl-1,3,2-dioxaborolan-2-yl)quinoline (24.43 mg, 85.66 µmol) and sodium carbonate (27.24 mg, 256.99 µmol) were dissolved in ethanol (3 mL) and water (0.5 mL). Under nitrogen protection, tetrakis(triphenylphosphine)palladium (9.90 mg, 8.57 µmol) was added. The mixture was reacted with stirring for 12 hours at 90°C. The reaction solution was concentrated under reduced pressure to obtain a crude product. The crude product was separated by preparative high-performance liquid chromatography to obtain (4-(5-(2-methoxyquinolin-6-yl)pyridin-3-yl)piperazin-1-yl)(1-methyl-1H-pyrazol-4-yl)methanone (7 mg) with a yield of 18.88%.

MS m/z (ESI): 429.2 [M+1].

¹H NMR (400 MHz, MeOD) δ 8.43 - 8.33 (m, 1H), 8.33 - 8.25 (m, 1H), 8.24 - 8.15 (m, 1H), 8.12 - 8.07 (m, 1H), 8.00 (s, 1H), 7.98 - 7.90 (m, 2H), 7.77 (s, 1H), 7.76 - 7.70 (m, 1H), 7.00 (d, J = 8.9 Hz, 1H), 4.07 (s, 3H), 4.00 - 3.88 (m, 7H), 3.53 - 3.39 (m, 4H).

### Example 51

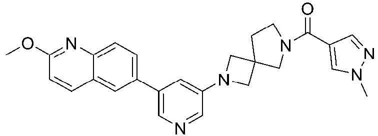

Referring to Example 5, with 3,5-dibromopyridine and tert-butyl 2,6-diazaspiro[3.4]octane-6-carboxylate as starting materials, the product (2-(5-(2-methoxyquinolin-6-yl)pyridin-3-yl)-2,6-diazaspiro[3.4]octane-6-yl)(1-methyl-1H-pyrazol-4-yl)methanone was obtained.

MS m/z (ESI): 455.2 [M+1].

1H NMR (400 MHz, MeOD) δ 8.29 - 8.23 (m, 1H), 8.19 (dd, 1H), 8.13 - 8.04 (m, 2H), 7.97 - 7.86 (m, 3H), 7.84 - 7.77 (m, 1H), 7.27 - 7.19 (m, 1H), 7.02 - 6.95 (m, 1H), 4.09 - 3.96(m, 8H), 3.96 - 3.92 (m, 3H), 3.92 - 3.82 (m, 2H), 3.74 - 3.66 (m, 1H), 2.40 - 2.32 (m, 1H), 2.32 - 2.24 (m, 1H).

### Example 52

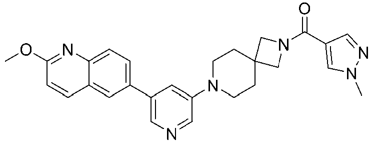

Referring to the synthesis conditions of Example 5, the title product (7-(5-(2-methoxyquinolin-6-yl)pyridin-3-yl)-2,7-diazaspiro[3.5]nonan-2-yl)(1-methyl-1H-pyrazol-4-yl)methanone was obtained.

MS m/z (ESI): 469.2 [M+1].

### Example 53

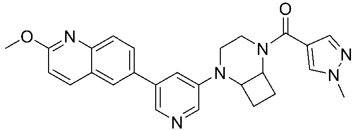

Referring to Example 5, the product (5-(5-(2-methoxyquinolin-6-yl)pyridin-3-yl)-2,5-diazabicyclo[4.2.0]octan-2-yl)(1-methyl-1H-pyrazol-4-yl)methanone was obtained.

MS m/z (ESI): 455.2 [M+1].

### Example 54

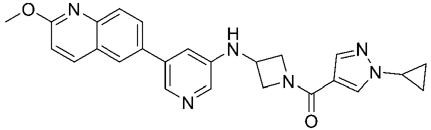

Referring to Example 5, the product (1-cyclopropyl-1H-pyrazol-4-yl)(3-((5-(2-methoxyquinolin-6-yl)pyridin-3-yl)amino)azetidin-1-yl)methanone was obtained.

MS m/z (ESI): 441.2 [M+1].

¹H NMR (400 MHz, DMSO-*d*₆) δ8.31 (d, *J=* 8.8 Hz, 1H), 8.28 (s, 1H), 8.22 (s, 1H), 8.20 (s, 1H), 8.01-7.98 (m, 2H), 7.87 (d, *J =* 8.8 Hz, 1H), 7.75 (s, 1H), 7.30-7.20 (m, 1H), 7.07 (d, *J =* 8.8 Hz, 1H), 6.77 (d, *J =* 6.8 Hz, 1H), 4.80-4.76 (m, 1H), 4.50-4.39 (m, 2H), 4.15-4.12 (m, 1H),4.01 (s, 3H), 3.90-3.86 (m, 1H), 3.80-3.74 (m, 1H), 1.09-1.06 (m, 2H), 0.98-0.94 (m, 2H).

### Example 55

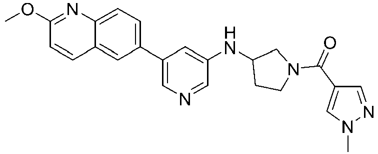

Referring to Example 5, the product (3-((5-(2-methoxyquinolin-6-yl)pyridin-3-yl)amino)pyrrolidin-1-yl)(1-methyl-1H-pyrazol-4-yl)methanone was obtained.

MS m/z (ESI): 429.1[M+1].

### Example 56

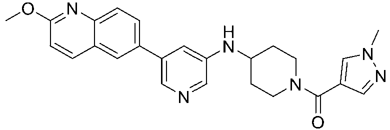

Referring to Example 5, the product (4-((5-(2-methoxyquinolin-6-yl)pyridin-3-yl)amino)piperidin-1-yl)(1-methyl-1H-pyrazol-4-yl)methanone was obtained.

MS m/z (ESI): 443.1[M+1].

¹H NMR (400 MHz, DMSO) δ 8.31 (d, *J =* 8.9 Hz, 1H), 8.18 (dd, *J* = 11.3, 2.0 Hz, 2H), 8.10 - 8.01 (m, 2H), 7.98 (dd, *J =* 8.7, 2.1 Hz, 1H), 7.87 (d, *J* = 8.7 Hz, 1H), 7.66 (s, 1H), 7.31 (t, *J* = 2.3 Hz, 1H), 7.08 (d, *J* = 8.8 Hz, 1H), 5.98 (d, *J=* 8.2 Hz, 1H), 4.20 (s, 2H), 4.02 (s, 3H), 3.86 (s, 3H), 3.76 (d, *J* = 9.6 Hz, 1H), 3.26-3.10 (m, 2H), 2.02 (d, *J =* 13.0 Hz, 2H), 1.36 (q, *J =* 9.7 Hz, 2H).

### Example 57

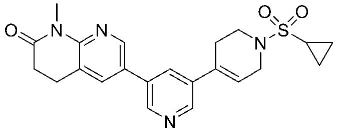

Referring to Example 41, the product 6-(1'-(cyclopropylsulfonyl)-1',2',3',6'-tetrahydro-[3,4'-bipyridine]-5-yl)-1-methyl-3,4-dihydro-[1,8]-diazanaphthalen-2(1H)-one was obtained.

MS m/z (ESI): 425.1[M+1].

### Example 58

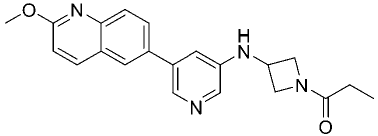

Referring to Example 41, the product 1-(3-((5-(2-methoxyquinolin-6-yl)pyridin-3-yl)amino)azetidin-1-yl)propan-1-one was obtained.

MS m/z (ESI): 363.2[M+1].

### Example 59

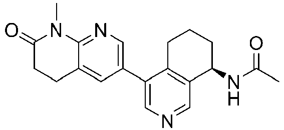

Referring to Example 1, the product (R)-N-(4-(8-methyl-7-carbonyl-5,6,7,8-tetrahydro-1,8-diazanaphthalen-3-yl)-5,6,7,8-tetrahydroisoquinolin-8-yl)acetamide was obtained.

MS m/z (ESI): 351.2[M+1].

### Example 60

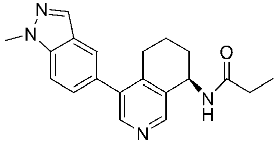

Referring to Example 1, the product (R)-N-(4-(1-methyl-1H-indazol-5-yl)-5,6,7,8-tetrahydroisoquinolin-8-yl)propanamide was obtained.

MS m/z (ESI): 335.2[M+1].

¹H NMR (400 MHz, DMSO) δ 8.44 (d, *J =* 2.1 Hz, 1H), 8.35 - 8.26 (m, 2H), 8.07 (dd, *J =* 8.6, 2.1 Hz, 1H), 8.02 (d, *J =* 8.6 Hz, 1H), 7.94 (d, *J =* 2.7 Hz, 1H), 7.90 (d, *J* = 8.7 Hz, 1H), 7.74 (d, *J =* 8.6 Hz, 1H), 7.42 (s, 1H), 7.09 (d, *J =* 8.9 Hz, 1H), 4.84 (s, 2H), 4.38 (s, 2H), 4.24 (s, 4H), 4.02 (s, 3H), 2.69 (s, 3H).

### Example 61

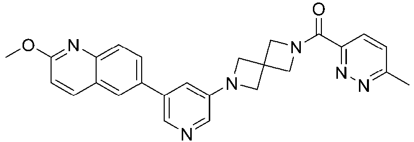

Referring to Example 7, with 6-bromo-2-methoxyquinoline, 3,5-dibromopyridine, 6-methylpyridazine-3-carboxylic acid, tert-butyl 2,6-diazaspiro[3.3]heptane-2-carboxylate as starting materials, the target product (6-(5-(2-methoxyquinolin-6-yl)pyridin-3-yl)-2,6-diazaspiro[3.3]heptan-2-yl)(6-methylpyridazine-3-yl)methanone was finally obtained.

MS m/z (ESI): 453.2 [M+1].

### Example 62

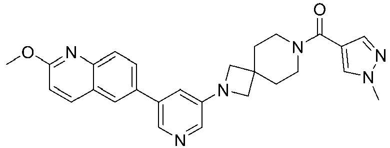

Referring to Example 5, with 3,5-dibromopyridine and tert-butyl 2,7-diazaspiro[3.5]nonane-7-carboxylate as starting materials, the product (2-(5-(2-methoxyquinolin-6-yl)pyridin-3-yl)-2,7-diazaspiro[3.5]nonan-7-yl)(1-methyl-1H-pyrazol-4-yl)methanone was obtained.

MS m/z (ESI): 469.2 [M+1].

¹H NMR (400 MHz, DMSO) δ 8.29 - 8.22 (m, 2H), 8.18 (d, *J =* 2.1 Hz, 1H), 8.01 - 7.93 (m, 2H), 7.84 - 7.75 (m, 2H), 7.58 (s, 1H), 7.09 (t, *J* = 2.3 Hz, 1H), 7.01 (d, *J* = 8.8 Hz, 1H), 3.94 (s, 3H), 3.79 (s, 3H), 3.71 (s, 4H), 3.52 (t, *J =* 5.3 Hz, 4H), 1.75 (t, *J =* 5.2 Hz, 4H).

### Example 63

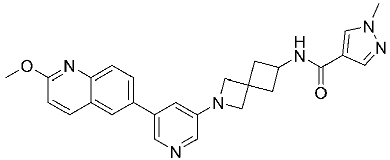

Referring to Example 5, with 3,5-dibromopyridine and tert-butyl (2-azaspiro[3.3]heptan-6-yl)carbamate as starting materials, the product N-(2-(5-(2-methoxyquinolin-6-yl)pyridin-3-yl)-2-azaspiro[3.3]heptan-6-yl)-1-methyl-1H-pyrazole-4-carboxamide was obtained.

MS m/z (ESI): 455.2 [M+1].

### Example 64

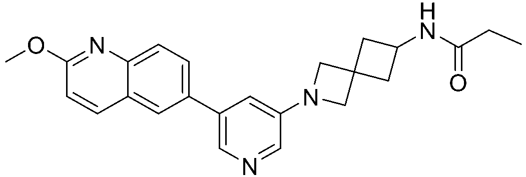

Referring to Example 5, with 3,5-dibromopyridine and tert-butyl (2-azaspiro[3.3]heptan-6-yl)carbamate as starting materials, the product N-(2-(5-(2-methoxyquinolin-6-yl)pyridin-3-yl)-2-azaspiro[3.3]heptan-6-yl)propanamide was obtained.

MS m/z (ESI): 403.2 [M+1].

¹H NMR (400 MHz, DMSO-*d*₆) δ 8.32-8.29 (m, 2H), 8.23 (s, 1H), 8.04-7.99 (m, 2H), 7.86 (d, *J =* 8.8 Hz, 1H), 7.81 (s, 1H), 7.13-7.10 (m, 1H), 7.07 (d, *J =* 8.8 Hz, 1H), 4.16-4.10 (m, 1H), 4.02-3.99 (m, 5H),3.90 (s, 2H), 2.49-2.45 (m, 2H), 2.15-2.10 (m, 2H), 2.04 (q, *J* = 7.6 Hz, 2H), 0.98 (t, *J =* 7.6 Hz, 3H).

### Example 65

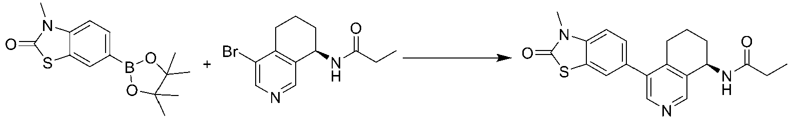

Referring to step 4 of Example 1, with 3-methyl-6-(4,4,5,5-tetramethyl-1,3,2-dioxaborolan-2-yl)benzo[d]thiazol-2(3H)-one and (R)-N-(4-bromo-5,6,7,8-tetrahydroisoquinolin-8-yl)propanamide as starting materials, the title product (R)-N-(4-(3-methyl-2-oxo-2,3-dihydrobenzo[d]thiazol-6-yl)-5,6,7,8-tetrahydroisoquinolin-8-yl)propanamide was obtained.

MS m/z (ESI): 368.1 [M+1].

¹H NMR (400 MHz, MeOD) δ 8.36 (s, 1H), 8.20 (s, 1H), 7.54 (d, 1H), 7.35 (s, 2H), 5.21 (t, 1H), 3.51 (s, 3H), 2.78 - 2.59 (m, 2H), 2.35 - 2.20 (m, 2H), 2.09 - 1.97 (m, 1H), 1.91 - 1.75 (m, 3H), 1.19 (t, 3H).

### Example 66

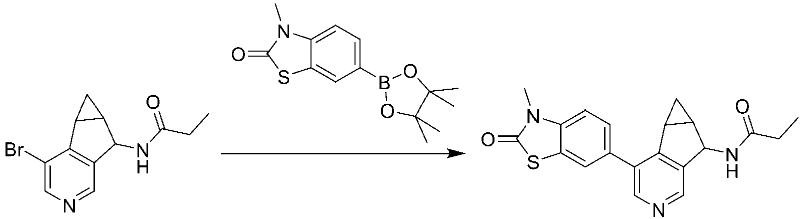

Referring to step 7 of Example 2, with N-((6R)-4-bromo-4b,5,5a,6-tetrahydrocyclopropyl[3,4]cyclopenta[1,2-c]pyridin-6-yl)propanamide and 3-methyl-6-(4,4,5,5-tetramethyl-1,3,2-dioxaborolan-2-yl)benzo[d]thiazol-2(3H)-one as starting materials, the product N-((6R)-4-(3-methyl-2-oxo-2,3-dihydrobenzo[d]thiazol-6-yl)-4b,5,5a,6-tetrahydrocyclopropyl[3,4]cyclopenta[1,2-c]pyridin-6-yl)propanamide was finally obtained. This product was subjected to the first chiral resolution to obtain products P1, P2 and P3. The resolution conditions were as follows.

| Column type | Mobile phase | Flow rate | Detection wavelength | Tempe rature | Retenti on time of P1 | Retenti on time of P2 | Retention time of P3 | Linear gradient |
|---|---|---|---|---|---|---|---|---|
| CHIRALCEL AS-H, 4.6 × 150 mm, 5 µm | Hexane : EtOH = 70 : 30 | 1.0 ml/min | 214 nm | 35°C | 3.7 min | 4.7 min | 6.2 min | Isometric |

P1 was subjected to the second chiral resolution to obtain products P1A and P1B. The resolution conditions were as follows.

| Column type | Mobile phase | Flow rate | Detection wavelength | Tempe rature | Retention time of P1A | Retention time of P1B | Linear gradient |
|---|---|---|---|---|---|---|---|
| CHIRALCEL OJ-H, 4.6 × 150 mm, 5 µm | Hexane : EtOH : MeOH = 70:15:15 | 1.0 ml/min | 214 nm | 35°C | 4.8 min | 5.7 min | Isometric |

MS m/z (ESI): 366.1[M+1].

P2: ¹H NMR (400 MHz, MeOD) δ 8.41 (s, 1H), 8.26 (s, 1H), 7.91 - 7.80 (m, 1H), 7.75 - 7.60 (m, 1H), 7.45 - 7.35 (m, 1H), 7.29 - 7.06 (m, 1H), 5.84 (d, J *=* 6.6 Hz, 1H), 3.52 (s, 3H), 2.63 - 2.49 (m, 1H), 2.38 - 2.29 (m, 2H), 2.29 - 2.20 (m, 1H), 1.21 (t, J = 7.7 Hz, 3H), 0.95 - 0.85 (m, 1H), 0.69 - 0.60 (m, 1H).

### Example 67

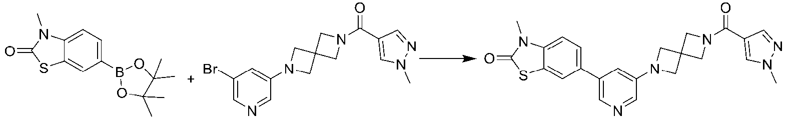

Referring to step 4 of Example 1, with 3-methyl-6-(4,4,5,5-tetramethyl-1,3,2-dioxaborolan-2-yl)benzo[d]thiazol-2(3H)-one and (6-(5-bromopyridin-3-yl)-2,6-diazaspiro[3.3]heptan-2-yl)(1-methyl-1H-pyrazol-4-yl)methanone as starting materials, the title product 3-methyl-6-(5-(6-(1-methyl-1H-pyrazole-4-carbonyl)-2,6-diazaspiro[3.3]heptan-2-yl)pyridin-3-yl)benzo[d]thiazol-2(3H)-one was obtained.

MS m/z (ESI): 447.1 [M+1].

¹H NMR (400 MHz, MeOD) δ 8.17 (d, 1H), 8.06 (s, 1H), 7.88 - 7.80 (m, 2H), 7.78 (d, 1H), 7.73 - 7.59 (m, 1H), 7.35 (d, 1H), 7.16 - 7.07 (m, 1H), 4.67 (s, 2H), 4.33 (s, 2H), 4.19 (s, 4H), 3.93 (s, 3H), 3.50 (s, 3H).

### Example 68

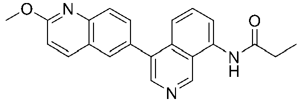

Referring to steps 3 and 4 of Example 5, with 4-bromoisoquinolin-8-amine as a starting material, the product N-(4-(2-methoxyquinolin-6-yl)isoquinolin-8-yl)propanamide was obtained.

MS m/z (ESI): 358.1 [M+1].

### Example 69

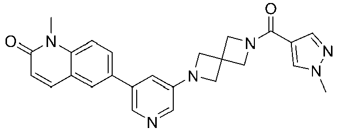

Referring to Example 7,With 6-bromo-1-methylquinolin-2(1H)-one, 3,5-dibromopyridine, tert-butyl 2,6-diazaspiro[3.3]heptane-2-carboxylate and 1-methyl-1H-pyrazole-4-carboxylic acid as starting materials, the target product 1-methyl-6-(5-(6-(1-methyl-1H-pyrazole-4-carbonyl)-2,6-diazaspiro[3.3]heptan-2-yl)pyridin-3-yl)quinolin-2(1H)-one was finally obtained.

MS m/z (ESI): 441.2 [M+1].

### Example 70

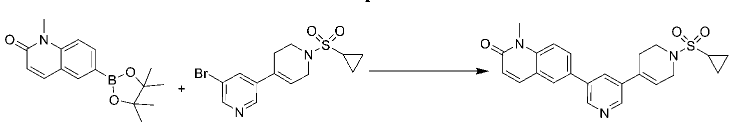

Referring to step 4 of Example 1, with 1-methyl-6-(4,4,5,5-tetramethyl-1,3,2-dioxaborolan-2-yl)quinolin-2(1H)-one and 5-bromo-1'-(cyclopropylsulfonyl)-1',2',3',6'-tetrahydro-3,4'-bipyridine as starting materials, the title product 6-(1'-(cyclopropylsulfonyl)-1',2',3',6'-tetrahydro-[3,4'-bipyridin]-5-yl)-1-methylquinolin-2(1H)-one was obtained.

MS m/z (ESI): 422.1 [M+1].

¹H NMR (400 MHz, MeOD) δ 8.79 (d, 1H), 8.63 (d, 1H), 8.18 (t, 1H), 8.08 (d, 1H), 8.02 (dd, 2H), 7.73 (d, 1H), 6.74 (d, 1H), 6.44 - 6.37 (m, 1H), 4.11 - 4.04 (m, 2H), 3.79 (s, 3H), 3.62 (t, 2H), 2.81 - 2.73 (m, 2H), 2.62 - 2.52 (m, 1H), 1.16 - 1.00 (m, 4H).

### Example 71

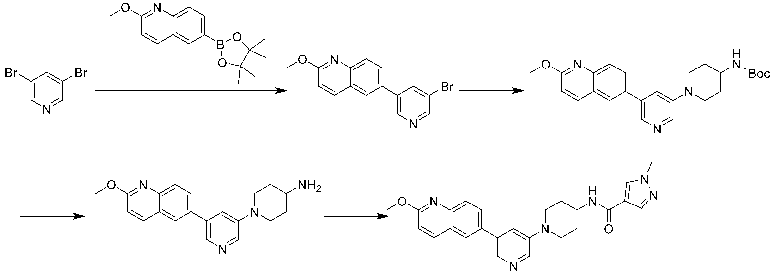

Referring to Example 7, with 2-methoxy-6-(4,4,5,5-tetramethyl-1,3,2-dioxaborolan-2-yl)quinoline, 3,5-dibromopyridine, and tert-butyl piperidin-4-ylcarbamate as starting materials, the target product N-(1-(5-(2-methoxyquinolin-6-yl)pyridin-3-yl)piperidin-4-yl)-1-methyl-1H-pyrazole-4-carboxamide was finally obtained.

MS m/z (ESI): 443.2 [M+1].

¹H NMR (400 MHz, MeOD) δ 8.34 - 8.30 (m, 1H), 8.30 - 8.25 (m, 1H), 8.21 (d, *J* = 8.9 Hz, 1H), 8.12 - 8.08 (m, 1H), 8.04 (s, 1H), 7.98 - 7.91 (m, 2H), 7.90 (s, 1H), 7.77 - 7.69 (m, 1H), 6.99 (d, *J* = 8.9 Hz, 1H), 4.14 - 4.01 (m, 4H), 4.02 - 3.92 (m, 2H), 3.91 (s, 3H), 3.11 - 2.94 (m, 2H), 2.15 - 1.99 (m, 2H), 1.85 - 1.68 (m, 2H).

### Example 72

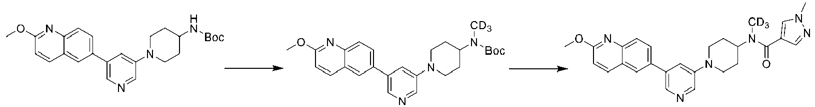

### Step 1

Referring to step 1 of Example 1, with deuterated iodomethane as a starting material, the product tert-butyl (1-(5-(2-methoxyquinolin-6-yl)pyridin-3-yl)piperidin-4-yl)(methyl-d3)carbamate was obtained.

MS m/z (ESI): 452.2 [M+1].

### Step 2

Referring to Example 7, with tert-butyl (1-(5-(2-methoxyquinolin-6-yl)pyridin-3-yl)piperidin-4-yl)(methyl-d3)carbamate as a starting material, the product N-(1-(5-(2-methoxyquinolin-6-yl)pyridin-3-yl)piperidin-4-yl)-1-methyl-N-(methyl-d3)-1H-pyrazole-4-carboxamide was obtained.

MS m/z (ESI): 460.2 [M+1].

### Example 73

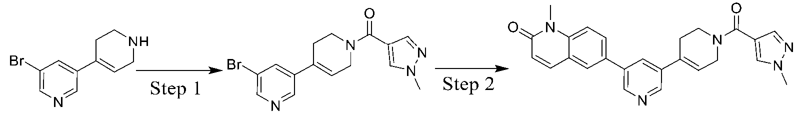

### Step 1

### (5-Bromo-3',6'-dihydro-[3,4'-bipyridin]-1'(2'H)-yl)(1-methyl-1H-pyrazol-4-yl)methanone

Referring to step 3 of Example 41, with 5-bromo-1',2',3',6'-tetrahydro-3,4'-bipyridine and 1-methyl-1H-pyrazole-4-carbonyl chloride as starting materials, the title product (5-bromo-3',6'-dihydro-[3,4'-bipyridin]-1'(2'H)-yl)(1-methyl-1H-pyrazol-4-yl)methanone was obtained.

MS m/z (ESI): 347.0 [M+1].

### Step 2

Referring to step 4 of Example 1, with 1-methyl-6-(4,4,5,5-tetramethyl-1,3,2-dioxaborolan-2-yl)quinolin-2(1H)-one and (5-bromo-3',6'-dihydro-[3,4'-bipyridin]-1'(2'H)-yl)(1-methyl-1H-pyrazol-4-yl)methanone as starting materials, the title product 1-methyl-6-(1'-(1-methyl-1H-pyrazole-4-carbonyl)-1',2',3',6'-tetrahydro-[3,4'-bipyridin]-5-yl)quinolin-2(1H)-one was obtained.

MS m/z (ESI): 426.1 [M+1].

¹H NMR (400 MHz, DMSO) δ 8.41 (d, *J* = 1.7 Hz, 1H), 8.35 (d, *J* = 2.7 Hz, 1H), 8.33 - 8.26 (m, 2H), 8.11 - 8.02 (m, 2H), 7.87 (d, *J* = 8.7 Hz, 1H), 7.71 (s, 1H), 7.67 (t, *J* = 2.4 Hz, 1H), 7.08 (d, *J =* 8.8 Hz, 1H), 4.05 (s, 1H), 4.01 (s, 4H), 3.87 (s, 3H), 2.90 (t, *J* = 12.2 Hz, 2H), 1.92 (d, *J=* 12.8 Hz, 2H), 1.73 (d, *J =* 11.9 Hz, 2H).

### Example 74

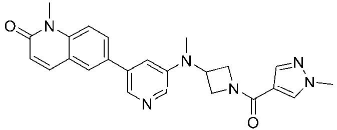

Referring to Example 7, with 6-bromo-1-methylquinolin-2(1H)-one, 3,5-dibromopyridine, 1-methyl-1H-pyrazole-4-carboxylic acid, and tert-butyl 3-aminoazetidine-1-carboxylate as starting materials, the target product 1-methyl-6-(5-(methyl(1-(1-methyl-1H-pyrazole-4-carbonyl)azetidin-3-yl)amino)pyridin-3-yl)quinolin-2(1)-one was finally obtained.

MS m/z (ESI): 429.2 [M+1].

### Example 75

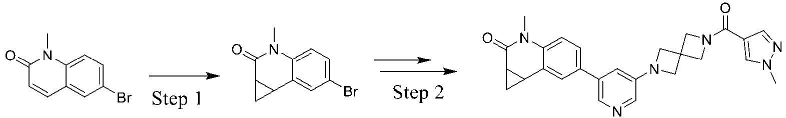

### Step 1

In a 25 mL reaction flask, trimethylsulfoxonium iodide (693.27 mg, 3.15 mmol) was dissolved in DMSO (10 mL). Then sodium hydride (142.80 mg, 3.57 mmol, 60% purity) was added at 0°C, and the reaction solution was stirred at 25°C for 1 hour. Then 6-bromo-1-methylquinolin-2-one (500 mg, 2.10 mmol) was added at 25°C, and the reaction solution was reacted at 90°C for 10 h. After the reaction was stopped, the reaction was quenched by adding water (10 mL), and the mixture was extracted with ethyl acetate (10 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product 6-bromo-3-methyl-1,1a,3,7b-tetrahydro-2H-cyclopropa[c]quinolin-2-one (300 mg, a yellow solid) with a yield of 56.6%.

MS m/z (ESI): 252.0, 254.0 [M+1].

### Step 2

Referring to steps 2 and 4 of Example 1, with 6-bromo-3-methyl-1,1a,3,7b-tetrahydro-2H-cyclopropa[c]quinolin-2-one as a starting material, replacing N-(4-bromo-5,6,7,8-tetrahydroisoquinolin-8-yl)propanamide with (6-(5-bromopyridin-3-yl)-2,6-diazaspiro[3.3]heptan-2-yl)(1-methyl-1H-pyrazol-4-yl)methanone, the title product 3-methyl-6-(5-(6-(1-methyl-1H-pyrazole-4-carbonyl)-2,6-diazaspiro[3.3] heptan-2-yl)pyridin-3-yl)-1,1a,3,7b-tetrahydro-2H-cyclopropa[c]quinolin-2-one was obtained.

MS m/z (ESI): 455.2 [M+1].

### Example 76

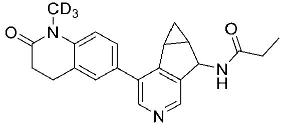

Referring to Example 2, with 1-(methyl-d3)-6-(4,4,5,5-tetramethyl-1,3,2-dioxaborolan-2-yl)-3,4-dihydroquinolin-2(1H)-one as a starting material, the product N-(4-(1-(methyl-d3)-2-carbonyl-1,2,3,4-tetrahydroquinolin-6-yl)-4b,5,5a,6-tetrahydrocyclopropa[3,4]cyclopenta[1,2-c]pyridin-6-yl)propanamide was obtained.

MS m/z (ESI): 365.2 [M+1].

### Example 77

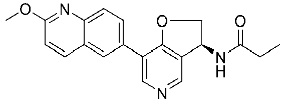

Referring to step 4 of Example 1, with (S)-7-bromo-2,3-dihydrofuro[3,2-c]pyridin-3-amine and 2-methoxy-6-(4,4,5,5-tetramethyl-1,3,2-dioxaborolan-2-yl)quinoline as starting materials, the product (S)-N-(7-(2-methoxyquinolin-6-yl)-2,3-dihydrofuro[3,2-c]pyridin-3-yl)propanamide was obtained.

MS m/z (ESI): 350.1[M+1].

### Example 78

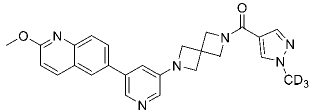

Referring to the synthesis method of Example 42, the product(6-(5-(2-methoxyquinolin-6-yl)pyridin-3-yl)-2,6-diazaspiro[3.3]heptan-2-yl)(1-(methyl-d3)-1H-pyrazol-4-yl)methanone was obtained.

MS m/z (ESI): 444.2 [M+1].

¹H NMR (400 MHz, CDCl₃) δ 8.37 (s, 1H), 8.04 (d, 1H), 7.95 (d, 1H), 7.89 (d, 1H), 7.87 (s, 1H), 7.82 (d, 2H), 7.70 (s, 1H), 7.09 (s, 1H), 6.97 (d, 1H), 4.60 (s, 2H), 4.38 (s, 2H), 4.23 (s, 4H), 4.10 (s, 3H).

### Example 79

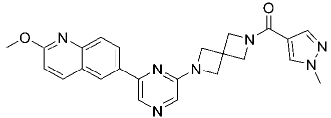

Referring to Example 5, with 3,5-dibromopyrazine as a starting material, the title product was obtained.

The specific method is as follows,

### Step 1: Tert-butyl 6-(6-bromopyrazin-2-yl)-2,6-diazaspiro[3.3]heptane-2-carboxylate

A mixture of 2,6-dibromopyrazine (2.90 g, 12.21 mmol), tert-butyl 2,6-diazaspiro[3.3]heptane-2-carboxylate (2.2 g, 11.10 mmol), potassium carbonate (4.59 g, 33.29 mmol), and N,N-dimethylformamide (30 mL) was stirred at room temperature for 1 hour, and rotary-evaporated to dryness. The residue was separated by column chromatography (PE/EtOAc = 1 : 1) to obtain tert-butyl 6-(6-bromopyrazin-2-yl)-2,6-diazaspiro[3.3]heptane-2-carboxylate as a white solid (2.7 g, yield: 68.5%).

MS m/z (ESI): 355.1 357.1[M+1].

### Step 2: Tert-butyl 6-(6-(2-methoxyquinolin-6-yl)pyrazin-2-yl)-2,6-diazaspiro[3.3]heptane-2-carboxylate

A mixture of 2-methoxy-6-(4,4,5,5-tetramethyl-1,3,2-dioxaborolan-2-yl)quinoline (2.09 g, 7.32 mmol), tert-butyl 6-(6-bromopyrazin-2-yl)-2,6-diazaspiro[3.3]heptane-2-carboxylate (2 g, 5.63 mmol), sodium carbonate (1.79 g, 16.89 mmol), tetrakis(triphenylphosphine)palladium (390 mg, 0.34 mmol), ethanol (15 mL) and water (3 mL) was subjected to nitrogen displacement three times. Then, the mixture was stirred at 85°C for 12 hours under nitrogen protection. After separation by column chromatography (DCM/MeOH = 10:1), tert-butyl 6-(6-(2-methoxyquinolin-6-yl)pyrazin-2-yl)-2,6-diazaspiro[3.3]heptane-2-carboxylate was obtained as a white solid (2 g, yield: 81.9%).

MS m/z (ESI): 434.2 [M+1].

### Step 3: 6-(6-(2,6-Diazaspiro[3.3]heptan-2-yl)pyrazin-2-yl)-2-methoxyquinoline

A mixture of tert-butyl 6-[6-(2-methoxy-6-quinolinyl)pyrazin-2-yl]-2,6-diazaspiro[3.3]heptane-2-carboxylate (2 g, 4.61 mmol), trifluoroacetic acid (5 mL), and DCM (15 mL) was stirred at room temperature for 15 minutes, and rotary-evaporated to dryness. Water was added, and the pH was adjusted to 10 with a saturated NaHCO₃ solution. A solid precipitated out, which was then filtered, and the filter cake was dried to obtain 6-(6-(2,6-diazaspiro[3.3]heptan-2-yl)pyrazin-2-yl)-2-methoxyquinoline (1.5 g, crude product).

MS m/z (ESI): 334.2 [M+1].

### Step 4: (6-(6-(2-Methoxyquinolin-6-yl)pyrazin-2-yl)-2,6-diazaspiro[3.3]heptan-2-yl)(1-methyl-1H-pyrazol-4-yl)methanone

To a solution of 6-(6-(2,6-diazaspiro[3.3]heptan-2-yl)pyrazin-2-yl)-2-methoxyquinoline (1.5 g, 4.50 mmol) and triethylamine (1.36 g, 13.50 mmol) in dichloromethane (15 mL), a solution of 1-methyl-1H-pyrazole-4-carbonyl chloride (845 mg, 5.85 mmol) in dichloromethane (5 mL) was added dropwise. The mixture was stirred at room temperature for 5 minutes. Water was added. The mixture was extracted with dichloromethane (30 mL * 3), and the organic phase was washed with a saturated aqueous sodium chloride solution, dried over anhydrous sodium sulfate, filtered, and rotary-evaporated to dryness. After separation by column chromatography (DCM/MeOH = 10 : 1) followed by preparative chromatography separation, (6-(6-(2-methoxyquinolin-6-yl)pyrazin-2-yl)-2,6-diazaspiro[3.3]heptan-2-yl)(1-methyl-1H-pyrazol-4-yl)methanone (1.02 g, yield: 51.1%) was obtained.

MS m/z (ESI): 442.2 [M+1].

¹H NMR (400 MHz, DMSO) δ 8.60 (d, *J* = 3.9 Hz, 2H), 8.42 - 8.26 (m, 2H), 8.13 (s, 1H), 7.95 - 7.81 (m, 2H), 7.73 (s, 1H), 7.08 (d, *J=* 8.9 Hz, 1H), 4.60 (s, 2H), 4.33 (s, 4H), 4.21 (s, 2H), 4.02 (s, 3H), 3.87 (s, 3H).

### Example 80

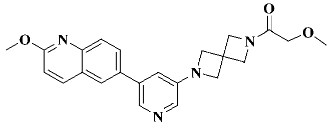

Referring to Example 23, the product 2-methoxy-1-(6-(5-(2-methoxyquinolin-6-yl)pyridin-3-yl)-2,6-diazaspiro[3.3]heptan-2-yl)ethan-1-one was obtained.

MS m/z (ESI): 405.2 [M+1].

¹H NMR (400 MHz, MeOD) δ 8.46 - 8.38 (m, 1H), 8.27 - 8.18 (m, 2H), 8.05 - 7.93 (m, 2H), 7.94 - 7.88 (m, 1H), 7.83 - 7.74 (m, 1H), 7.04 (d, *J =* 8.9 Hz, 1H), 4.54 (s, 2H), 4.39 - 4.30 (m, 4H), 4.27 (s, 2H), 4.08 (s, 3H), 4.00 (s, 2H), 3.39 (s, 3H).

### Example 81

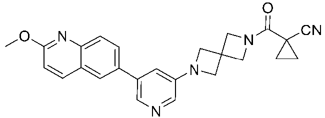

Referring to steps 1 and 2 of Example 5 as well as steps 3 and 4 of Example 6, with 3,5-dibromopyridine and 1-cyanocyclopropane-1-carboxylic acid as starting materials, the product 1-(6-(5-(2-methoxyquinolin-6-yl)pyridin-3-yl)-2,6-diazaspiro[3.3]heptane-2-carbonyl)cyclopropane-1-carbonitrile was obtained.

MS m/z (ESI): 426.1 [M+1].

¹H NMR (400 MHz, DMSO-*d*₆) δ 8.57-8.55 (m, 1H), 8.41-8.38 (m, 1H), 8.32 (d, *J*= 8.8 Hz, 1H), 8.15-8.10 (m, 1H), 8.06-8.02 (m, 1H), 7.95-7.92 (m, 1H), 7.84-7.65 (m, 1H), 7.12 (dd, *J₁* = 8.8 Hz, *J₂ =* 2.0 Hz, 1H), 4.76 (s, 2H), 4.32-4.26 (m, 4H), 4.20 (s, 2H), 4.03 (s, 3H), 1.60-1.55 (m, 2H), 1.53-1.48 (m, 2H).

### Example 82

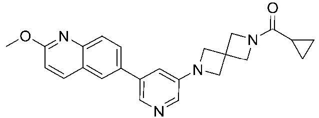

Referring to the synthesis route of Example 5, the title product cyclopropyl (6-(5-(2-methoxyquinolin-6-yl)pyridin-3-yl)-2,6-diazaspiro[3.3]heptan-2-yl)methanone was obtained.

MS m/z (ESI): 401.2 [M+1].

¹H NMR (400 MHz, DMSO) δ 8.36 (d, *J* = 1.9 Hz, 1H), 8.31 (d, *J* = 8.9 Hz, 1H), 8.25 (d, *J* = 2.1 Hz, 1H), 8.02 (dd, *J* = 8.7, 2.2 Hz, 1H), 7.91 - 7.83 (m, 2H), 7.18 (t, *J* = 2.3 Hz, 1H), 7.08 (d, *J* = 8.8 Hz, 1H), 4.46 (s, 2H), 4.19 - 4.10 (m, 4H), 4.07 (s, 2H), 4.01 (s, 3H), 1.53 (tt, *J* = 7.5, 4.9 Hz, 1H), 0.70 (ddd, *J =* 7.7, 6.1, 2.5 Hz, 4H).

### Example 83

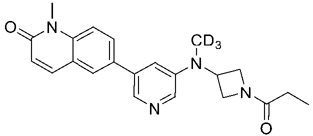

Referring to Example 7, with 6-bromo-1-methylquinolin-2(1H)-one, 3,5-dibromopyridine, tert-butyl 3-aminoazetidine-1-carboxylate, and deuterated iodomethane as starting materials, the target product 1-methyl-6-(5-((methyl-d3)(1-propionylazetidin-3-yl)amino)pyridin-3-yl)quinolin-2(1H)-one was finally obtained.

MS m/z (ESI): 380.2 [M+1].

### Example 84

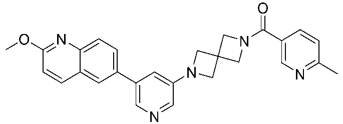

Referring to steps 1 and 2 of Example 5 as well as steps 3 and 4 of Example 6, with 3,5-dibromopyridine and 6-methylnicotinic acid as starting materials, the product (6-(5-(2-methoxyquinolin-6-yl)pyridin-3-yl)-2,6-diazaspiro[3.3]heptan-2-yl)(6-methylpyridin-3-yl)methanone was obtained.

MS m/z (ESI): 452.1 [M+1].

¹H NMR (400 MHz, DMSO-*d*₆) δ 8.83 (s, 1H), 8.58 (s, 1H), 8.41 (s, 1H), 8.20 (d, *J =* 8.8 Hz, 1H), 8.15-8.05 (m, 3H), 7.93 (d, *J =* 8.8 Hz, 1H), 7.86 (s, 1H), 7.60-7.50 (m, 1H), 7.13 (d, *J =* 8.4 Hz, 1H), 4.62 (s, 2H), 4.32-4.24 (m, 6H), 4.03 (s, 3H), 2.60 (s, 3H).

### Example 85

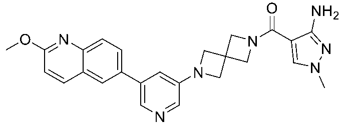

Referring to steps 1 and 2 of Example 5 as well as steps 3 and 4 of Example 6, with 3,5-dibromopyridine and 3-amino-1-methyl-1H-pyrazole-4-carboxylic acid as starting materials, the product (3-amino-1-methyl-1H-pyrazol-4-yl)(6-(5-(2-methoxyquinolin-6-yl)pyridin-3-yl)-2,6-diazaspiro[3.3]heptan-2-yl)methanone was obtained.

MS m/z (ESI): 456.1 [M+1].

### Example 86

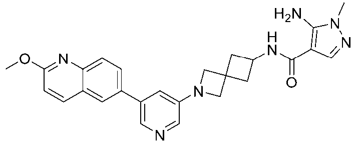

Referring to steps 1 and 2 of Example 5 as well as steps 3 and 4 of Example 6, with 3,5-dibromopyridine and tert-butyl (2-azaspiro[3.3]heptan-6-yl)carbamate as starting materials, the product 5-amino-N-(2-(5-(2-methoxyquinolin-6-yl)pyridin-3-yl)-2-azaspiro[3.3]heptan-6-yl)-1-methyl-1H-pyrazole-4-carboxamide was obtained.

MS m/z (ESI): 470.2 [M+1].

### Example 87

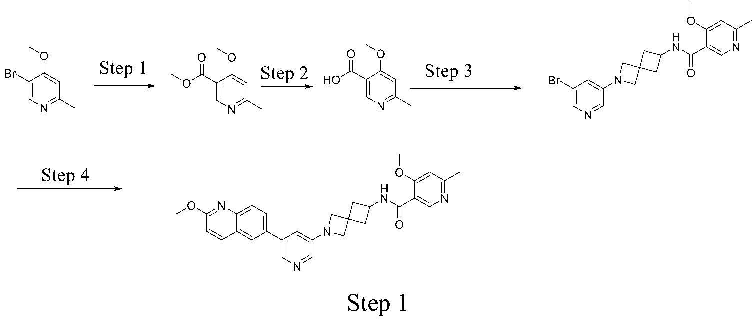

At room temperature, 5-bromo-4-methoxy-2-methylpyridine (1.0 g, 5 mmol), triethylamine (5.06 g, 50 mmol), and [1,1'-bis(diphenylphosphino)ferrocene]dichloropalladium dichloromethane complex (0.41 g, 0.5 mmol) were dissolved in N,N-dimethylformamide (10 mL) and methanol (10 mL). The atmosphere was displaced with carbon monoxide gas, then the mixture heated to 100°C and reacted for 24 hours. The mixture was cooled to room temperature, and rotary-evaporated to remove methanol and triethylamine. The residue was diluted with ethyl acetate, then washed with a saturated aqueous sodium chloride solution. The organic phase was dried with anhydrous sodium sulfate, filtered, and dried. The residue was separated by Flash column chromatography (eluted with PE:EA = 10: 1-3: 1) to obtain the product methyl 4-methoxy-6-methylnicotinate (0.36 g, yield: 40%).

MS m/z (ESI): 182.1 [M+1].

### Step 2

At room temperature, methyl 4-methoxy-6-methylnicotinate (0.36 g, 2 mmol) was dissolved in tetrahydrofuran (5 mL), then an aqueous sodium hydroxide solution (5 M, 2 mL) was added. The mixture was stirred at room temperature overnight. LCMS indicated the completion of the reaction. The reaction solution was neutralized with dilute hydrochloric acid (2 M) and the pH value was adjusted to 5-6. Then the mixture was extracted with ethyl acetate (15 mL × 3), the organic phases were combined, dried over anhydrous sodium sulfate, filtered, and rotary-evaporated to dryness to obtain the target product 4-methoxy-6-methylnicotinic acid (0.27 g, yield: 80%).

MS m/z (ESI): 168.1 [M+1].

### Step 3

Referring to step 3 of Example 6, with 4-methoxy-6-methylnicotinic acid and 2-(5-bromopyridin-3-yl)-2-azaspiro[3.3]heptan-6-amine as starting materials, the product N-(2-(5-bromopyridin-3-yl)-2-azaspiro[3.3]heptan-6-yl)-4-methoxy-6-methylnicotinamide was obtained.

MS m/z (ESI): 417.1 [M+1].

### Step 4

Referring to step 4 of Example 6, with N-(2-(5-bromopyridin-3-yl)-2-azaspiro[3.3]heptan-6-yl)-4-methoxy-6-methylnicotinamide and 2-methoxy-6-(4,4,5,5-tetramethyl-1,3,2-dioxaborolan-2-yl)quinoline as starting materials, the product 4-methoxy-N-(2-(2-methoxyquinolin-6-yl)pyridin-3-yl)-2-azaspiro[3.3] heptan-6-yl)-6-methylnicotinamide was obtained.

MS m/z (ESI): 496.2 [M+1].

### Example 88

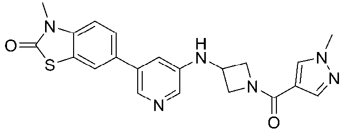

Referring to the synthesis route of Example 5, the title product 3-methyl-6-(5-((1-(1-methyl-1H-pyrazole-4-carbonyl)azetidin-3-yl)amino)pyridin-3-yl)benzo[d]thiazol-2(3H)-one was obtained.

MS m/z (ESI): 421.1 [M+1].

### Example 89

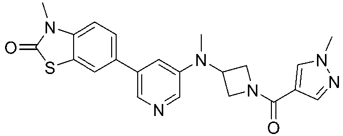

Referring to the synthesis route of Example 5, the title product 3-methyl-6-(5-(methyl(1-(1-methyl-1H-pyrazole-4-carbonyl)azetidin-3-yl)amino)pyridin-3-yl)benzo[d]thiazol-2(3H)-one was obtained.

MS m/z (ESI): 435.1 [M+1].

¹H NMR (400 MHz, DMSO) δ 8.38 (d, J = 1.7 Hz, 1H), 8.20 (d, J = 2.7 Hz, 1H), 8.17 (s, 1H), 8.14 (d, J = 2.1 Hz, 1H), 7.82 (dd, J = 8.4, 1.9 Hz, 1H), 7.77 (s, 1H), 7.61 (s, 1H), 7.44 (d, J = 8.5 Hz, 1H), 4.89 (t, J = 6.6 Hz, 1H), 4.67-4.64 (m, 1H), 4.44 (s, 1H), 4.36-4.32 (m, 1H), 4.07 (s, 1H), 3.86 (s, 3H), 3.46 (s, 3H), 3.07 (s, 3H).

### Example 90

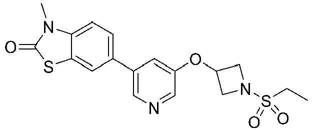

Referring to the synthesis route of Example 4, with 3-methyl-6-(4,4,5,5-tetramethyl-1,3,2-dioxaborolan-2-yl)-1,3-benzothiazol-2-one as a starting material, the title product 6-(5-((1-(ethylsulfonyl)azetidin-3-yl)oxy)pyridin-3-yl)-3-methylbenzo[d]thiazol-2(3H)-one was obtained.

MS m/z (ESI): 406.1 [M+1].

¹H NMR (400 MHz, DMSO) δ 8.59 (s, 1H), 8.25 (d, J = 2.7 Hz, 1H), 8.13 (s, 1H), 7.82 (d, J = 8.4 Hz, 1H), 7.57 (s, 1H), 7.43 (d, J = 8.4 Hz, 1H), 5.27 (t, J = 5.6 Hz, 1H), 4.39 (t, J = 7.9 Hz, 2H), 3.97 (dd, J = 9.4, 4.6 Hz, 2H), 3.46 (s, 3H), 3.20 (q, J = 7.4 Hz, 2H), 1.26 (d, J = 7.4 Hz, 3H).

### Example 91

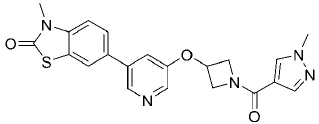

Referring to the synthesis route of Example 4, the title product 3-methyl-6-(5-((1-(1-methyl-1H-pyrazole-4-carbonyl)azetidin-3-yl)oxy)pyridin-3-yl)benzo[d]thiazol-2(3H)-one was obtained.

The specific method is as follows,

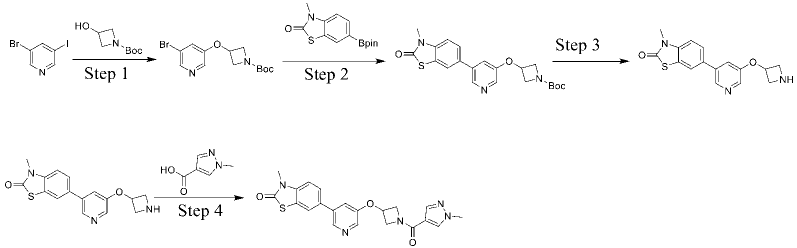

### Step 1: Tert-butyl 3-((5-bromopyridin-3-yl)oxy)azetidine-1-carboxylate

A mixture of 3-bromo-5-iodo-pyridine (2 g, 7.04 mmol), tert-butyl 3-hydroxyazetidine-1-carboxylate (1.46 g, 8.45 mmol), cuprous iodide (66.93 mg, 352 µmol), 1,10-phenanthroline (126.96 mg, 704 µmol), cesium carbonate (6.89 g, 21.13 mmol), and toluene (20 mL) was subjected to nitrogen displacement three times. Then the mixture was stirred at 110°C for 12 hours under nitrogen protection, and rotary-evaporated to dryness. The residue was separated by column chromatography (PE/EtOAC = 3:1) to obtain tert-butyl 3-((5-bromopyridin-3-yl)oxy)azetidine-1-carboxylate (1.2 g, yield: 51.7%).
MS m/z (ESI): 329.0 331.0[M+H]

### Step 2: Tert-butyl 3-((5-(3-methyl-2-carbonyl-2,3-dihydrobenzo[d]thiazol-6-yl)pyridin-3-yl)oxy)azetidine-1-carboxylate

A mixture of 3-methyl-6-(4,4,5,5-tetramethyl-1,3,2-dioxaborolan-2-yl)benzo[d]thiazol-2(3H)-one (126 mg, 434 µmol), tert-butyl 3-((5-bromopyridin-3-yl)oxy)azetidine-1-carboxylate (110 mg, 334 µmol), sodium carbonate (106 mg, 1.00 mmol), tetrakis(triphenylphosphine)palladium (19 mg, 16 µmol), ethanol (5 mL), and water (1 mL) was subjected to nitrogen displacement three times. Then the mixture was stirred at 90°C for 2 hours under nitrogen protection, and rotary-evaporated to dryness. The residue was separated by column chromatography to obtain tert-butyl 3-((5-(3-methyl-2-carbonyl-2,3-dihydrobenzo[d]thiazol-6-yl)pyridin-3-yl)oxy)azetidine-1-carboxylate as a pale yellow solid (130 mg, yield: 94.1%).
MS m/z (ESI): 414.1 [M+H]

### Step 3: 6-(5-(Azetidin-3-oxy)pyridin-3-yl)-3-methylbenzo[d]thiazol-2(3H)-one

A mixture of tert-butyl 3-((5-(3-methyl-2-carbonyl-2,3-dihydrobenzo[d]thiazol-6-yl)pyridin-3-yl)oxy)azetidine-1-carboxylate (130 mg, 314 µmol), trifluoroacetic acid (1 mL), and DCM (3 mL) was stirred at room temperature for 0.5 hours, and rotary-evaporated to dryness. Water was added, and the pH was adjusted to 10 with a saturated NaHCO3 solution. The mixture was extracted with DCM, the combined organic phases were washed with a saturated aqueous sodium chloride solution, dried over anhydrous sodium sulfate, and filtered. The filtrate was concentrated to dryness under reduced pressure to obtain 6-(5-(azetidin-3-oxy)pyridin-3-yl)-3-methylbenzo[d]thiazol-2(3H)-one as a pale yellow oil (100 mg, crude product).
MS m/z (ESI): 314.1 [M+H]

### Step 4: 3-Methyl-6-(5-((1-(1-methyl-1H-pyrazole-4-carbonyl)azetidin-3-yl)oxy)pyridin-3-yl)benzo[d]thiazol-2(3H)-one

To a solution of 6-(5-(azetidin-3-oxy)pyridin-3-yl)-3-methylbenzo[d]thiazol-2(3H)-one (50 mg, 159 µmol), (7-azabenzotriazole)-N,N,N',N'-tetramethyluronium hexafluorophosphate (120 mg, 319 µmol), and triethylamine (48.44 mg, 478 µmol) in DMF (3 mL) was added 1 1-methyl-1H-pyrazole-4-carboxylic acid (30 mg, 239 µmol). The mixture was stirred at room temperature for 2 hours. Water was added, and the mixture was extracted with dichloromethane (30 mL * 3). The organic phase was washed with a saturated aqueous sodium chloride solution, dried over anhydrous sodium sulfate, filtered, and rotary-evaporated to dryness. The residue was separated by preparative chromatography under an acidic condition to obtain 3-methyl-6-(5-((1-(1-methyl-1H-pyrazole-4-carbonyl)azetidin-3-yl)oxy)pyridin-3-yl)benzo[d]thiazol-2(3H)-one (32.7 mg, yield: 48.1%).

MS m/z (ESI): 422.1 [M+1].

¹H NMR (400 MHz, DMSO) δ 8.59 (d, J = 1.8 Hz, 1H), 8.25 (d, J = 2.6 Hz, 1H), 8.18 (s, 1H), 8.12 (d, J = 1.9 Hz, 1H), 7.81 (dd, J = 8.4, 2.0 Hz, 1H), 7.77 (s, 1H), 7.56 (t, J = 2.3 Hz, 1H), 7.43 (d, J = 8.4 Hz, 1H), 5.33 (tt, J = 6.7, 3.7 Hz, 1H), 4.87 -4.81(m, 1H), 4.54-4.51 (m, 1H), 4.39-4.37 (m, 1H), 4.10 - 3.93 (m, 1H), 3.86 (s, 3H), 3.45 (s, 3H).

### Example 92

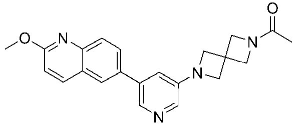

Referring to the synthesis method of Example 42, with 2-(5-bromopyridin-3-yl)-2,6-diazaspiro[3.3]heptane and acetic acid as starting materials, the title product 1-(6-(5-(2-methoxyquinolin-6-yl)pyridin-3-yl)-2,6-diazaspiro[3.3]heptan-2-yl)ethan-1-one was obtained.

MS m/z (ESI): 375.1 [M+1].

¹H NMR (400 MHz, DMSO) δ 8.36 (d, J = 1.9 Hz, 1H), 8.31 (d, J = 8.9 Hz, 1H), 8.24 (d, J = 2.1 Hz, 1H), 8.01 (dd, J = 8.7, 2.2 Hz, 1H), 7.90 - 7.83 (m, 2H), 7.18 (t, J = 2.3 Hz, 1H), 7.07 (d, J = 8.8 Hz, 1H), 4.32 (s, 2H), 4.11 (s, 4H), 4.04 (s, 2H), 4.01 (s, 3H), 1.76 (s, 3H).

### Example 93

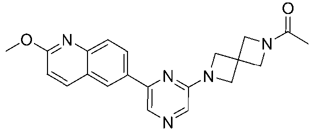

Referring to Example 5, with 3,5-dibromopyrazine as a starting material, and replacing 1-methyl-1H-pyrazole-4-carbonyl chloride and 1-methyl-6-(4,4,5,5-tetramethyl-1,3,2-dioxaborolan-2-yl)-1,4-dihydro-2H-benzo[d][1,3]oxazin-2-one with acetyl chloride and 2-methoxy-6-(4,4,5,5-tetramethyl-1,3,2-dioxaborolan-2-yl)quinoline, the title product 1-(6-(6-(2-methoxyquinolin-6-yl)pyrazin-2-yl)-2,6-diazaspiro[3.3]heptan-2-yl)ethan-1-one was obtained.

MS m/z (ESI): 376.1 [M+1].

¹H NMR (400 MHz, DMSO) δ 8.52 (d, J = 3.0 Hz, 2H), 8.37 - 8.16 (m, 2H), 7.81 (t, J = 4.4 Hz, 2H), 7.01 (d, J = 8.9 Hz, 1H), 4.27 (s, 2H), 4.22 (s, 4H), 3.99 (s, 2H), 3.95 (s, 3H), 1.70 (s, 3H).

### Example 94

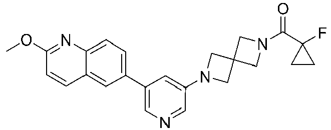

Referring to the synthesis method of Example 42, with 2-(5-bromopyridin-3-yl)-2,6-diazaspiro[3.3]heptane and 1-fluorocyclopropane-1-carboxylic acid as starting materials, the title product (1-fluorocyclopropyl)(6-(5-(2-methoxyquinolin-6-yl)pyridin-3-yl)-2,6-diazaspiro[3.3]heptan-2-yl)methanone was obtained.

MS m/z (ESI): 419.1 [M+1].

¹H NMR (400 MHz, DMSO-d6) δ 8.46 (s, 1H), 8.33-8.30 (m, 2H), 8.07 (d, J = 8.8 Hz, 1H), 7.95 (s, 1H), 7.90 (d, J = 8.8 Hz, 1H), 7.48 (s, 1H), 7.11 (d, J= 8.8 Hz, 1H), 4.61 (s, 2H), 4.22 (s, 4H), 4.19 (s, 2H), 4.02 (s, 3H), 1.30-1.26 (m, 2H), 1.21-1.18 (m, 2H).

### Example 95

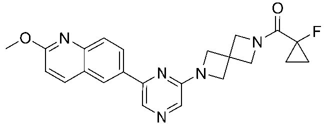

Referring to Example 5, with 3,5-dibromopyrazine as a starting material, and replacing 1-methyl-1H-pyrazole-4-carbonyl chloride and 1-methyl-6-(4,4,5,5-tetramethyl-1,3,2-dioxaborolan-2-yl)-1,4-dihydro-2H-benzo[d][1,3]oxazin-2-one with 1-fluorocyclopropane-1-carbonyl chloride and 2-methoxy-6-(4,4,5,5-tetramethyl-1,3,2-dioxaborolan-2-yl)quinoline, the title product (1-fluorocyclopropyl)(6-(6-(2-methoxyquinolin-6-yl)pyrazin-2-yl)-2,6-diazaspiro[3.3]heptan-2-yl)methanone was obtained.

MS m/z (ESI): 420.1 [M+1].

### Example 96

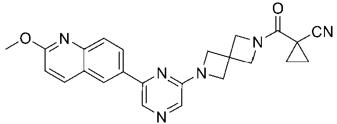

Referring to Example 5, with 3,5-dibromopyrazine as a starting material, and replacing 1-methyl-1H-pyrazole-4-carbonyl chloride and 1-methyl-6-(4,4,5,5-tetramethyl-1,3,2-dioxaborolan-2-yl)-1,4-dihydro-2H-benzo[d][1,3]oxazin-2-one with 1-cyanocyclopropane-1-carbonyl chloride and 2-methoxy-6-(4,4,5,5-tetramethyl-1,3,2-dioxaborolan-2-yl)quinoline, the title product 1-(6-(6-(2-methoxyquinolin-6-yl)pyrazin-2-yl)-2,6-diazaspiro[3.3]heptane-2-carbonyl)cyclopropane-1-carbonitrile was obtained.

MS m/z (ESI): 427.1 [M+1].

### Example 97

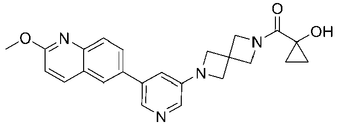

Referring to the synthesis method of Example 42, with 2-(5-bromopyridin-3-yl)-2,6-diazaspiro[3.3]heptane and 1-hydroxycyclopropane-1-carboxylic acid as starting materials, the title product (1-hydroxycyclopropyl)(6-(5-(2-methoxyquinolin-6-yl)pyridin-3-yl)-2,6-diazaspiro[3.3]heptan-2-yl)methanone was obtained.

MS m/z (ESI): 417.1 [M+1].

¹H NMR (400 MHz, DMSO) δ 8.36 (d, J = 1.9 Hz, 1H), 8.31 (d, J = 8.9 Hz, 1H), 8.25 (d, J = 2.1 Hz, 1H), 8.02 (dd, J = 8.7, 2.2 Hz, 1H), 7.90 - 7.83 (m, 2H), 7.18 (t, J = 2.3 Hz, 1H), 7.08 (d, J = 8.9 Hz, 1H), 6.04 (s, 1H), 4.64 (s, 2H), 4.13-4.10 (m, 6H), 4.01 (s, 3H), 1.03 (q, J = 4.1 Hz, 2H), 0.79 (q, J = 4.1 Hz, 2H).

### Example 98

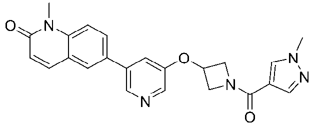

Referring to steps 3 and 4 of Example 4, with 3-(azetidin-3-oxy)-5-bromopyridine and 1-methyl-1H-pyrazole-4-carbonyl chloride as starting materials, replacing 1-methyl-6-(4,4,5,5-tetramethyl-1,3,2-dioxaborolan-2-yl)-1,4-dihydro-2H-benzo[d][1,3]oxazin-2-one with 1-methyl-6-(4,4,5,5-tetramethyl-1,3,2-dioxaborolan-2-yl)quinolin-2(1H)-one, the title product 1-methyl-6-(5-((1-(1-methyl-1H-pyrazole-4-carbonyl)azetidin-3-yl)oxy)pyridin-3-yl)quinolin-2(1H)-one was obtained.

MS m/z (ESI): 416.1 [M+1].

¹H NMR (400 MHz, DMSO) δ 8.66 (d, J = 1.8 Hz, 1H), 8.27 (d, J = 2.7 Hz, 1H), 8.19 (d, J = 2.2 Hz, 1H), 8.17 (s, 1H), 8.06 (dd, J = 8.8, 2.2 Hz, 1H), 7.97 (d, J = 9.5 Hz, 1H), 7.77 (s, 1H), 7.70 - 7.60 (m, 2H), 6.69 (d, J = 9.5 Hz, 1H), 5.35 (tt, J = 6.9, 3.9 Hz, 1H), 4.85 (s, 1H), 4.53 (s, 1H), 4.39 (s, 1H), 4.02 (s, 1H), 3.85 (s, 3H), 3.67 (s, 3H).

### Example 99

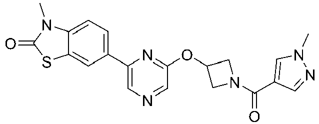

Referring to Example 4, with 2-bromo-6-fluoropyrazine as a starting material, replacing ethylsulfonyl chloride and 1-methyl-6-(4,4,5,5-tetramethyl-1,3,2-dioxaborolan-2-yl)-1,4-dihydro-2H-benzo[d][1,3]oxazin-2-one with 1-methyl-1H-pyrazole-4-carbonyl chloride and 3-methyl-6-(4,4,5,5-tetramethyl-1,3,2-dioxaborolan-2-yl)benzo[d]thiazol-2(3H)-one, the title product 3-methyl-6-(6-((1-(1-methyl-1H-pyrazole-4-carbonyl)azetidin-3-yl)oxy)pyrazin-2-yl)benzo[d]thiazol-2(3H)-one was obtained.

MS m/z (ESI): 423.1 [M+1].

¹H NMR (400 MHz, DMSO) δ 8.93 (s, 1H), 8.50 (d, *J =* 1.8 Hz, 1H), 8.34 (s, 1H), 8.20 (dd, *J =* 8.5, 1.9 Hz, 1H), 8.17 (s, 1H), 7.77 (s, 1H), 7.46 (d, *J =* 8.5 Hz, 1H), 5.66-5.61 (m, 1H), 4.87 (s, 1H), 4.56 (s, 1H), 4.45 (d, *J* = 9.7 Hz, 1H), 4.06 (d, *J= 11.2* Hz, 1H), 3.85 (s, 3H), 3.47 (s, 3H).

### Example 100

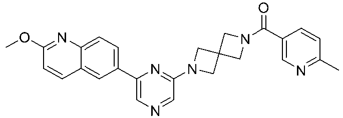

Referring to Example 5, with 3,5-dibromopyrazine as a starting material, replacing 1-methyl-1H-pyrazole-4-carbonyl chloride and 1-methyl-6-(4,4,5,5-tetramethyl-1,3,2-dioxaborolan-2-yl)-1,4-dihydro-2H-benzo[d][1,3]oxazin-2-one with 6-methylnicotinoyl chloride and 2-methoxy-6-(4,4,5,5-tetramethyl-1,3,2-dioxaborolan-2-yl)quinoline, the title product (6-(6-(2-methoxyquinolin-6-yl)pyrazin-2-yl)-2,6-diazaspiro[3.3]heptan-2-yl)(6-methylpyridin-3-yl)methanone was obtained.

MS m/z (ESI): 453.2 [M+1].

¹H NMR (400 MHz, DMSO) δ 8.72 (d, J = 2.3 Hz, 1H), 8.59 (d, J = 3.0 Hz, 2H), 8.36 (dd, J = 8.7, 2.1 Hz, 1H), 8.33 (d, J = 9.1 Hz, 1H), 7.95 (dd, J = 8.1, 2.3 Hz, 1H), 7.88 (d, J = 8.4 Hz, 2H), 7.38 (d, J = 8.1 Hz, 1H), 7.08 (d, J = 8.9 Hz, 1H), 4.60 (s, 2H), 4.42 - 4.19 (m, 6H), 4.02 (s, 3H), 2.53 (s, 3H).

### Example 101

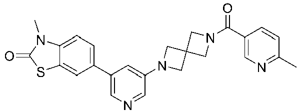

Referring to the synthesis method of Example 42, with 2-(5-bromopyridin-3-yl)-2,6-diazaspiro[3.3]heptane and 6-methylnicotinic acid as starting materials, replacing 2-ethoxy-6-(4,4,5,5-tetramethyl-1,3,2-dioxaborolan-2-yl)quinoline with 3-methyl-6-(4,4,5,5-tetramethyl-1,3,2-dioxaborolan-2-yl)benzo[d]thiazol-2(3H)-one, the title product 3-methyl-6-(5-(6-(6-methylnicotinoyl)-2,6-diazaspiro[3.3]heptan-2-yl)pyridin-3-yl)benzo[d]thiazol-2(3H)-one was obtained.

MS m/z (ESI): 458.1 [M+1].

¹H NMR (400 MHz, DMSO-d6) δ 8.70 (d, J = 2.0 Hz, 1H), 8.25 (d, J = 2.0 Hz, 1H), 8.05 (d, J = 2.0 Hz, 1H), 7.93 (dd, J1 = 8.0 Hz, J2 = 2.0 Hz, 1H), 7.82 (d, J = 2.0 Hz, 1H), 7.74 (dd, J1 = 8.0 Hz, J2 = 2.0 Hz, 1H), 7.40 (d, J = 8.0 Hz, 1H), 7.35 (d, J = 8.0 Hz, 1H), 7.08 (t, J = 2.0 Hz, 1H), 4.57 (s, 2H), 4.28 (s, 2H), 4.13-4.10 (m, 4H), 3.44 (s, 3H), 2.52 (s, 3H).

### Example 102

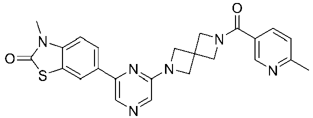

Referring to Example 5, with 3,5-dibromopyrazine as a starting material, replacing 1-methyl-1H-pyrazole-4-carbonyl chloride and 1-methyl-6-(4,4,5,5-tetramethyl-1,3,2-dioxaborolan-2-yl)-1,4-dihydro-2H-benzo[d][1,3]oxazin-2-one with 6-methylnicotinoyl chloride and 3-methyl-6-(4,4,5,5-tetramethyl-1,3,2-dioxaborolan-2-yl)benzo[d]thiazol-2(3H)-one, the title product 3-methyl-6-(6-(6-(6-methylnicotinoyl)-2,6-diazaspiro[3.3]heptan-2-yl)pyrazin-2-yl)benzo[d]thiazol-2(3H)-one was obtained.

MS m/z (ESI): 459.1 [M+1].

### Example 103

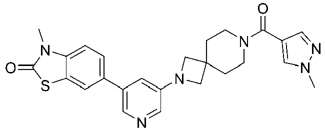

Referring to Example 5, with 3,5-dibromopyridine and tert-butyl 2,7-diazaspiro[3.5]nonane-7-carboxylate as starting materials, replacing 1-methyl-6-(4,4,5,5-tetramethyl-1,3,2-dioxaborolan-2-yl)-1,4-dihydro-2H-benzo[d][1,3]oxazin-2-one with 3-methyl-6-(4,4,5,5-tetramethyl-1,3,2-dioxaborolan-2-yl)benzo[d]thiazol-2(3H)-one, the title product 3-methyl-6-(5-(7-(1-methyl-1H-pyrazole-4-carbonyl)-2,7-diazaspiro[3.5]nonan-2-yl)pyridin-3-yl)benzo[d]thiazol-2(3H)-one was obtained.

MS m/z (ESI): 475.1 [M+1].

¹H NMR (400 MHz, DMSO) δ 8.23 (d, J = 1.9 Hz, 1H), 8.06 (d, J = 1.9 Hz, 1H), 8.04 (s, 1H), 7.81 (d, J = 2.6 Hz, 1H), 7.74 (dd, J = 8.4, 1.9 Hz, 1H), 7.65 (s, 1H), 7.40 (d, J = 8.5 Hz, 1H), 7.06 (t, J = 2.3 Hz, 1H), 3.86 (s, 3H), 3.75 (s, 4H), 3.58 (t, J = 5.5 Hz, 4H), 3.45 (s, 3H), 1.81 (t, J = 5.5 Hz, 4H).

### Example 104

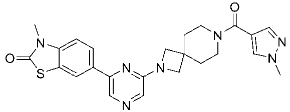

Referring to Example 5, with 3,5-dibromopyrazine and tert-butyl 2,7-diazaspiro[3.5]nonane-7-carboxylate as starting materials, replacing 1-methyl-6-(4,4,5,5-tetramethyl-1,3,2-dioxaborolan-2-yl)-1,4-dihydro-2H-benzo[d][1,3]oxazin-2-one with 3-methyl-6-(4,4,5,5-tetramethyl-1,3,2-dioxaborolan-2-yl)benzo[d]thiazol-2(3H)-one, the title product 3-methyl-6-(6-(7-(1-methyl-1H-pyrazole-4-carbonyl)-2,7-diazaspiro[3.5]nonan-2-yl)pyrazin-2-yl)benzo[d]thiazol-2(3H)-one was obtained.

MS m/z (ESI): 476.1 [M+1].

### Example 105

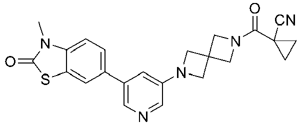

Referring to the synthesis method of Example 42, with 2-(5-bromopyridin-3-yl)-2,6-diazaspiro[3.3]heptane and 1-cyanocyclopropane-1-carboxylic acid as starting materials, replacing 2-ethoxy-6-(4,4,5,5-tetramethyl-1,3,2-dioxaborolan-2-yl)quinoline with 3-methyl-6-(4,4,5,5-tetramethyl-1,3,2-dioxaborolan-2-yl)benzo[d]thiazol-2(3H)-one, the title product 1-(6-(5-(3-methyl-2-carbonyl-2,3-dihydrobenzo[d]thiazol-6-yl)pyridin-3-yl)-2,6-diazaspiro[3.3]heptane-2-carbonyl)cyclopropane-1-carbonitrile was obtained.

MS m/z (ESI): 432.1 [M+1].

¹H NMR (400 MHz, DMSO) δ 8.25 (d, J = 1.9 Hz, 1H), 8.05 (d, J = 1.9 Hz, 1H), 7.82 (d, J = 2.6 Hz, 1H), 7.73 (dd, J = 8.4, 1.9 Hz, 1H), 7.41 (d, J = 8.4 Hz, 1H), 7.07 (t, J = 2.3 Hz, 1H), 4.72 (s, 2H), 4.30 - 4.03 (m, 6H), 3.45 (s, 3H), 1.60 - 1.54 (m, 2H), 1.53 - 1.46 (m, 2H).

### Example 106

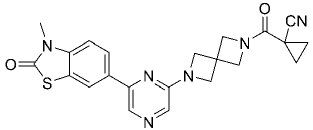

Referring to Example 5, with 3,5-dibromopyrazine as a starting material, replacing 1-methyl-1H-pyrazole-4-carbonyl chloride and 1-methyl-6-(4,4,5,5-tetramethyl-1,3,2-dioxaborolan-2-yl)-1,4-dihydro-2H-benzo[d][1,3]oxazin-2-one with 1-cyanocyclopropane-1-carbonyl chloride and 3-methyl-6-(4,4,5,5-tetramethyl-1,3,2-dioxaborolan-2-yl)benzo[d]thiazol-2(3H)-one, the title product 1-(6-(6-(3-methyl-2-carbonyl-2,3-dihydrobenzo[d]thiazol-6-yl)pyrazin-2-yl)-2,6-diazaspiro[3.3]heptane-2-carbonyl)cyclopropane-1-carbonitrile was obtained.

MS m/z (ESI): 433.1 [M+1].

¹H NMR (400 MHz, DMSO-d6) δ 8.48 (s, 1H), 8.36 (s, 1H), 8.10 (d, J = 8.4 Hz, 1H), 7.84 (s, 1H), 7.43 (d, J = 8.4 Hz, 1H), 4.73 (s, 2H), 4.33-4.29 (m, 4H), 4.18 (s, 2H), 3.45 (s, 3H), 1.60-1.55 (m, 2H), 1.52-1.46 (m, 2H).

### Example 107

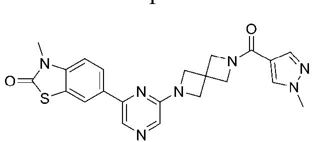

Referring to Example 5, with 3,5-dibromopyrazine as a starting material, replacing 1-methyl-6-(4,4,5,5-tetramethyl-1,3,2-dioxaborolan-2-yl)-1,4-dihydro-2H-benzo[d][1,3]oxazin-2-one with 3-methyl-6-(4,4,5,5-tetramethyl-1,3,2-dioxaborolan-2-yl)benzo[d]thiazol-2(3H)-one, the title product 3-methyl-6-(6-(6-(1-methyl-1H-pyrazole-4-carbonyl)-2,6-diazaspiro[3.3]heptan-2-yl)pyrazin-2-yl)benzo[d]thiazol-2(3H)-one was obtained.

MS m/z (ESI): 448.1 [M+1].

¹H NMR (400 MHz, MeOD) δ 8.35 (s, 1H), 8.25 (d, 1H), 8.11 - 8.04 (m, 2H), 7.83 (s, 1H), 7.75 (s, 1H), 7.33 (d, 1H), 4.72 - 4.62 (m, 2H), 4.39 - 4.33 (m, 6H), 3.93 (s, 3H), 3.50 (s, 3H).

### Example 108

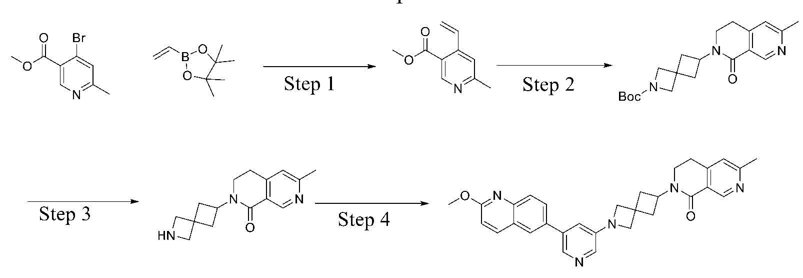

### Step 1

### Methyl 6-methyl-4-vinylnicotinate

At room temperature, 4,4,5,5-tetramethyl-2-vinyl-1,3,2-dioxaborolane (0.52 g, 3.39 mmol), methyl 4-bromo-6-methyl-pyridine-3-carboxylate (0.65 g, 2.83 mmol), sodium carbonate (0.89 g, 8.48 mmol), and 1,1'-bis(diphenylphosphino)ferrocene palladium (II) dichloride (0.12 g, 0.14 mmol) were dissolved in water (2 mL) and 1'4-dioxane (8 mL). The atmosphere was displaced with nitrogen, and the mixture was heated to 80°C, reacted for 14 hours and cooled to room temperature. The reaction solution was diluted with ethyl acetate (25 mL), and filtered. The organic phase was washed with a saturated aqueous sodium chloride solution (25 mL × 2), dried over anhydrous sodium sulfate, filtered, and rotary-evaporated to dryness. The residue was separated by Flash column chromatography (eluted with PE:EA = 90: 10-70: 30) to obtain the target compound as a pale yellow oil (0.39 g, yield: 77.9%).

MS m/z (ESI): 178.1 [M+1].

### Step 2

### Tert-butyl 6-(6-methyl-1-carbonyl-3,4-dihydro-2,7-diazanaphthalen-2(1H)-yl)-2-azaspiro[3.3]heptane-2-carboxylate

At room temperature, tert-butyl 6-amino-2-azaspiro[3.3]heptane-2-carboxylate (0.09 g, 0.43 mmol), acetic acid (0.15 mL), and methyl 6-methyl-4-vinylnicotinate (0.04 g, 0.21 mmol) were dissolved in methanol (2 mL). The atmosphere was displaced with nitrogen, and the mixture was heated to 100°C, reacted for 14 hours and cooled to room temperature. LCMS indicated the completion of the reaction. The mixture was rotary-evaporated to dryness. The residue was dissolved in ethyl acetate (25 mL), and then washed sequentially with a saturated sodium bicarbonate solution (25 mL × 1) and a saturated aqueous sodium chloride solution (25 mL × 1). The organic phase was dried over anhydrous sodium sulfate, filtered, and rotary-evaporated to dryness. The residue was separated by preparative large thin layer chromatography plate (DCM : MeOH = 20: 1) to obtain the target product as a colorless oil (0.06 g, yield: 78.3%).

MS m/z (ESI): 358.2 [M+1].

### Step 3

### 6-Methyl-2-(2-azaspiro[3.3]heptan-6-yl)-3,4-dihydro-2,7-diazanaphthalen-1(2H)-one

At room temperature, tert-butyl 6-(6-methyl-1-carbonyl-3,4-dihydro-2,7-diazanaphthalen-2(1H)-yl)-2-azaspiro[3.3]heptane-2-carboxylate (0.10 g, 0.28 mmol) was dissolved in dichloromethane (2 mL), then trifluoroacetic acid (0.5 mL) was added, and the mixture was stirred at room temperature for 1 hour. LCMS indicated the completion of the reaction. The mixture was rotary-evaporated to dryness, the residue was neutralized with a saturated sodium bicarbonate solution, and lyophilized. The resulting solid was dissolved in dichloromethane (25 mL) again, and filtered. The resulting solution was rotary-evaporated to dryness, and the resulting solid was directly used for the next step (0.06 g, yield: 83.3%).

MS m/z (ESI): 258.1 [M+1].

### Step 4

### 2-(2-(5-(2-Methoxyquinolin-6-yl)pyridin-3-yl)-2-azaspiro[3.3]heptan-6-yl)-6-methyl-3,4-dihydro-2,7-diazanaphthalen-1(2H)-one

At room temperature, 6-(5-bromo-3-pyridyl)-2-methoxy-quinoline (0.025 g, 0.07 mmol), 6-methyl-2-(2-azaspiro[3.3]heptan-6-yl)-3,4-dihydro-2,7-diazanaphthalen-1(2H)-one (0.02 g, 0.078 mmol), tris(dibenzylideneacetone)dipalladium (10 mg, 0.012 mmol), 4,5-bis(diphenylphosphino)-9,9-dimethylxanthene (13.5 mg, 0.024 mmol) and sodium tert-butoxide (11.19 mg, 0.12 mmol) were dissolved in 1'4-dioxane (2 mL). The atmosphere was displaced with nitrogen, and the mixture was heated to 100°C, reacted for 14 hours and cooled to room temperature. The reaction solution was diluted with ethyl acetate (25 mL), and filtered. Then the resultant was washed with a saturated aqueous sodium chloride solution (25 mL × 2). The organic phase was dried over anhydrous sodium sulfate, filtered, and rotary-evaporated to dryness. The residue was separated by preparative large thin layer chromatography plate (DCM : MeOH = 15 : 1). The resulting crude product was further separated by reversed-phase preparative chromatography, and lyophilized, to obtain the target compound as a pale yellow solid (5.0 mg, yield: 13%).

MS m/z (ESI): 492.2 [M+1].

### Example 109

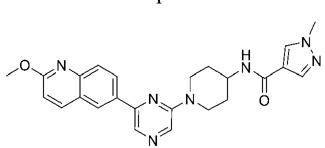

Referring to Example 5, with 3,5-dibromopyrazine and tert-butyl piperidin-4-ylcarbamate as starting materials, replacing 1-methyl-6-(4,4,5,5-tetramethyl-1,3,2-dioxaborolan-2-yl)-1,4-dihydro-2H-benzo[d][1,3]oxazin-2-one with 2-methoxy-6-(4,4,5,5-tetramethyl-1,3,2-dioxaborolan-2-yl)quinoline, the title product N-(1-(6-(2-methoxyquinolin-6-yl)pyrazin-2-yl)piperidin-4-yl)-1-methyl-1H-pyrazole-4-carboxamide was obtained.

MS m/z (ESI): 444.2 [M+1].

¹H NMR (400 MHz, DMSO) δ 8.63 (d, 1H), 8.57 (s, 1H), 8.42 - 8.32 (m, 3H), 8.11 (s, 1H), 7.87 (t, 2H), 7.82 (s, 1H), 7.08 (d, 1H), 4.57 - 4.49 (m, 2H), 4.14 - 4.05 (m, 1H), 4.02 (s, 3H), 3.83 (s, 3H), 3.17 - 3.06 (m, 2H), 2.04 - 1.94 (m, 2H), 1.62 - 1.50 (m, 2H).

### Biological assay and evaluation

The present invention will be further described and explained below in conjunction with test examples, and these examples are not meant to limit the scope of the present invention.

### Test Example 1

G-402 cell line was used as host cells to express (by transient or stable transfection) an enzyme of the human CYP11 family. Specifically, G-402 cell lines stably expressing human CYP11B1 and human CYP11B2 were established respectively. It has been confirmed that the G-402 cell line can express cofactors (adrenodoxin and adrenodoxin reductase) quite essential for the activity of the CYP11 family, and compared to H295R cells, this cell line itself does not exhibit any enzymatic activity associated with the CYP11 family. Therefore, the G-402 cell line is very suitable as a host cell for the ectopic expression of an enzyme of the CYP11 family.

The G-402 cell line, originally derived from renal myoblastoma, can be obtained from ATCC (CRL-1440). The main elements of an expression plasmid include ORF of human CYP11B1 or human CYP11B2, a suitable promoter (CMV promoter), and a suitable resistance marker (neomycin). The expression plasmid was transfected into G-402 cells by standard technology, and a specific antibiotic was then given for screening. The activity of the enzyme expressed by the screened monoclonal cells was assessed by 11-deoxycorticosterone (substrate of CYP11B2) and 11-deoxycortisol (substrate of CYP11B1).

The G-402 cells expressing CYP11 plasmid, as established in the above solution, were cultured in McCoy's 5a Modified Medium (ATCC Catalog No. 30-2007) containing 10% FCS and 400 µg/ml G418 in an incubator at 37°C and 5% CO₂. A cell enzyme experiment was carried out using DMEM/F12 medium containing 2.5% charcoal-treated FBS and an appropriate concentration of a substrate (1 µM 11-deoxycorticosterone or 1 µM 11-deoxycortisol). In order to detect the activity of the enzyme in the cells, the cells were plated in a 96-well plate and incubated for 16 h. The supernatant was then transferred and analyzed for the concentration of the expected product (the product of CYP11B2: aldosterone; the product of CYP11B1: cortisol). The concentrations of these products were determined by an HTRF experiment of CisBio.

In the cell enzyme experiment, the inhibitory effect of the test compound on the generated product can reflect the inhibitory effect thereof on the enzyme. The dose-dependent inhibition of the enzyme activity by the compound was calculated by plotting the concentration of the test compound (x axis) versus the measured product level (y axis). Then, the original data was fitted to a 4-parameter sigmoidal function (Morgan-Mercer-Flodin, MMF model) by least square method: y = (AB + Cx^{D}) / (B + x^{D})
in which A is the maximum y value, B is the EC50 determined by XLFit, C is the minimum y value, and D is the slope value. The maximum value A corresponds to the amount of the product detected without an inhibitor, and C corresponds to the amount of the product detected in the G402 cells expressing an empty plasmid.

The EC₅₀ value of the compound of the present invention was determined by the above G-402 experiment system. The activity of the CYP11B2 enzyme was determined under the conditions of 1 µM 11-deoxycorticosterone and a variable amount of the inhibitor, and the activity of the CYP11B1 enzyme was determined under the conditions of 1 µM 11-deoxycortisol and a variable amount of the inhibitor.

| **Example** | **Human CYP11B2 EC₅₀ (nM)** | **Example** | **Human CYP11B2 EC₅₀ (nM)** |
|---|---|---|---|
| 1-A | 70 | 37 | 35 |
| 4 | 48 | 14-A | 24 |
| 22 | 5 | 23 | 9 |
| 24-A | 16 | 36 | 26 |
| 40 | 13 | 41 | 7 |
| 43 | 50 | 50 | 8 |
| 51 | 24 | 54 | 21 |
| 56 | 7 | 60 | 69 |
| 62 | 14 | 63 | 6 |
| 64 | 32 | 65 | 89 |
| 68 | 33 | 70 | 55 |
| 71 | 4 | 72 | 10 |
| 78 | 24 | 79 | 12 |
| 80 | 17 | 81 | 5 |
| 82 | 18 | 84 | 8 |
| 89 | 9 | 90 | 23 |
| 91 | 12 | 92 | 11 |
| 93 | 48 | 94 | 13 |
| 97 | 11 | 100 | 9 |
| 101 | 43 | 103 | 26 |
| 105 | 124 | 107 | 104 |
| 108 | 71 | 109 | 6 |

| **Example** | **hsF** | **Example** | **hsF** |
|---|---|---|---|
| 1-A | 144x | 48-P2B | 326x |
| 5 | > 1279x | 23 | > 22909x |
| 36 | 2766x | 40 | 2541x |
| 41 | 394x | 43 | > 4023x |
| 42 | 393x | 54 | 404x |
| 51 | 157x | 56 | 159x |
| 59 | > 388x | 60 | 170x |
| 62 | 174x | 63 | 1912x |
| 66-P2 | > 1471x | 67 | > 485x |
| 69 | 585x | 70 | 252x |
| 74 | 371x | 75 | > 16666x |
| 78 | > 8488x | 79 | 3999x |
| 81 | 261x | 82 | 842x |
| 84 | 1652x | 91 | 2278x |
| 92 | 1285x | 93 | 229x |
| 94 | 350x | 97 | 971x |
| 98 | 2014x | 99 | > 3509x |
| 100 | 1048x | 101 | > 4651x |
| 103 | 1292x | 105 | 1372x |
| 106 | 3103x | 107 | > 1923x |
| 108 | > 2817x | 109 | 159x |

| | | | |
|---|---|---|---|
| Note: hSF, the abbreviation of human selective factor, refers to the selectivity of the compound for CYP11B2 over CYP11B1. The numerical value of hSF is the ratio of CYP11B1 EC₅₀ to CYP11B2 EC₅₀. | | | |

As can be seen from the above data, the compound of the present invention has better CYP11B2 activity and also higher selectivity compared with CYP11B1 enzyme.

### Test Example 2 Pharmacokinetic determination in rats

### 1. Study objective:

SD rats were used as test animals to study the pharmacokinetic behavior of the compounds of the present invention in rats (plasma) after oral administration.

### 2. Test scheme

### 2.1 Test drugs:

Compounds of the present invention, made in house

### 2.2 Test animals:

3 SD rats per group, male.

### 2.3 Drug formulation:

Formulation of drug for oral administration: 0.5% CMC-Na (1% Tween 80)

5 g of sodium carboxymethyl cellulose (CMC-Na, viscosity: 800-1200 Cps) was weighed and dissolved in 1000 mL of purified water, and 10 g of Tween 80 was added. These materials were uniformly mixed to form a clear solution.

The example compounds were weighed and dissolved in the solution and the mixture was shaken well and ultrasonicated for 15 min to obtain a colorless clear solution with a concentration of 0.5 mg/mL.
Formulation of drug for intravenous administration: 5% DMSO + 10% Solutol HS15 + 85% PBS

The example compounds were weighed, and 5% DMSO was added based on the total administration volume; the mixture was vortexed and ultrasonicated for 2 min to ensure complete dissolution; then, 10% Solutol HS15 was added, and the mixture was vortexed and ultrasonicated for 2 min to achieve complete dissolution; finally, 85% of PBS was added, and the mixture was vortexed, ultrasonically treated for 5 min, and filtered through a 0.22 um filter membrane to obtain a colorless and transparent clear solution with a concentration of 0.2 mg/mL.

### 2.4 Administration:

After overnight fasting, 3 male SD rats per group were administered orally (PO) at a dose of 5 mg/kg with an administration volume of 10 mL/kg.

After overnight fasting, 3 male SD rats per group were administered intravenously (IV) at a dose of 1 mg/kg with an administration volume of 5 mL/kg.

### 2.5 Sample collection:

0.2 mL of blood was collected from the jugular vein of the experimental animals before administration and at 0.25 h, 0.5 h, 1.0 h, 2.0 h, 4.0 h, 6.0 h, 8.0 h, and 24.0 h after administration and placed in an EDTA-2K test tube, and the test tube was centrifuged at 8000 rpm for 6 min at 4°C to separate the plasma, which was stored at -80°C; and the animals were fed 4 h after administration.

3 Experimental results: The final determination results were obtained by using LCMS/MS method.

| Example | tₘₐₓ (h) | Cₘₐₓ (ng/mL) | AUC₀₋ₜ (ng/mL*h) | AUC_{0-∞} (ng/mL*h) | t_{1/2} (h) | MRT_{0-∞} (h) |
|---|---|---|---|---|---|---|
| 1-A | 1.0 | 6333 | 27618 | 27652 | 2.6 | 3.7 |
| 23 | 2.0 | 5723 | 33621 | 33633 | 2.2 | 3.8 |
| 60 | 2.0 | 4897 | 21755 | 22437 | 1.3 | 3.1 |
| 67 | 4.0 | 4913 | 31381 | 31390 | 2.0 | 3.9 |
| 78 | 4.0 | 3210 | 21637 | 21642 | 1.9 | 4.7 |
| 79 | 4.0 | 3160 | 16683 | 16719 | 2.8 | 4.5 |
| 91 | 1.0 | 2560 | 18181 | 18191 | 2.2 | 4.2 |
| 100 | 2.0 | 3950 | 20098 | 22490 | 2.0 | 4.1 |
| 103 | 4.0 | 2705 | 24736 | 24741 | 1.8 | 5.6 |
| 105 | 4.0 | 6040 | 60378 | 66480 | 7.0 | 9.6 |

### Test Example 3 Ki assay

G-402 cell line was used as host cells to express (by transient or stable transfection) an enzyme of the human CYP11 family. Specifically, G-402 cell lines stably expressing human CYP11B1 and human CYP11B2 were established respectively. To determine the inhibition constants of the test compounds against CYP11B2 and CYP11B1, cells were incubated for 16 hours across different substrate concentrations (for the CYP11B2 cell line: 11-DOC at 0.125-4 µM; for the CYP11B1 cell line: 11-deoxycortisol at 0.3125-10 µM) and different inhibitor concentrations (for the CYP11B2 cell line: 0-100 nM; for the CY11B1 cell line: 0-10000 nM). The product levels (for the CYP11B2 cell line: aldosterone; for the CYP11B1 cell line: cortisol) were quantified using the homogeneous time-resolved fluorescence (HTRF) assay from CisBio. The Ki was determined using the fitting algorithm provided in the GraphPad Prism software.

The compounds of the present invention exhibit higher affinity for CYP11B2, with a Ki value of less than 5 nM, and preferably, the compounds have a Ki value of less than 1 nM. Relative to CYP11B2, the compounds of the present invention show a selectivity for CYP11B1 of more than 200-fold; preferably, the compounds have a selectivity of more than 500-fold, and more preferably, the compounds have a selectivity of more than 2000-fold.

### Test Example 4 In vivo efficacy assay

### 1 Experimental objective

To evaluate the *in vivo* PD efficacy of the compounds in the monkey ACTH Challenge model.

### 2 Experimental instruments and reagents

### 2.1 Instrument

A refrigerator, a biological safety cabinet, a super clean bench, an electronic pipette aid, a constant temperature water bath, an ultrasonic cleaner, a water purification system, a magnetic stirrer, an electronic balance, an electronic balance, an ultrasonic cell disruptor, and LC-MS/MS-BT.

### 2.2 Reagent

ACTH, normal saline, CMC-Na, Tween 80, HP-β-CD, HPMC.

### 3 Experimental operations and data processing

### 3.1 Animals

Cynomolgus monkeys, 35-61 months, 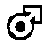 .

### 3.2 Animal model

After the animals arrived at the barrier system, they were acclimated for 14 days, followed by intravenous injection of ACTH.

### 3.3 Grouping and administration

a. The animals were grouped by a random grouping method.
b. According to the grouping results, the test drug was started to be administered (administration method: oral administration; administration volume: 5 mL/kg; frequency of administration: single administration on the same day; vehicle: 0.5% CMC-Na + 1% Tween 80; 20% HP-β-CD + 0.25% HPMC K4M).
c. One hour after administration, ACTH was injected intravenously to establish the monkey ACTH Challenge model (administration route: intravenous injection; administration volume: 1 mL/kg; administration dose: 5 µg/kg; vehicle: normal saline).
d. Blood samples were collected at a series of time points after ACTH injection, and plasma was prepared therefrom.
e. The contents of four relevant hormones in plasma at each time point were detected by the LC/MS-MS method.
f. Data were processed and graphically analyzed using software such as Excel and Graphpad Prism 9. The changing trends of hormones were observed, the time points were determined when the level of each hormone reached its peak, and these time points were selected for inter-group comparison.

### 4. Experimental conclusion:

In the monkey ACTH Challenge experiment, the compounds of the present invention can effectively reduce the aldosterone level at a dose as low as 0.1 mg/kg, without causing significant changes in hormones such as cortisol.

### V. Pharmacokinetic evaluation test in cynomolgus monkeys

### 1. Study objective:

Cynomolgus monkeys were used as test animals to study the pharmacokinetic behavior of the compounds of the present invention in cynomolgus monkeys (plasma) after oral administration at a dose of 3 mg/kg.

### 2. Experimental scheme:

### 2.1 Experimental drugs:

The example compounds of the present invention, made in house.

### 2.2 Experimental animals:

3 cynomolgus monkeys per group, all male.

### 2.3 Formulation of preparations:

Formulation of drug for oral administration: 0.5% CMC-Na (1% Tween 80).

0.5 g of CMC-Na (viscosity: 800-1200) and 1.0 g of Tween 80 were weighed into a 100 ml volumetric flask, then vortexed, mixed well, and ultrasonically treated to obtain a clear solution.

The compound of the present invention was weighed and added to a 100 mL glass bottle, the solution was added, and the mixture was vortexed and ultrasonically treated for 10 minutes to obtain a white suspension with a concentration of 0.6 mg/mL.

### 2.4 Administration:

After overnight fasting, 3 male cynomolgus monkeys were administered orally (p.o.) at a dose of 3 mg/kg and an administration volume of 5 mL/kg.

### 2.5 Sample collection:

Blood collection: 0.3 mL of blood was collected from the forelimb vein of cynomolgus monkeys before administration and at 0.25, 0.5, 1, 2, 4, 6, 8 and 24 h after administration, and placed in an EDTA-K₂ anticoagulation tube, and the plasma was separated by centrifugation at 6000 rpm at 4°C for 6 min and stored at -80°C; the animals were fed 4 h after administration.

### 2.6 Sample treatment:

1) Acetonitrile (160 µL) was added to plasma sample (40 µL) for precipitation, and the mixture was centrifuged at 3500 × g for 5-20 min.
2) The treated supernatant solution was taken and subjected to LC/MS/MS to analyze the concentrations of the test compound. LC/MS/MS analysis instrument: AB Sciex API 4000 Qtrap.

### 2.7 Liquid phase analysis:

• Liquid phase conditions: Shimadzu LC-20AD pump
• Chromatographic column: Agilent ZORBAX XDB-C18 (50×2.1 mm, 3.5 µm), mobile phase: liquid A: 0.1% formic acid in water, liquid B: acetonitrile
• Flow rate: 0.4 mL/min
• Elution time: 0-4.0 min, the eluent was as follows:

| Time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental conclusion:

The compounds of the present invention exhibit excellent metabolic behaviors in cynomolgus monkeys. At an oral administration (PO) dose of 3 mg/kg, the plasma exposure AUC_{0-∞} can reach 7000-12000 ng/mL*h.

## Claims

1. A compound as represented by general formula (I), or a stereoisomer or a pharmaceutically acceptable salt thereof: 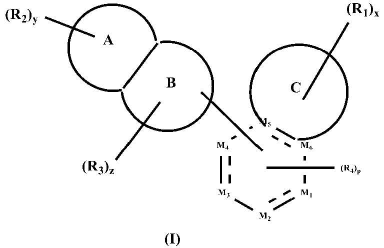 **characterized in that**
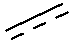 is a single bond or a double bond;
ring A is phenyl, 4- to 7-membered heterocyclyl, 5- to 6-membered heteroaryl, or absent;
ring B is 4- to 7-membered heterocyclyl, 5- to 6-membered heteroaryl, or phenyl;
ring C is cycloalkyl, heteroaryl, heterocyclyl, or absent;
M₁, M₂, M₄, M₅, and M₆ are each independently selected from N, NH, or CH;
M₃ is a bond, N, or CH;
each R₁ is independently selected from cycloalkyl, heterocyclyl, cycloalkyloxy, heterocyclyloxy, cycloalkylamino, heterocyclylamino, cycloalkylthio, heterocyclylthio, -C(O)(CH₂)ₙR_{b}, -NRₐC(O)(CH₂)ₙR_{b}, -O(CH₂)ₙR_{b}, -NH(CH₂)ₙR_{b}, - S(CH₂)ₙR_{b}, -S(O)₂(CH₂)ₙR_{b}, -S(O)(NH)(CH₂)ₙR_{b}, -NRₐS(O)₂(CH₂)ₙR_{b}, - NRₐS(O)(NH)(CH₂)ₙR_{b}, or -NS(O)(CH₂)ₙRₐR_{b}, wherein optionally the cycloalkyl, heterocyclyl, cycloalkyloxy, heterocyclyloxy, cycloalkylamino, heterocyclylamino, cycloalkylthio, or heterocyclylthio is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, - C(O)(CH₂)ₙR_{b}, -S(O)₂(CH₂)ₙR_{b}, -S(O)(NH)(CH₂)ₙR_{d}, -S(O)₂(CH₂)ₙR_{d}, or - NRₐS(O)(NH)(CH₂)ₙR_{b};
or any two R₁, together with adjacent carbon atoms, form 3- to 8-membered cycloalkyl or 4- to 7-membered heterocyclyl, wherein optionally the 3- to 8-membered cycloalkyl or 4- to 7-membered heterocyclyl is further substituted with oxo, -C(O)R_{b}, -NRₐC(O)R_{b}, -S(O)₂R_{b}, -S(O)(NH)R_{b}, -NRₐS(O)₂R_{b}, or - NRₐS(O)(NH)R_{b};
Rₐ or R_{b} is each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, aryl, heteroaryl, or heterocyclyl, wherein optionally the cycloalkyl, aryl, heteroaryl, or heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, or -S(O)₂alkyl;
R₂, R₃, or R₄ is each independently selected from hydrogen, deuterium, oxo, halogen, amino, hydroxyl, cyano, nitro, alkyl, alkenyl, alkynyl, deuteroalkyl, haloalkyl, alkoxy, haloalkoxy, hydroxyalkyl, cycloalkyl, heterocyclyl, aryl, or heteroaryl, wherein the alkyl, alkenyl, alkynyl, deuteroalkyl, haloalkyl, alkoxy, haloalkoxy, hydroxyalkyl, cycloalkyl, heterocyclyl, aryl, heteroaryl, - NRₐC(O)(CH₂)ₙR_{b}, or -C(O)NRₐ(CH₂)ₙR_{b} can be optionally further substituted;
or R₂ and R₃, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or any two R₂, together with adjacent atoms, form 3- to 8-membered cycloalkyl, 5- to 6-membered heteroaryl, or 4- to 7-membered heterocyclyl, wherein optionally the 3- to 8-membered cycloalkyl, 5- to 6-membered heteroaryl, or 4- to 7-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
or R₂ and R₄, together with adjacent atoms, form 5- to 14-membered cycloalkyl, 5- to 14-membered heteroaryl, or 5-14-membered heterocyclyl, wherein optionally the 5- to 14-membered cycloalkyl, 5- to 14-membered heteroaryl, or 5-14-membered heterocyclyl is further substituted with one or more substituents selected from oxo, deuterium, halogen, amino, hydroxyl, cyano, nitro, alkyl, alkenyl, alkynyl, deuteroalkyl, haloalkyl, alkoxy, haloalkoxy, or hydroxyalkyl;
p, x, y, and z are each independently selected from 1, 2, 3, or 4; and
n is selected from 0, 1, 2, or 3;
when 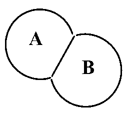 is 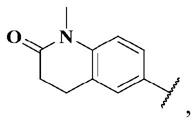 M₁ is N, and ring C is 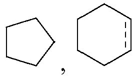 or 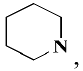 R₁ is not -NRₐC(O)R_{b} and -NRₐS(O)₂R_{b};
when M₁ is N, and ring C is absent, or is 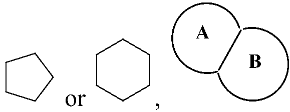 is not

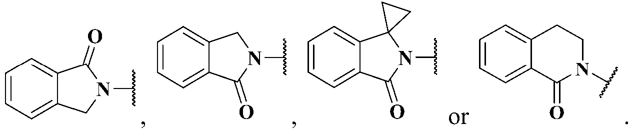

2. The compound, or the stereoisomer or the pharmaceutically acceptable salt thereof according to claim 1, **characterized in that** ring A is absent or selected from 5- to 10-membered heterocyclyl or 5- to 10-membered heteroaryl containing 1-3 moieties selected from C(O), N, O, S, SO₂ or SONH; and preferably 5- to 7-membered heterocyclyl or 5- to 6-membered heteroaryl containing 1-3 moieties selected from C(O), N, O, S, SO₂ or SONH.

3. The compound, or the stereoisomer or the pharmaceutically acceptable salt thereof according to claim 1, **characterized in that** ring B is selected from phenyl, or 5- to 7-membered heterocyclyl or 5- to 6-membered heteroaryl containing 1-3 moieties selected from C(O), N, O or S.

4. The compound, or the stereoisomer or the pharmaceutically acceptable salt thereof according to claim 1, **characterized in that**
ring A is selected from 5- to 7-membered heterocyclyl containing C(O), N or S, or 5- to 6-membered heteroaryl containing 1-3 moieties selected from C(O), N, O, S, SO₂ or SONH; ring B is selected from phenyl;
or 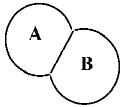 is selected from 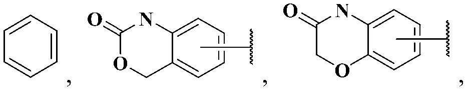 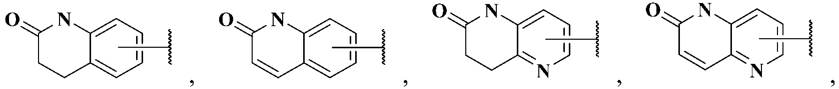 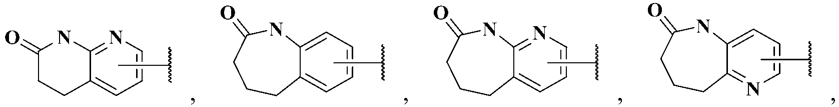 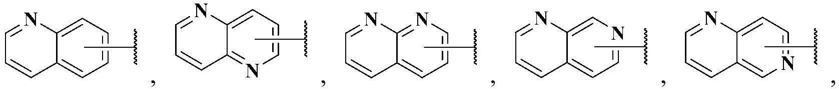 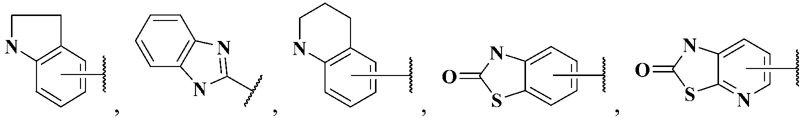 or 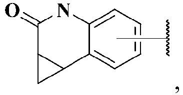
more preferably 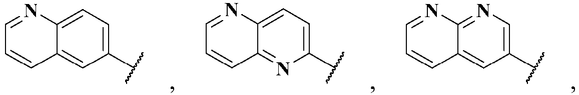 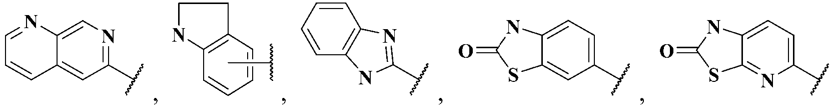 or

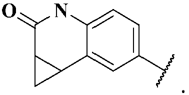

5. The compound, or the stereoisomer or the pharmaceutically acceptable salt thereof according to claim 1, **characterized in that** ring C is absent or selected from 3- to 10-membered cycloalkyl, or 4- to 10-membered heterocyclyl containing 1-3 moieties selected from C(O), N, O, S, SO₂, or SONH;
preferably, ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, or 7- to 10-membered bicyclic heterocyclyl containing 1-3 moieties selected from C(O), N, O, S, SO₂, or SONH.

6. The compound, or the stereoisomer or the pharmaceutically acceptable salt thereof according to claim 1, **characterized in that**
each R₁ is independently selected from 3- to 8-membered cycloalkyl, 4- to 8-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, 4- to 8-membered heterocyclylthio, -C(O)(CH₂)ₙR_{d}, -NRₐC(O)(CH₂)ₙR_{b}, -O(CH₂)ₙR_{d}, -NH(CH₂)ₙR_{b}, - S(CH₂)ₙR_{b}, -S(O)₂(CH₂)ₙR_{b}, -S(O)(NH)(CH₂)ₙR_{d}, -NRₐS(O)₂(CH₂)ₙR_{d}, or - NRₐS(O)(NH)(CH₂)ₙR_{b}, wherein optionally the 3- to 8-membered cycloalkyl, 4- to 8-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, or 4- to 8-membered heterocyclylthio is further substituted with one or more substituents selected from oxo, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, -C(O)(CH₂)ₙR_{d}, -S(O)₂(CH₂)ₙR_{b}, -S(O)(NH)(CH₂)ₙR_{d}, - S(O)₂(CH₂)ₙR_{b}, or -NRₐS(O)(NH)(CH₂)ₙR_{b};
Rₐ or R_{b} is each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl, 3- to 8-membered cycloalkyl, 5- to 8-membered heteroaryl containing 1-3 moieties selected from C(O), N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 moieties selected from C(O), N, O, or S, or 4-to 8-membered heterocyclyl containing 1-3 moieties selected from C(O), N, O, or S is further substituted with one or more substituents selected from oxo, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ hydroxyalkyl or - SO₂-C₁₋₆ alkyl;
preferably,
each R₁ is independently selected from 3- to 8-membered cycloalkyl, 4- to 8-membered heterocyclyl containing 1-3 moieties selected from N, O, S, SO₂, or SONH, 3- to 8-membered cycloalkyloxy, 4- to 8-membered heterocyclyloxy containing 1-3 moieties selected from N, O, S, SO₂, or SONH, -C(O)(CH₂)ₙR_{b}, - NRₐC(O)(CH₂)ₙR_{b}, -O(CH₂)ₙR_{b}, -S(O)₂(CH₂)ₙR_{b}, -S(O)(NH)(CH₂)ₙR_{b}, - NRₐS(O)₂(CH₂)ₙR_{d}, or -NRₐS(O)(NH)(CH₂)ₙR_{d}, wherein optionally the 3- to 8-membered cycloalkyl, 4- to 8-membered heterocyclyl containing 1-3 moieties selected from N, O, S, SO₂, or SONH, 3- to 8-membered cycloalkyloxy, or 4- to 8-membered heterocyclyloxy containing 1-3 moieties selected from N, O, S, SO₂, or SONH is further substituted with one or more substituents selected from oxo, - C(O)(CH₂)ₙR_{b}, -S(O)₂(CH₂)ₙR_{b}, -S(O)(NH)(CH₂)ₙR_{d}, -S(O)₂(CH₂)ₙR_{d}, or - NRₐS(O)(NH)(CH₂)ₙR_{b};
Rₐ or R_{b} is each independently selected from hydrogen, deuterium, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 5- to 8-membered heteroaryl containing 1-3 heteroatoms selected from N, O, or S or the 4-to 8-membered heterocyclyl containing 1-3 moieties selected from C(O), N, O, or S is further substituted with one or more substituents selected from oxo, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, or - SO₂-C₁₋₃ alkyl.

7. The compound, or the stereoisomer or the pharmaceutically acceptable salt thereof according to claim 1, **characterized in that**
each R₁ is independently selected from 3- to 10-membered cycloalkyl, 4- to 10-membered heterocyclyl containing 1-3 moieties selected from N, O, S, SO₂, or SONH, 3- to 8-membered cycloalkyloxy, 3- to 8-membered cycloalkylamino, 4- to 8-membered heterocyclyloxy containing 1-3 moieties selected from N, O, S, SO₂, or SONH, 4- to 8-membered heterocyclylamino containing 1-3 moieties selected from N, O, S, SO₂, or SONH, -C(O)(CH₂)ₙR_{d}, -NRₐC(O)(CH₂)ₙR_{d}, -O(CH₂)ₙR_{b}, - S(O)₂(CH₂)ₙR_{S}, -S(O)(NH)(CH₂)ₙR_{b}, -NRₐS(O)₂(CH₂)ₙR_{d}, or - NRₐS(O)(NH)(CH₂)ₙR_{b}, wherein optionally the 3- to 10-membered cycloalkyl, 4- to 10-membered heterocyclyl containing 1-3 moieties selected from N, O, S, SO₂, or SONH, 3- to 8-membered cycloalkyloxy, 3- to 8-membered cycloalkylamino, 4- to 8-membered heterocyclyloxy containing 1-3 moieties selected from N, O, S, SO₂, or SONH, or 4- to 8-membered heterocyclylamino containing 1-3 moieties selected from N, O, S, SO₂, or SONH is further substituted with one or more substituents selected from oxo, -C(O)(CH₂)ₙR_{b}, -S(O)₂(CH₂)ₙR_{b}, -S(O)(NH)(CH₂)ₙR_{d}, - S(0)₂(CH₂)ₙR_{b}, or -NRₐS(O)(NH)(CH₂)ₙR_{b}; and
Rₐ or R_{b} is each independently selected from hydrogen, deuterium, halogen, hydroxyl, cyano, C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 5- to 8-membered heteroaryl containing 1-3 heteroatoms selected from N, O, or S, or 4- to 8-membered heterocyclyl containing 1-3 moieties selected from C(O), N, O, or S is further substituted with one or more substituents selected from oxo, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, 3- to 6-membered cycloalkyl, or -SO₂-C₁₋₃ alkyl.

8. The compound, or the stereoisomer or the pharmaceutically acceptable salt thereof according to claim 1, **characterized in that**
R₂, R₃, or R₄ is each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ deuteroalkoxy, C₁₋₆ hydroxyalkyl, 3- to 8-membered cycloalkyl, 3- to 8-membered cycloalkyloxy, 3- to 8-membered cycloalkylamino, C₆₋₁₀ aryl, 5- to 6-membered heteroaryl containing 1-3 heteroatoms selected from N, O, or S or 4- to 8-membered heterocyclyl containing 1-3 moieties selected from C(O), N, O, or S, -NRₐR_{b}, -NRₐC(O)R_{b}, or - C(O)NRₐR_{b};
preferably, R₂, R₃, or R₄ is each independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy.

9. The compound, or the stereoisomer or the pharmaceutically acceptable salt thereof according to claim 1, **characterized in that** 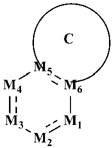 is selected from the following groups:

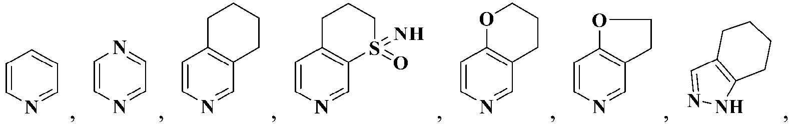

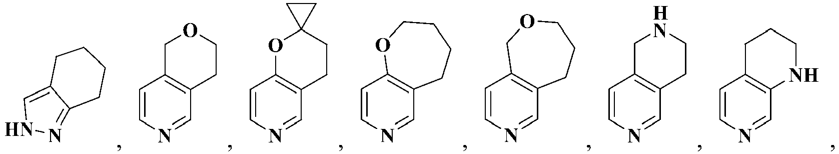

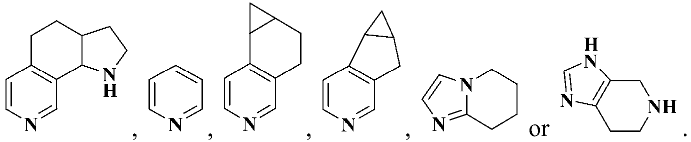

10. The compound, or the stereoisomer or the pharmaceutically acceptable salt thereof according to any one of claims 1-9, **characterized in that** the compound, or the stereoisomer or the pharmaceutically acceptable salt thereof is further a compound as represented by general formula (IV-a) or (IV-b), or a stereoisomer or a pharmaceutically acceptable salt thereof: 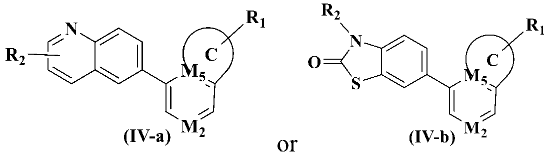 wherein
M₂ or M₃ is each independently selected from N or CH;
ring C is selected from 5- to 7-membered monocyclic cycloalkyl, 6- to 10-membered bicyclic cycloalkyl, 5- to 7-membered monocyclic heterocyclyl containing 1-3 moieties selected from C(O), N, O, S, SO₂, or SONH, 7- to 10-membered bicyclic heterocyclyl containing 1-3 moieties selected from C(O), N, O, S, SO₂, or SONH, or absent;
R₁ is selected from -C(O)R_{b}, -NRₐC(O)R_{b}, -OR_{b}, -S(O)₂R_{b}, -S(O)(NH)R_{b}, - NRₐS(O)₂R_{b}, -NRₐS(O)(NH)R_{b}, 4- to 8-membered nitrogen-containing heterocyclyl, 4- to 8-membered nitrogen-containing heterocyclyloxy, 4- to 8-membered nitrogen-containing heterocyclylthio, or 4- to 8-membered nitrogen-containing heterocyclylamino, wherein the 4- to 8-membered nitrogen-containing heterocyclyl, 4- to 8-membered nitrogen-containing heterocyclyloxy, 4- to 8-membered nitrogen-containing heterocyclylthio, or 4- to 8-membered nitrogen-containing heterocyclylamino is optionally further substituted with -C(O)R_{b} or -S(O)₂R_{b};
Rₐ or R_{b} is each independently selected from hydrogen, deuterium, halogen, hydroxy, cyano, C₁₋₃ alkyl, C₁₋₃ deuteroalkyl, C₁₋₃ haloalkyl, C₁₋₃ alkoxy, C₁₋₃ haloalkoxy, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the C₁₋₃ alkyl, 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, S, SO₂ or SONH is further substituted with hydroxyl, amino, CN, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or 3- to 6-membered cycloalkyl;
R₂ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy.

11. The compound, or the stereoisomer or the pharmaceutically acceptable salt thereof according to claim 10, **characterized in that** the compound, or the stereoisomer or the pharmaceutically acceptable salt thereof is further a compound as represented by general formula (V-a) or (V-b), or a stereoisomer or a pharmaceutically acceptable salt thereof: 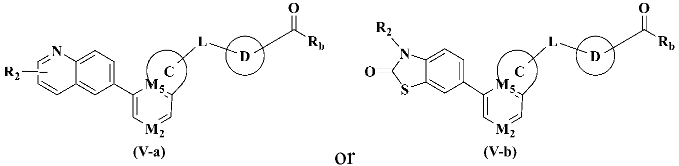 wherein
L is selected from a bond, NH or O; and
ring D is selected from 4- to 8-membered heterocyclyl containing 1 to 3 nitrogen atoms.

12. The compound, or the stereoisomer or the pharmaceutically acceptable salt thereof according to any one of claims 1-9, **characterized in that** the compound, or the stereoisomer or the pharmaceutically acceptable salt thereof is further a compound as represented by general formula (VI-a), (VI-c), (VI-d), (VI-e), (VI-f), (VI-g) and (VI-i), or a stereoisomer or a pharmaceutically acceptable salt thereof: 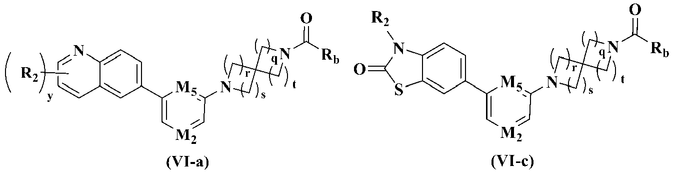 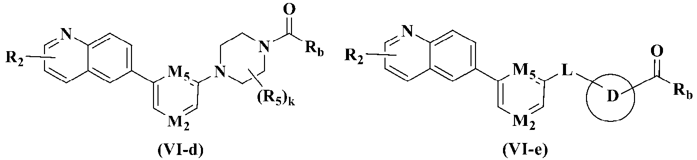 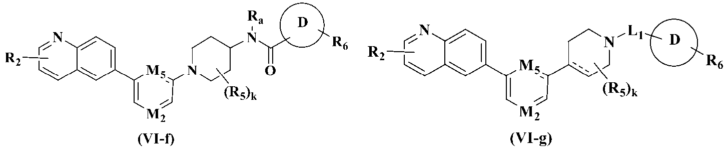 or 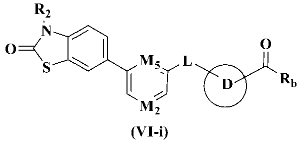 wherein
L is selected from NRₐ, O, or S;
L₁ is selected from C(O) or S(O)₂;
Rₐ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
M₂ is selected from N or CH;
M₅ is selected from N or CH;
R₂ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
or any two R₂, together with adjacent atoms, form 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S;
each R_{b} is independently selected from hydrogen, deuterium, halogen, hydroxy, cyano, C₁₋₃ alkyl, C₁₋₃ deuteroalkyl, C₁₋₃ haloalkyl, C₁₋₃ alkoxy, C₁₋₃ haloalkoxy, 3-to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S, wherein optionally the 3- to 8-membered cycloalkyl, 5- to 8-membered heteroaryl containing 1-3 heteroatoms selected from N, O, or S, or 4- to 8-membered heterocyclyl containing 1-3 moieties selected from C(O), N, O, or S is further substituted with hydroxyl, amino, CN, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or 3- to 6-membered cycloalkyl;
each R₅ is independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
R₆ is selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyl;
or any two R₅, together with adjacent carbon atoms, form 3- to 8-membered cycloalkyl or 3- to 8-membered heterocyclyl;
ring D is selected from 3- to 8-membered cycloalkyl or 4- to 8-membered heterocyclyl, preferably 4- to 6-membered nitrogen-containing heterocyclyl, more preferably 4- to 6-membered heterocyclyl containing 1-2 nitrogen atoms;
q, r, s, and t are each independently selected from 1 or 2; and
k is independently selected from 1, 2, or 3.

13. A compound as follows, or a stereoisomer or a pharmaceutically acceptable salt thereof: 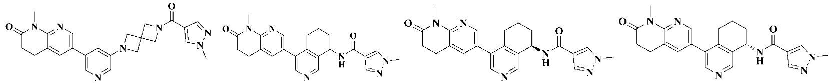 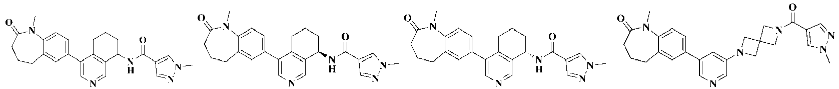 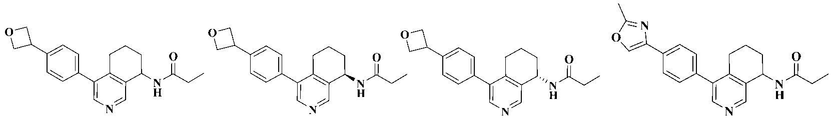 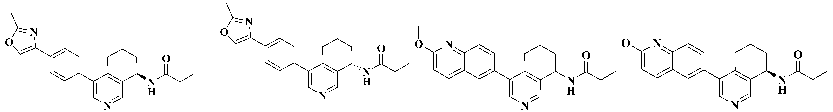 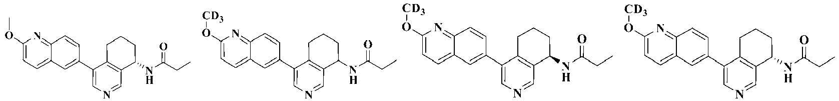 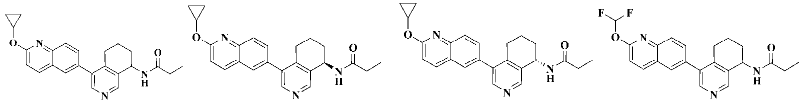 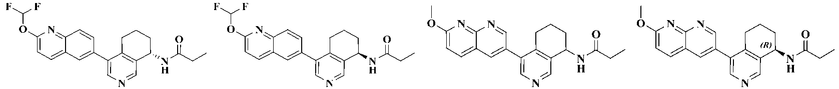 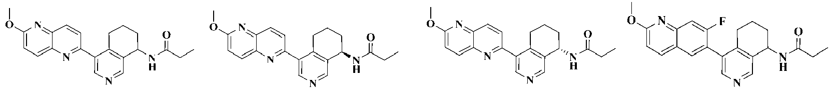 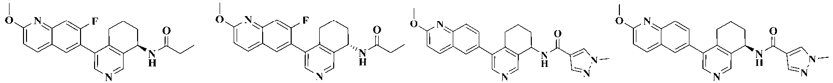 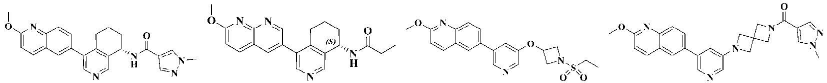 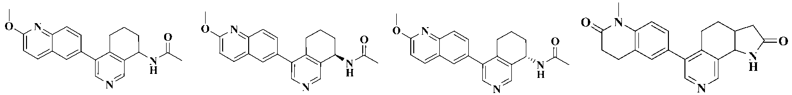 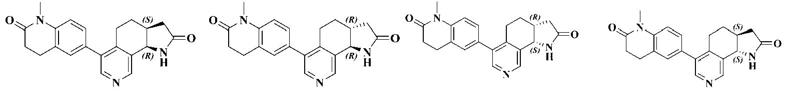 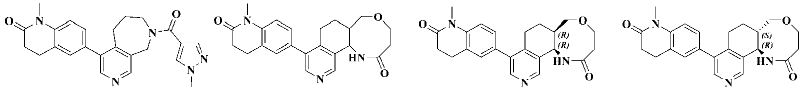 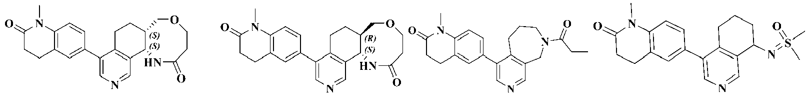 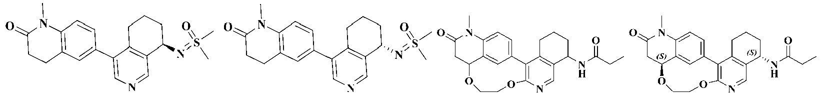 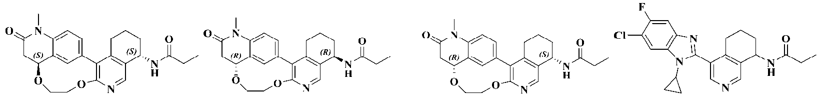 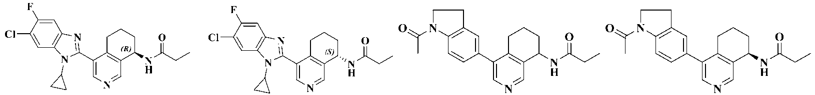 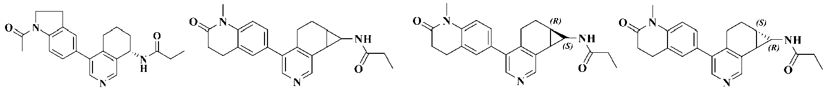 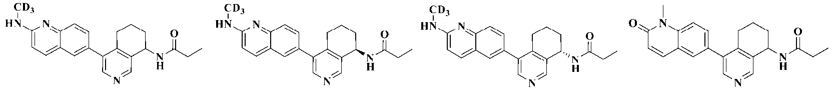 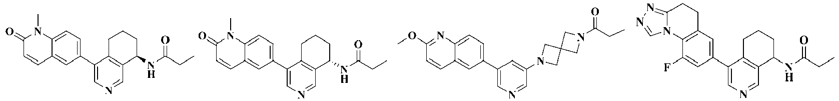 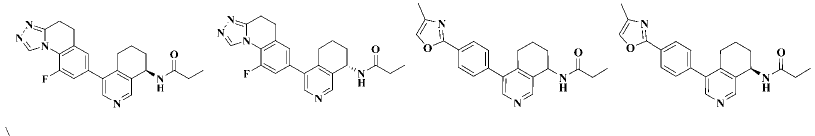 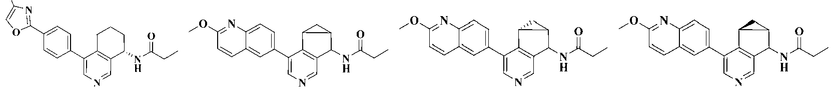 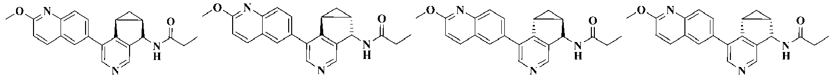 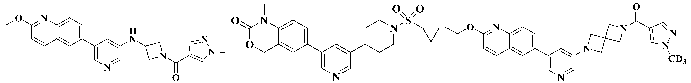 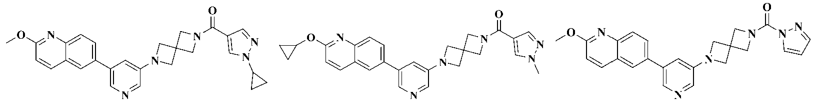 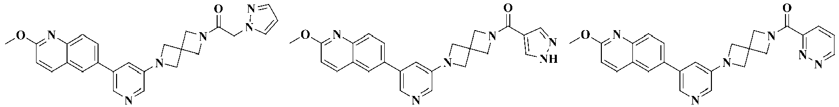 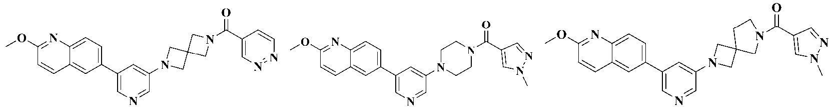 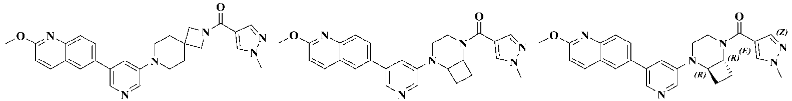 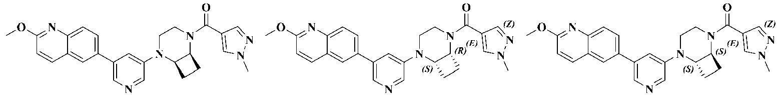 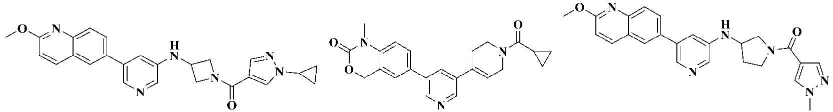 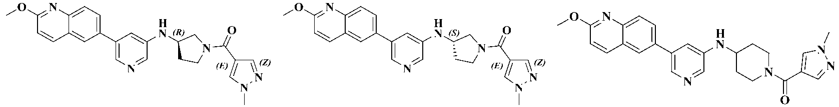 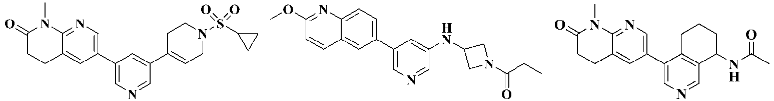 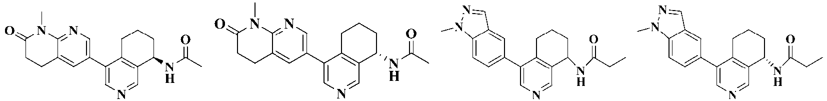 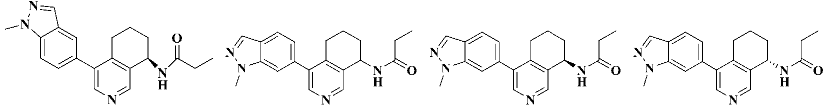 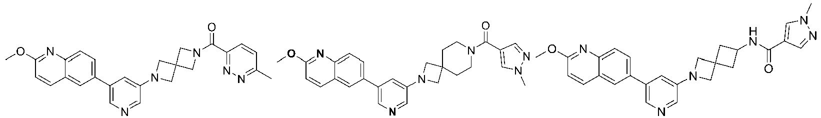 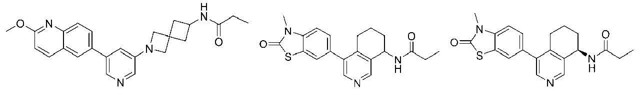 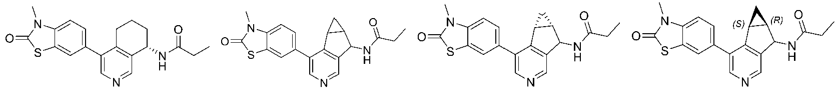 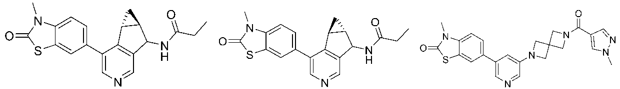 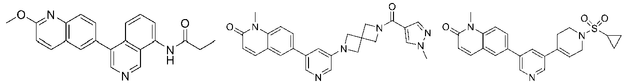 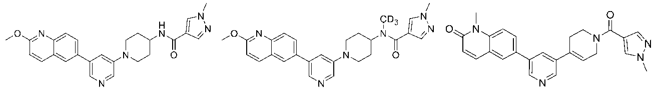 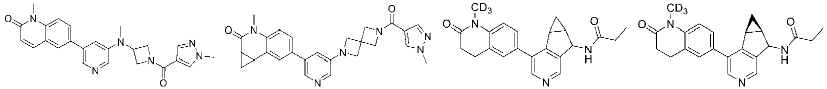 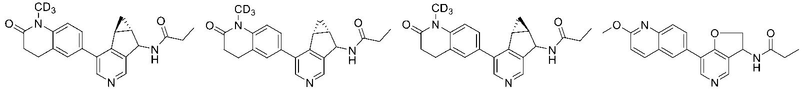 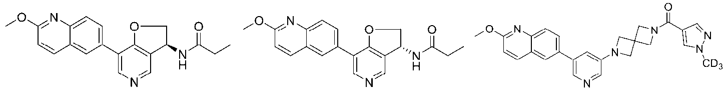 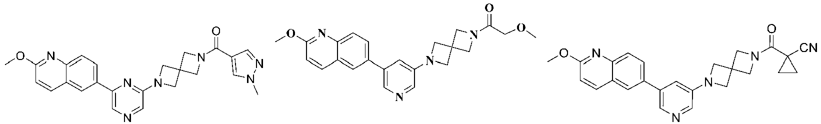 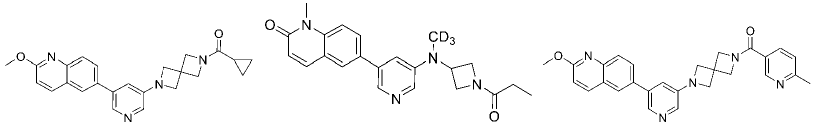 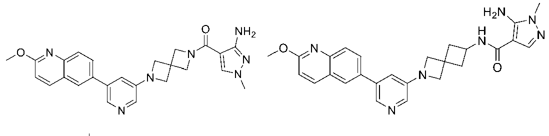 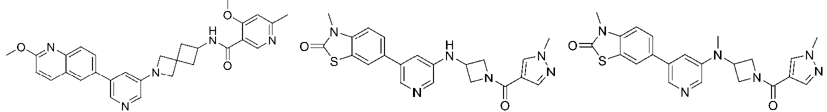 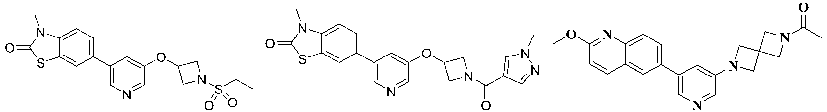 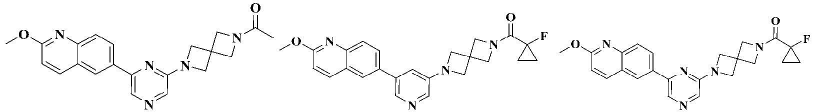 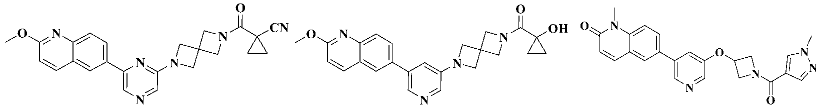 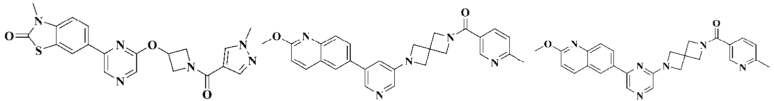 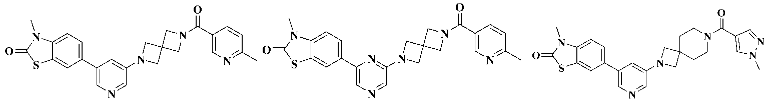 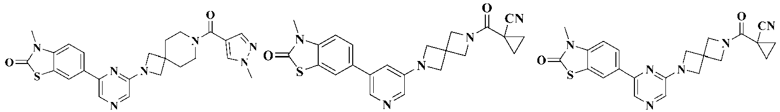 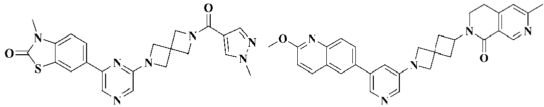 or

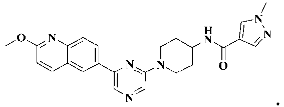

14. A pharmaceutical composition, comprising a therapeutically effective dose of the compound, or the stereoisomer or the pharmaceutically acceptable salt thereof according to any one of claims 1-13, and one or more pharmaceutically acceptable carriers, diluents, or excipients.

15. A compound as represented by general formula (X-a) or (X-b), or a stereoisomer or a pharmaceutically acceptable salt thereof: 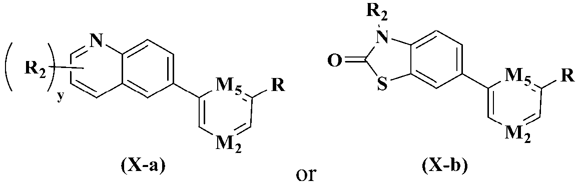
M₂ or M₅ is each independently selected from N or CH;
each R is independently selected from hydrogen, 3- to 10-membered cycloalkyl, 4- to 10-membered heterocyclyl, 3- to 8-membered cycloalkyloxy, 4- to 8-membered heterocyclyloxy, 3- to 8-membered cycloalkylamino, 4- to 8-membered heterocyclylamino, 3- to 8-membered cycloalkylthio, or 4- to 8-membered heterocyclylthio;
each R₂ is independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ haloalkoxy, C₁₋₆ deuteroalkoxy, C₁₋₆ hydroxyalkyl, 3- to 8-membered cycloalkyl, 3- to 8-membered cycloalkyloxy, 3- to 8-membered cycloalkylamino, C₆₋₁₀ aryl, 5- to 6-membered heteroaryl containing 1-3 heteroatoms selected from N, O, or S or 4- to 8-membered heterocyclyl containing 1-3 moieties selected from C(O), N, O, or S, -NRₐR_{b}, -NRₐC(O)R_{b}, or - C(O)NRₐR_{b};
preferably, each R₂ is independently selected from hydrogen, deuterium, halogen, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl, or C₃₋₆ cycloalkyloxy;
y is selected from 1, 2 or 3.

16. Use of the compound according to any one of claims 1-13 or the pharmaceutical composition according to claim 14 in the preparation of a drug for treating or preventing chronic kidney disease, kidney or cardiac fibrosis, diabetic nephropathy, congestive heart failure, hypertension, primary aldosteronism and Cushing's syndrome; preferably, the hypertension is refractory hypertension, the chronic kidney disease is chronic kidney disease with type II diabetes.

17. Use of the compound of the general formula, or the stereoisomer or the pharmaceutically acceptable salt thereof according to any one of claims 1-13 or the pharmaceutical composition according to claim 14 in the preparation of a drug for treating a disease related to CYP11B2.

18. A method for preparing a compound as represented by formula (V-a-3), (V-a-6) or (V-b-2), or a stereoisomer or a pharmaceutically acceptable salt thereof, comprising the following steps: 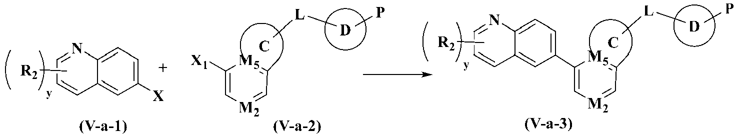
performing a coupling reaction between formula (V-a-1) and formula (V-a-2) to prepare a compound as represented by formula (V-a-3), or a stereoisomer or a pharmaceutically acceptable salt thereof; optionally, performing a deprotection on formula (V-a-3) to obtain a compound as represented by formula (V-a-4), or a stereoisomer or a pharmaceutically acceptable salt thereof; 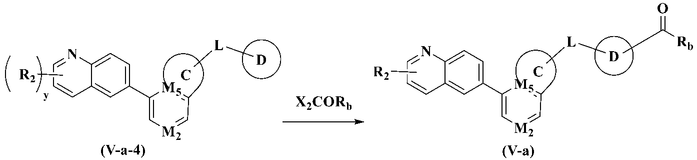
optionally, using formula (V-a-4) and X₂COR_{b} to prepare a compound as represented by general formula (V-a), or a stereoisomer or a pharmaceutically acceptable salt thereof;
or 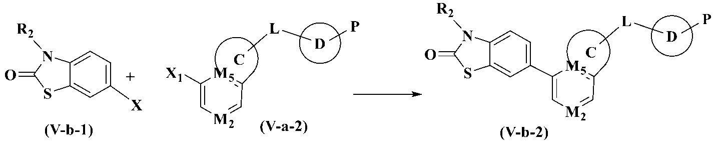
performing a coupling reaction between formula (V-b-1) and formula (V-a-2) to prepare a compound as represented by formula (V-b-2), or a stereoisomer or a pharmaceutically acceptable salt thereof; optionally, performing a deprotection to obtain a compound as represented by formula (V-b-3), or a stereoisomer or a pharmaceutically acceptable salt thereof; 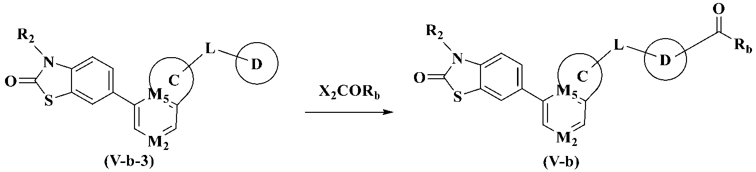
optionally, using formula (V-b-3) and X₂COR_{b} to prepare a compound as represented by formula (V-b), or a stereoisomer or a pharmaceutically acceptable salt thereof;
or 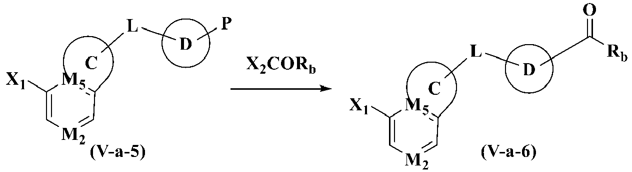
using formula (V-a-5) and X₂COR_{b} to prepare a compound as represented by formula (V-a-6), or a stereoisomer or a pharmaceutically acceptable salt thereof; 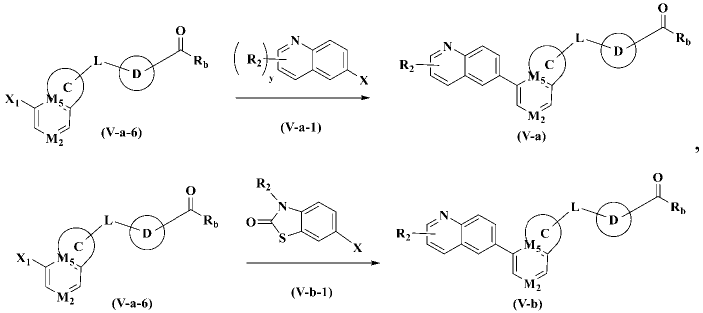
optionally, further performing a coupling reaction between formula (V-a-6) with formula (V-a-1) or (V-b-1) to prepare a compound as represented by formula (V-a) or formula (V-b), or a stereoisomer or a pharmaceutically acceptable salt thereof;
wherein
X is boranyl; X₁ is halogen; X₂ is halogen or hydroxy; P is an amino protecting group; and
M₂, M₅, L, R₂, R_{b}, ring C, ring D and y are as defined in claim 11.
